# EUROPEAN PATENT APPLICATION

(11) **EP 4 624 460 A1**
(43) Date of publication of application: **01.10.2025**
(21) Application number: 23894018.3
(22) Date of filing: 24.11.2023
(51) Int. Cl.: C07D 295/088, A61K 9/127, A61K 9/51

(54) **IONIZABLE LIPID AND USE THEREOF**

(30) Priority: 25.11.2022 CN 202211494231
(71) Applicant: Axter Therapeutics (Beijing) Co., Ltd., Daxing District, Beijing 100023 (CN)
(72) Inventor: ZHANG, Yuanyuan, Beijing 100023 (CN); LI, Ke, Beijing 100023 (CN); LI, Huo, Beijing 100023 (CN); GU, Hanqing, Beijing 100023 (CN); XU, Zengjun, Beijing 100023 (CN)
(74) Representative: Lavoix
(86) International application number: PCT/CN2023/133961
(87) International publication number: WO 2024/109929

(57) **Abstract**

Provided in the present invention are an ionizable lipid, and a drug delivery system containing the ionizable lipid. Specifically, provided in the present invention is an ionizable lipid having the structure of formula (I), or a pharmaceutically acceptable salt, tautomer, or stereoisomer thereof. A lipid nanoparticle constructed by means of using the ionizable lipid can realize safe and efficient delivery of nucleic acid drugs, small-molecule drugs, peptide drugs and protein drugs.

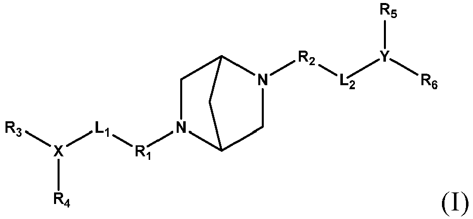

## Description

### TECHNICAL FIELD

The present invention relates to the technical field of biomedicine, in particular to an ionizable lipid and the use thereof in drug delivery.

### BACKGROUND

In recent years, messenger RNA drugs have become a key therapy for preventing and treating infectious diseases and tumors. The messenger RNA drug technology has been recognized by the industry, due to its short research and development cycle, low insertion mutagenesis risk, and diversity of encoded proteins. mRNAs are highly suitable for the development of vaccines or therapeutic drugs. However, mRNA itself is highly unstable and easily degraded by ubiquitous RNases. In addition, due to the inherent negativity and large molecular weight (usually greater than 10⁶ Da) of mRNA, the entry of mRNA molecules into cells is limited. Therefore, developing a suitable delivery carrier to protect fragile mRNA molecules and deliver them into the cytoplasm is of great significance.

At present, various mRNA delivery carriers have been developed, including lipid nanoparticles (LNPs), inorganic nanoparticles, polymer nanoparticles, viral vectors, and exosomes. Currently, LNP is widely used as a delivery carrier for various drugs, such as nucleic acid drugs (mRNA, DNA, siRNA, ASO, etc.), small molecule drugs, antibodies, polypeptides, etc. Its main components include ionizable lipids, phospholipids, cholesterol, and lipids containing polyethylene glycol. The most important component in LNP is ionizable lipids. Early cationic lipids with permanent positive charge have lower circulation time, higher toxicity, and severe allergic reactions in the body, since their inherent positive charge adsorbs proteins during circulation and they are easily captured and cleared by the reticular endothelial system. The inherent positive charge can interact with negatively charged cell membranes, leading to membrane instability and severe toxicity; Cationic lipids with permanent positive charge can activate the complement system, leading to allergic reactions. Ionizable lipids are not charged under physiological pH conditions, therefore, LNP prepared from ionizable lipids has relatively high safety. Ionizab-le lipids endow LNP with lysosome-escaping ability, causing LNP to escape and release mRNA into the cytoplasm through proton sponge effect and membrane fusion mechanism, then the mRNA binds to the ribosome encoding a protein and translates the encoded protein.

With the deepening of research and the demand for therapeutic effects on diseases, precise targeted organ delivery of LNP has emerged. The advantages of targeted delivery mainly lie in improving the effective accumulation and release of therapeutic drugs in target tissues or cells, thereby enhancing efficacy and reducing systemic toxicity. At present, research on targeted organs includes lymph node targeting, lung targeting, liver targeting, spleen targeting, etc. The main targeted development strategies for the carrier type of LNP include the design and screening of novel lipid molecules; optimization of LNP component formulation; LNP surface modification; selection of administration routes, etc.

Recently, the team led by Xu Qiaobing independently developed an ionizable lipid 113-O12B, which was used for lymph node targeting research on LNP mRNA delivery. It was compared with the formulation of ALC-0315 lipid, which has been commercially available from Biotech, for lymph node targeting. The results showed that the 113-O12B formulation has better lymph node targeting, but both still have high liver aggregation.

In summary, the development of suitable ionizable lipids is one of the key factors in developing LNPs with high safety and high lysosomal escape efficiency. In addition, exploring and optimizing organ-targeted delivery formulation of LNP is also an important research and development direction in this field.

### SUMMARY OF THE INVENTION

The objective of the present invention is to provide an ionizable lipid with low toxicity, high delivery efficiency, which can be used as an important component of drug delivery systems.

Another objective of the present invention is to provide an LNP formulation with organ targeting, particularly lymph node targeting.

In the first aspect of the present invention, is provided an ionizable lipid, or a pharmaceutically acceptable salt, tautomer, or stereoisomer thereof, wherein the ionizable lipid has a structure shown in the following Formula I: 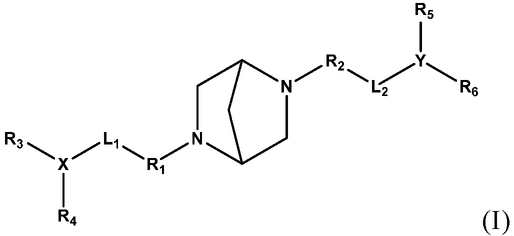 wherein
R₁ and R₂ are each independently selected from -(CH₂)n-, wherein n is a positive integer from 1 to 14;
X and Y are each independently -CH- or N;
L₁ and L₂ are each independently a divalent linking group or absent;
R₃, R₄, R₅, and R₆ are each independently H, CH₃, C2-C30 hydrocarbyl (such as C2-C30 alkyl, C2-C30 alkene, C2-C30 alkynyl), or -(CH₂)s-R^{a}-(CH₂)g-R^{b}-(CH₂)m-R^{c}, wherein s and g are each independently selected from a positive integer of 1-20, m is selected from an integer of 0-20, preferably s+g+m is 2-35; R^{c} is CH₃ or C2-C15 hydrocarbyl (such as C2-C15 alkyl, C2-C15 alkene, C2-C15 alkynyl);
R^{a} and R^{b} are each independently selected from -CH₂-, C2-C6 alkenyl structure or a functional group as shown below: 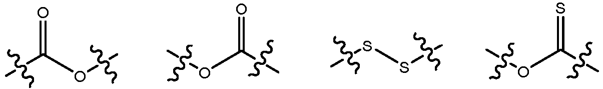 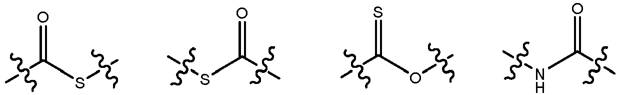 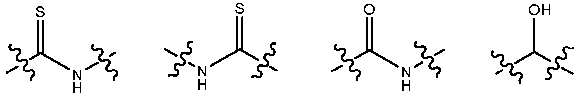
wherein 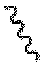 represents a linking bond;
and R₃ and R₄ are not simultaneously H, R₅ and R₆ are not simultaneously H.

In another preferred embodiment, in Formula I,
R₁ and R₂ are each independently selected from -(CH₂)n-, where n is a positive integer from 1 to 14;
X and Y are each independently -CH- or N;
L₁ and L₂ are each independently a divalent linking group or absent;
R₃, R₄, R₅, and R₆ are each independently H, CH₃, C2-C30 hydrocarbyl (such as C2-C30 alkyl, C2-C30 alkene, C2-C30 alkynyl), or -(CH₂)s-R^{a}-(CH₂)g-R^{b}-(CH₂)m-CH₃, wherein s and g are each independently selected from a positive integer of 1-20, m is selected from an integer of 0-20, preferably s+g+m is 2-35;
R^{a} and R^{b} are each independently selected from functional groups as shown below: 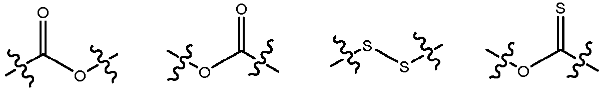 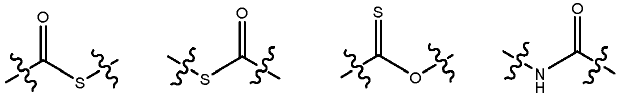 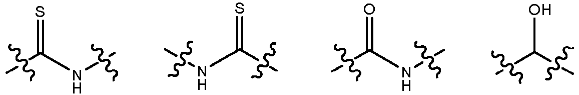
wherein 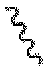 represents a linking bond;
and R₃ and R₄ are not simultaneously H, R₅ and R₆ are not simultaneously H.

In another preferred embodiment, R₃, R₄, R₅, and R₆ are each independently a C4-C30 hydrocarbyl (such as C4-C30 alkyl, C4-C30 alkene, C4-C30 alkynyl), preferably a C4-C20 hydrocarbyl (such as C4-C20 alkyl, C4-C20 alkene, C4-C20 alkynyl).

In another preferred embodiment, at least 2, 3, or 4 of R₃, R₄, R₅, and R₆ are C2-C30 hydrocarbyl (such as C2-C30 alkyl, C2-C30 alkene, C2-C30 alkynyl), or - (CH₂)s-R^{a}-(CH₂)g-R^{b}-(CH₂)m-R^{c}, wherein s, g, m, R^{a}, R^{b}, and R^{c} are as defined above. In another preferred embodiment, at least 2, 3, or 4 of R₃, R₄, R₅, and R₆ are C2-C30 hydrocarbyl (such as C2-C30 alkyl, C2-C30 alkene, C2-C30 alkynyl), or - (CH₂)s-R^{a}-(CH₂)g-R^{b}-(CH₂)m-CH₃, wherein s, g, m, R^{a}, and R^{b} are as defined above. In another preferred embodiment, s+g+m is 3-20, preferably 4-15.

In another preferred embodiment, R₃ has a structure of -R₃ₐ-R_{3b}-R_{3c}-R_{3d}-R₃ₑ,
wherein R₃ₐ and R_{3c} are each independently -(CH₂)n-, wherein n is a positive integer selected from 1-14;
R_{3b} and R_{3d} are each independently selected from the following functional groups: -CH₂-, -(C=O)O-, -O(C=O)-, -(S-S)-, -O(S=O)-, -(C=O)S-, -S(C=O)-, - (C=S)O-, -NH(C=O)-, -(C=S)NH-, -NH(C=S)-, -(C=O)NH-, -CH(OH)-, -(C=C)-(CH₂)-(C=C)-, -(C=C)- or -(C≡C)-, preferably -CH₂-, -(C=O)O-, -O(C=O)-, -(S-S)-, -(C=C)-(CH₂)-(C=C)- or -CH(OH)-;
R₃ₑ is a C2-C20 hydrocarbyl.

In another preferred embodiment, R₄ has a structure of -R₄ₐ-R_{4b}-R_{4c}-R_{4d}-R₄ₑ,
wherein R₄ₐ and R_{4c} are each independently -(CH₂)n-, where n is a positive integer selected from 1-14;
R_{4b} and R_{4d} are each independently selected from the following functional groups: -CH₂-, -(C=O)O-, -O(C=O)-, -(S-S)-, -O(S=O)-, -(C=O)S-, -S(C=O)-, - (C=S)O-, -NH(C=O)-, -(C=S)NH-, -NH(C=S)-, -(C=O)NH-, -CH(OH)-, -(C=C)-(CH₂)-(C=C)-, -(C=C)- or -(C≡C)-, preferably -CH₂-, -(C=O)O-, -O(C=O)-, -(S-S)-, -(C=C)-(CH₂)-(C=C)- or -CH(OH)-;
R₄ₑ is a C2-C20 hydrocarbyl.

In another preferred embodiment, R₅ has a structure of -R₅ₐ-R_{5b}-R_{5c}-R_{5d}-R₅ₑ,
wherein R₅ₐ and R_{5c} are each independently -(CH₂)n-, where n is a positive integer selected from 1-14;
R_{5b} and R_{5d} are each independently selected from the following functional groups: -CH₂-, -(C=O)O-, -O(C=O)-, -(S-S)-, -O(S=O)-, -(C=O)S-, -S(C=O)-, - (C=S)O-, -NH(C=O)-, -(C=S)NH-, -NH(C=S)-, -(C=O)NH-, -CH(OH)-, -(C=C)-(CH₂)-(C=C)-, -(C=C)- or -(C≡C)-, preferably -CH₂-, -(C=O)O-, -O(C=O)-, -(S-S)-, -(C=C)-(CH₂)-(C=C)- or -CH(OH)-;
R₅ₑ is a C2-C20 hydrocarbyl.

In another preferred embodiment, R₆ has a structure of -R₆ₐ-R_{6b}-R_{6c}-R_{6d}-R₆ₑ,
wherein R₆ₐ and R_{6c} are each independently -(CH₂)n-, where n is a positive integer selected from 1-14;
R_{6b} and R_{6d} are each independently selected from the following functional groups: -CH₂-, -(C=O)O-, -O(C=O)-, -(S-S)-, -O(S=O)-, -(C=O)S-, -S(C=O)-, - (C=S)O-, -NH(C=O)-, -(C=S)NH-, -NH(C=S)-, -(C=O)NH-, -CH(OH)-, -(C=C)-(CH₂)-(C=C)-, -(C=C)- or -(C≡C)-, preferably -CH₂-, -(C=O)O-, -O(C=O)-, -(S-S)-, -(C=C)-(CH₂)-(C=C)- or -CH(OH)-;
R₆ₑ is a C2-C20 hydrocarbyl.

In another preferred embodiment, L₁ has a structure of -(L₁ₐ-L_{1b}-L_{1c})- from right to left, wherein L_{1b} is -(CH₂)n-, where n is a positive integer selected from 1-14; L₁ₐ and L_{1c} are each independently selected from the following functional groups: -CH₂-, -(C=O)O-, -O(C=O)-, -(S-S)-, -O(S=O)-, -(C=O)S-, -S(C=O)-, -(C=S)O-, - NH(C=O)-, -(C=S)NH-, -NH(C=S)-, -(C=O)NH- or -CH(OH)-, preferably -CH₂-, - (C=O)O-, -O(C=O)-, -(S-S)- or -CH(OH)-.

In another preferred embodiment, L₂ has a structure of -(L₂ₐ-L_{2b}-L_{2c})- from left to right;
wherein L_{2b} is -(CH₂)n-, where n is a positive integer selected from 1-14; L₂ₐ and L_{2c} are each independently selected from the following functional groups: -CH₂-, -NH-, -(C=O)O-, -O(C=O)-, -(S-S)-, -O(S=O)-, -(C=O)S-, -S(C=O)-, -(C=S)O-, - NH(C=O)-, -(C=S)NH-, -NH(C=S)-, -(C=O)NH-, -CH(OH)-, -(C=C)-(CH₂)-(C=C)-, -(C=C)- or -(C≡C)-, preferably -CH₂-, -(C=O)O-, -O(C=O)-, -(S-S)-, -(C=C)-(CH₂)-(C=C)- or -CH(OH)-.

In another preferred embodiment, X and Y are -CH-.

In another preferred embodiment, the ionizable lipid has a sub-structure as shown in Formula (I-1): 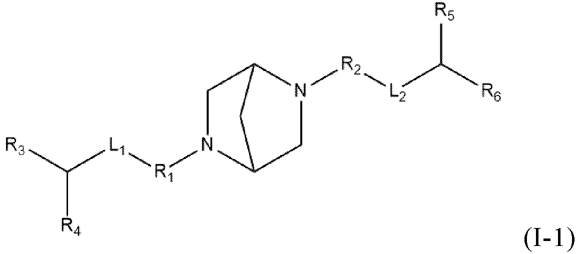 wherein
R₁ and R₂ are each independently selected from -(CH₂)n-, wherein n is a positive integer from 1 to 14;
L₁ has a structure of -(L₁ₐ-L_{1b}-L_{1c})- from right to left, wherein L_{1b} is -(CH₂)n-, wherein n is a positive integer selected from 1-14; L₁ₐ and L_{1c} are each independently selected from the following functional groups: -CH₂-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, - (C=O)NH- or -CH(OH)-, preferably -CH₂-, - (C=O)O-, -O(C=O)-, -(S-S)- or - CH(OH)-;
L₂ has a structure of -(L₂ₐ-L_{2b}-L_{2c})- from left to right, wherein L_{2b} is -(CH₂)n-, wherein n is a positive integer selected from 1-14; L₂ₐ and L_{2c} are each independently selected from the following functional groups: -CH₂-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, - (C=O)NH- or -CH(OH)-, preferably -CH₂-, - (C=O)O-, -O(C=O)-, -(S-S)- or - CH(OH)-;
R₃, R₄, R₅, and R₆ are each independently a C2-C20 hydrocarbyl.

In another preferred embodiment, X is N and Y is -CH-.

In another preferred embodiment, L₁ is absent.

In another preferred embodiment, R₅ is H.

In another preferred embodiment, the ionizable lipid has a sub-structure as shown in Formula (I-2): 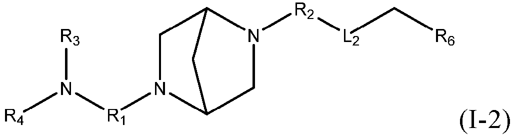 wherein
R₁ and R₂ are each independently selected from -(CH₂)n-, wherein n is a positive integer from 1 to 14;
L₂ has a structure of -(L₂ₐ-L_{2b}-L_{2c})- from left to right,
wherein L_{2b} is -(CH₂)n-, wherein n is a positive integer selected from 1-14; L₂ₐ and L_{2c} are each independently selected from the following functional groups: -CH₂-, -NH-, -(C=O)O-, -O(C=O)-, -(S-S)-, -O(S=O)-, -(C=O)S-, -S(C=O)-, -(C=S)O-, - NH(C=O)-, -(C=S)NH-, -NH(C=S)-, -(C=O)NH-, -CH(OH)-, -(C=C)-(CH₂)-(C=C)-, -(C=C)- or -(C≡C)-, preferably -CH₂-, -NH-, -(C=O)O-, -O(C=O)-, -(S-S)-, -(C=C)-(CH₂)-(C=C)- or -CH(OH)-;
R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ;
wherein R₃ₐ, R_{3c}, R₄ₐ and R_{4c} are each independently -(CH₂)n-, wherein n is a positive integer selected from 1-14;
R_{3b}, R_{3d}, R_{4b} and R_{4d} are each independently selected from the following functional groups: -CH₂-, -(C=O)O-, -O(C=O)-, -(S-S)-, -O(S=O)-, -(C=O)S-, - S(C=O)-, -(C=S)O-, -NH(C=O)-, -(C=S)NH-, -NH(C=S)-, -(C=O)NH-, -CH(OH)-, - (C=C)-(CH₂)-(C=C)-, -(C=C)- or -(C≡C)-, preferably -CH₂-, -(C=O)O-, -O(C=O)-, - (S-S)-, -(C=C)-(CH₂)-(C=C)- or -CH(OH)-;
R₃ₑ and R₄ₑ are each independently a C2-C20 hydrocarbyl;
R₆ is a C2-C20 hydrocarbyl.

In another preferred embodiment, X and Y are N.

In another preferred embodiment, L₁ and L₂ are absent.

In another preferred embodiment, the ionizable lipid has a sub-structure as shown in Formula (I-3): 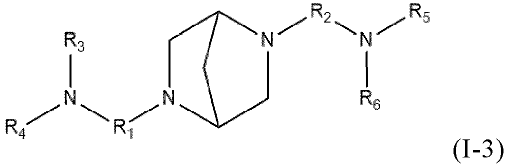 wherein
R₁ and R₂ are each independently selected from -(CH₂)n-, wherein n is a positive integer from 1 to 14;
R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ; R₅ has a structure of -R₅ₐ-R_{5b}-R_{5c}-R_{5d}-R₅ₑ, and R₆ has a structure of -R₆ₐ-R_{6b}-R_{6c}-R_{6d}-R₆ₑ;
wherein R₃ₐ, R_{3c}, R₄ₐ, R_{4c}, R₅ₐ, R_{5c}, R₆ₐ and R_{6c} are each independently -(CH₂)n-, wherein n is a positive integer selected from 1-14;
R_{3b}, R_{3d}, R_{4b}, R_{4d}, R_{5b}, R_{5d}, R_{6b} and R_{6d} are each independently selected from the following functional groups: -CH₂-, -(C=O)O-, -O(C=O)-, -(S-S)-, -O(S=O)-, - (C=O)S-, -S(C=O)-, -(C=S)O-, -NH(C=O)-, -(C=S)NH-, -NH(C=S)-, -(C=O)NH-, - CH(OH)-, -(C=C)-(CH₂)-(C=C)-, -(C=C)-, -(C≡C)-, preferably -CH₂-, -(C=O)O-, - O(C=O)-, -(S-S)-, -(C=C)-(CH₂)-(C=C)-, -CH(OH)-;
R₃ₑ, R₄ₑ, R₅ₑ and R₆ₑ are each independently a C2-C20 hydrocarbyl.

In another preferred embodiment, the ionizable lipid has a structure as shown in Formula Ia: 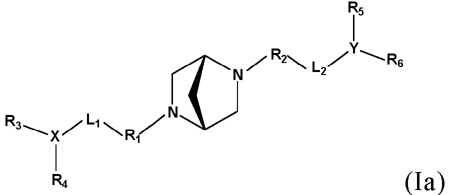 wherein each functional group is as described above.

In another preferred embodiment, the ionizable lipid has a structure selected from Table 1:

**Table 1**

| Serial numb er | Structural formula |
|---|---|
| AL-1 | 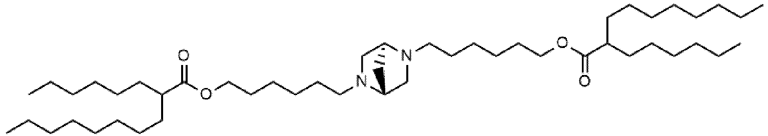 |
| AL-2 | 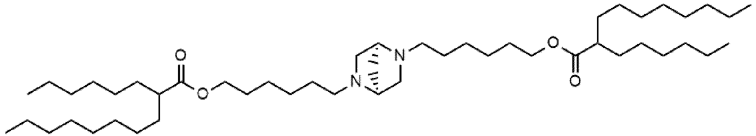 |
| AL-3 | 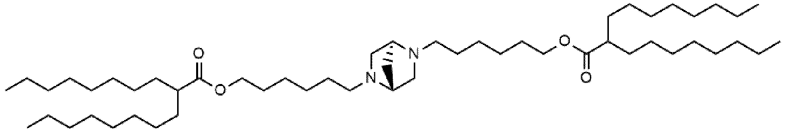 |
| AL-4 | 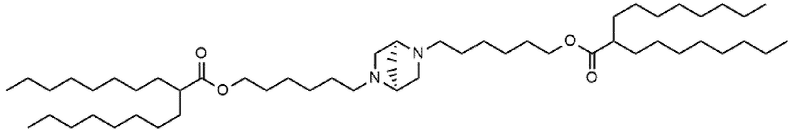 |
| AL-5 | 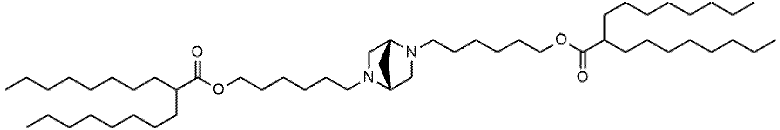 |
| AL-6 | 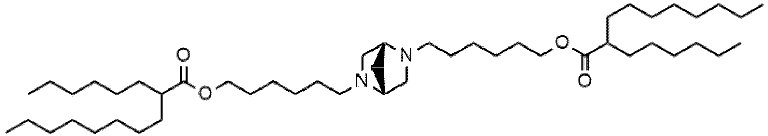 |
| AL-7 | 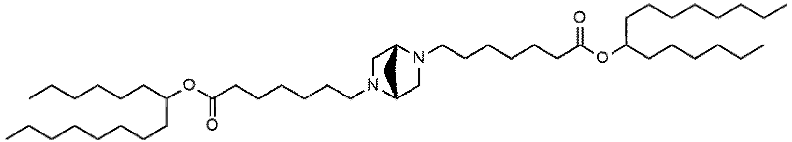 |
| AL-8 | 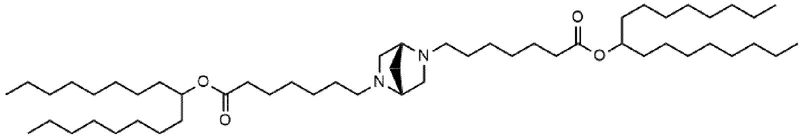 |
| AL-9 | 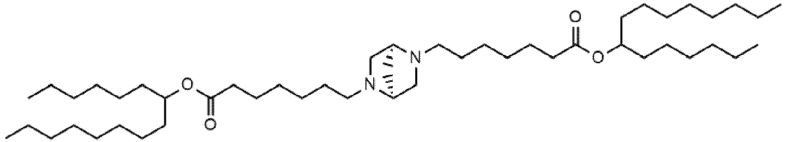 |
| AL-10 | 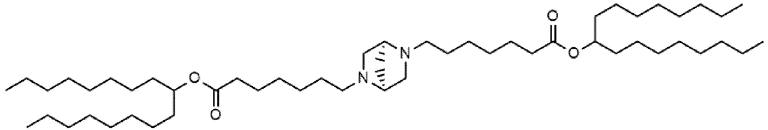 |
| AL-11 | 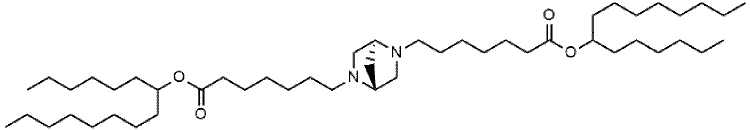 |
| AL-12 | 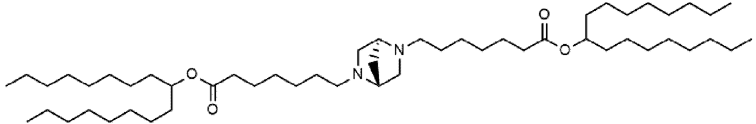 |
| AL-13 | 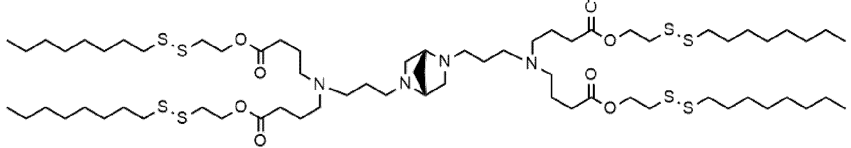 |
| AL-14 | 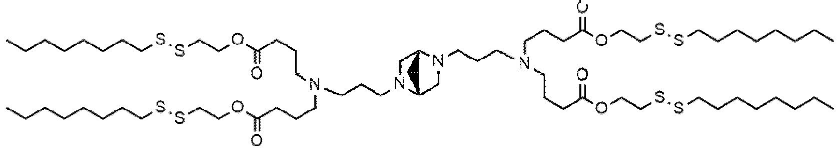 |
| AL-15 | 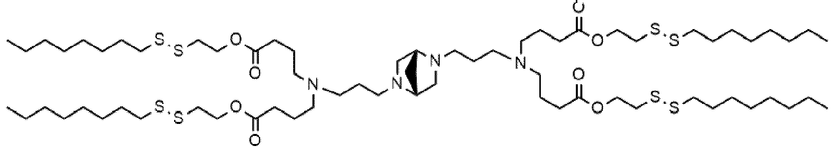 |
| AL-16 | 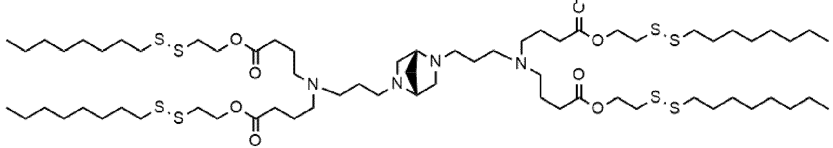 |
| AL-17 | 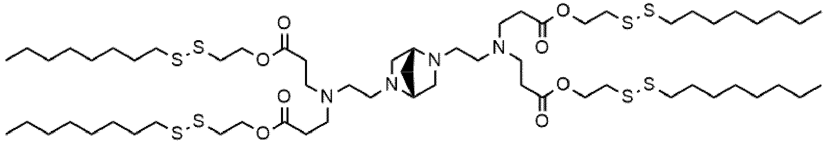 |
| AL-18 | 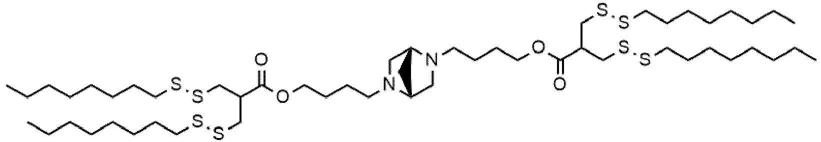 |
| AL-19 | 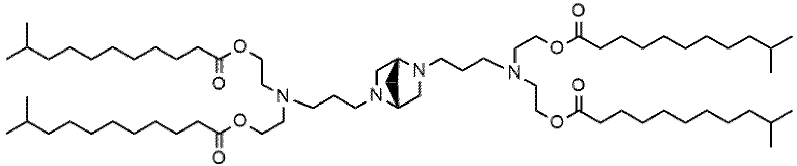 |
| AL-20 | 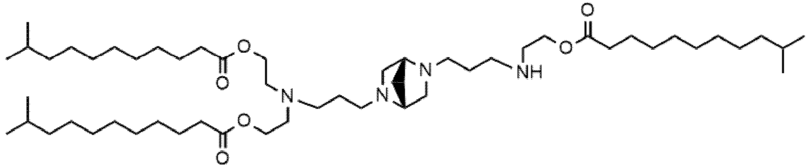 |
| AL-21 | 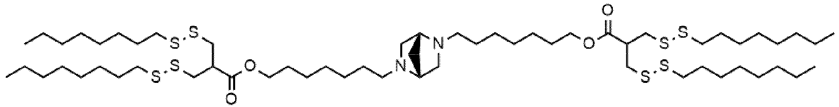 |
| AL-22 | 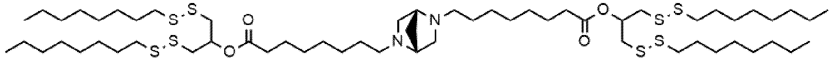 |
| AL-23 | 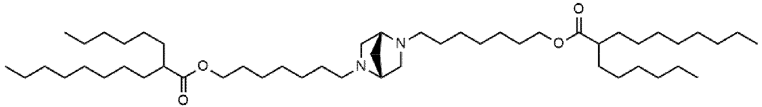 |
| AL-24 | 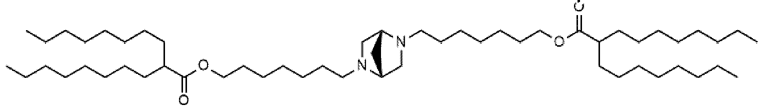 |
| AL-25 | 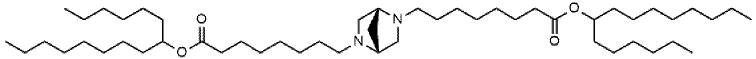 |
| AL-26 | 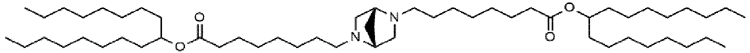 |
| AL-27 | 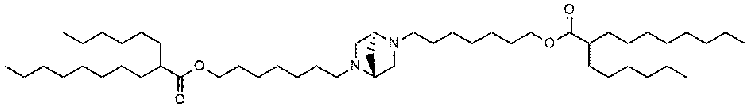 |
| AL-28 | 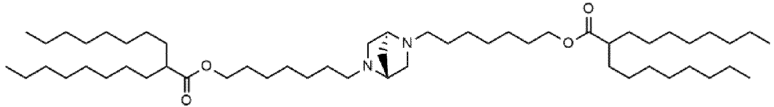 |
| AL-29 | 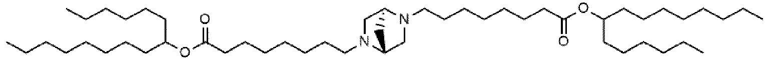 |
| AL-30 | 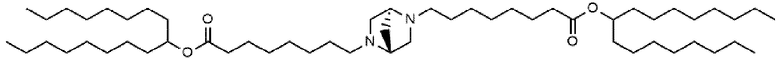 |
| AL-31 | 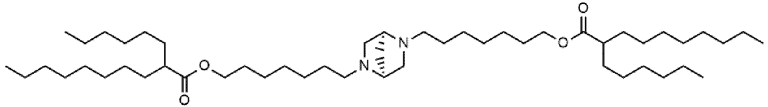 |
| AL-32 | 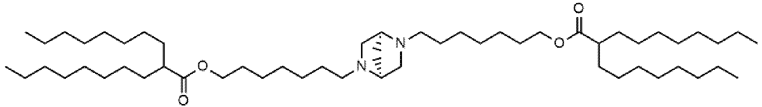 |
| AL-33 | 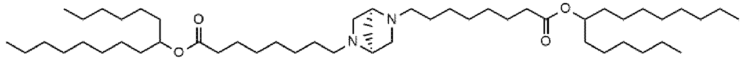 |
| AL-34 | 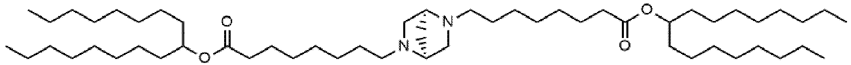 |
| AL-35 | 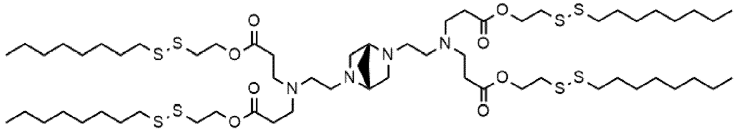 |
| AL-36 | 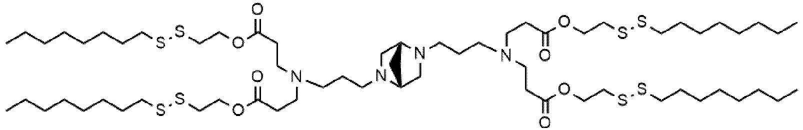 |
| AL-37 | 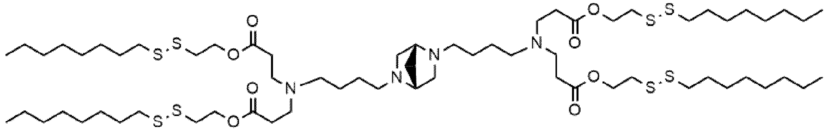 |
| AL-38 | 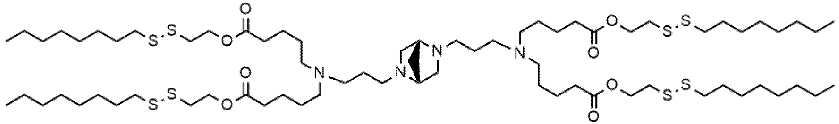 |
| AL-39 | 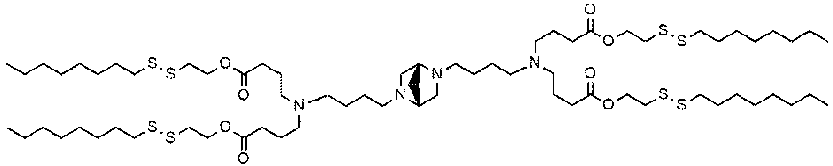 |
| AL-40 | 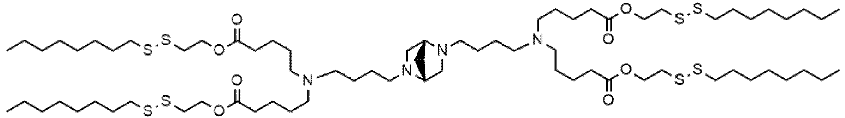 |
| AL-41 | 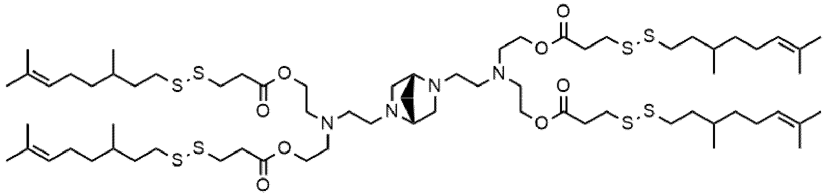 |
| AL-42 | 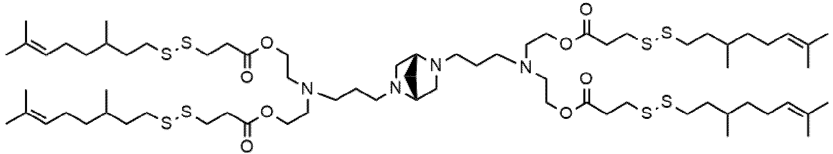 |
| AL-43 | 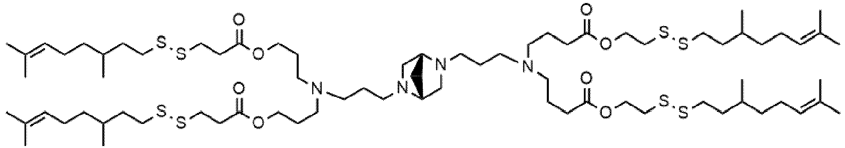 |
| AL-44 | 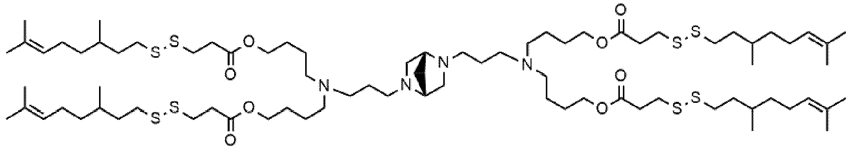 |
| AL-45 | 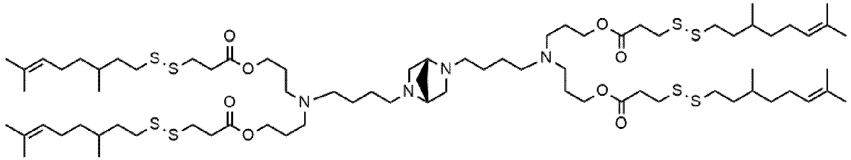 |
| AL-46 | 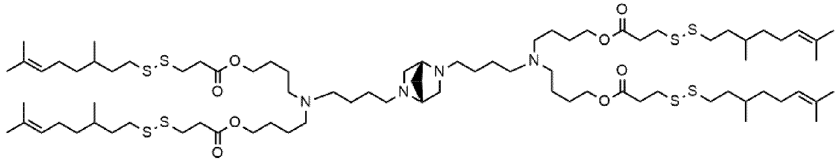 |
| AL-47 | 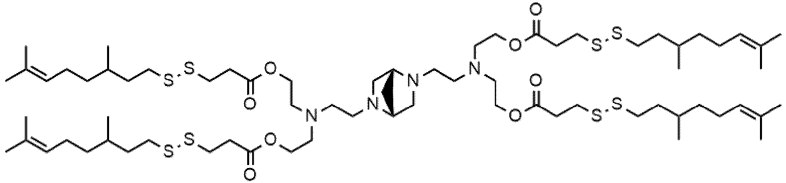 |
| AL-48 | 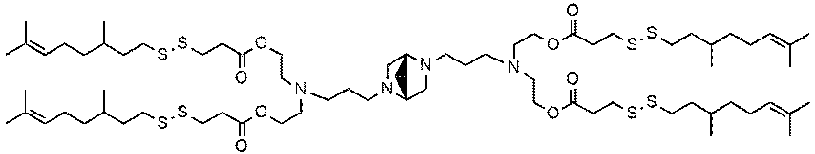 |
| AL-49 | 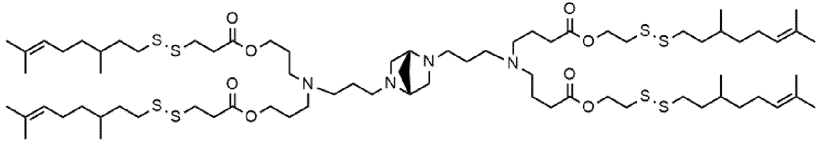 |
| AL-50 | 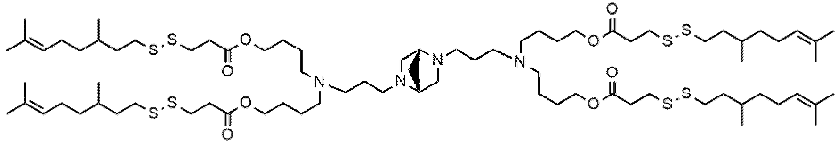 |
| AL-51 | 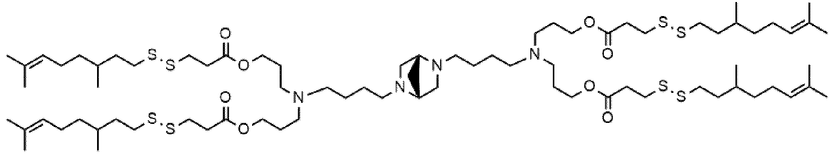 |
| AL-52 | 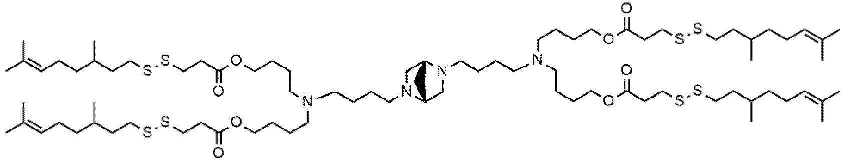 |
| AL-53 | 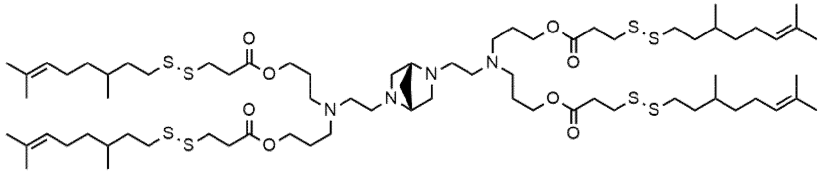 |
| AL-54 | 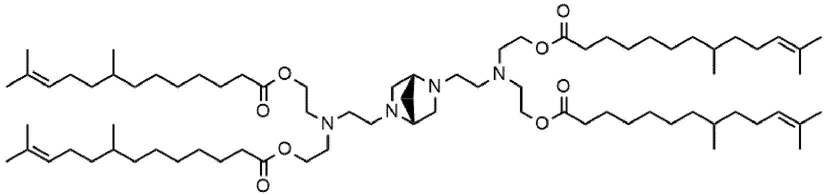 |
| AL-55 | 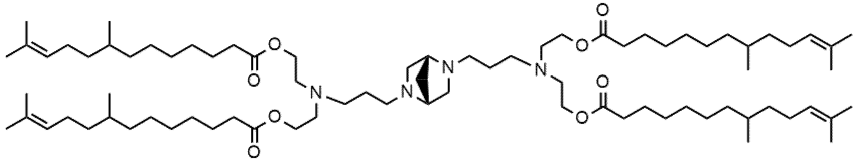 |
| AL-56 | 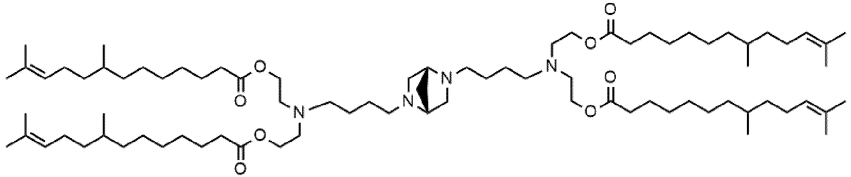 |
| AL-57 | 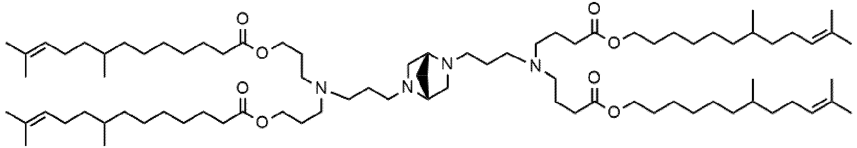 |
| AL-58 | 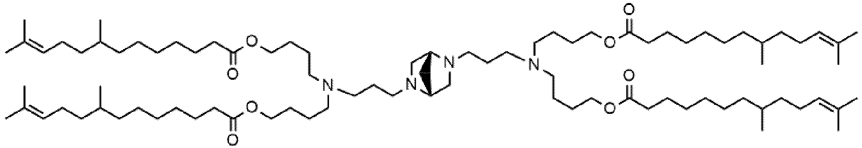 |
| AL-59 | 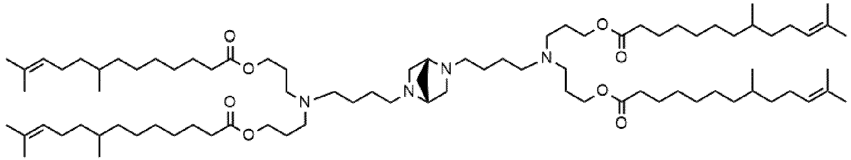 |
| AL-60 | 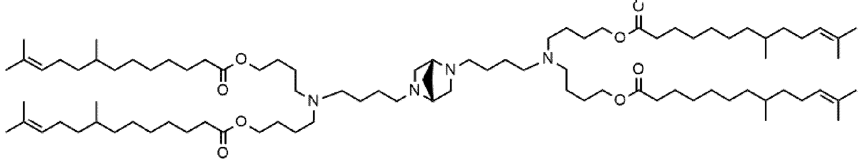 |
| AL-61 | 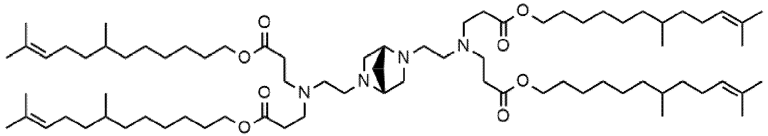 |
| AL-62 | 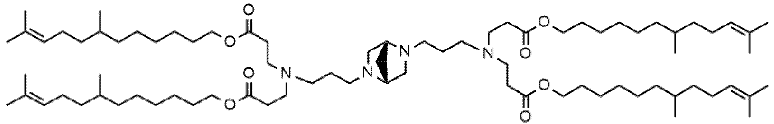 |
| AL-63 | 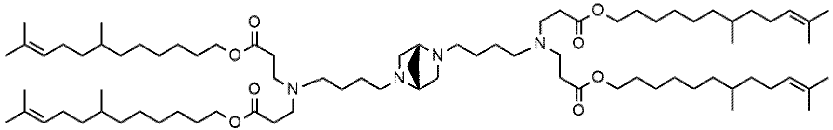 |
| AL-64 | 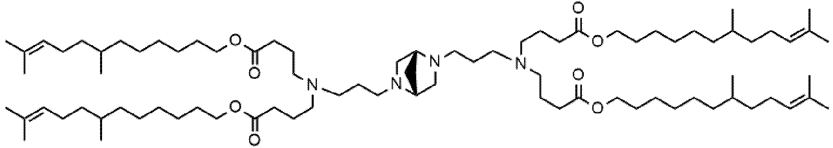 |
| AL-65 | 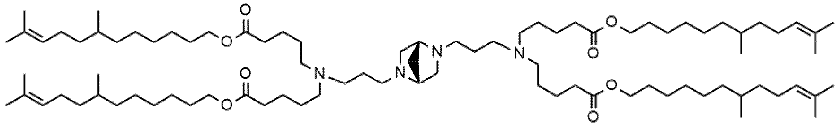 |
| AL-66 | 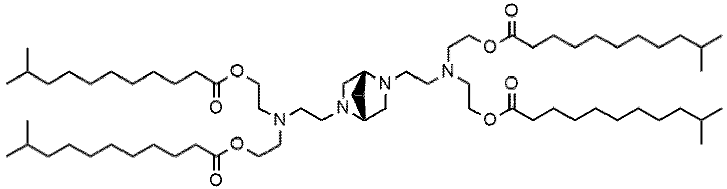 |
| AL-67 | 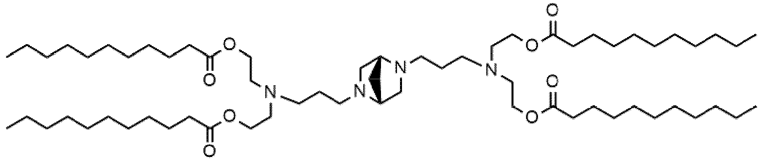 |
| AL-68 | 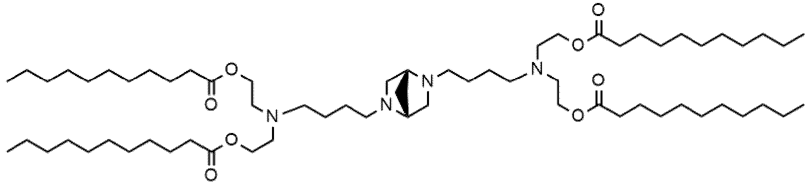 |
| AL-69 | 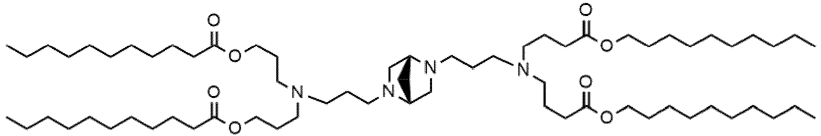 |
| AL-70 | 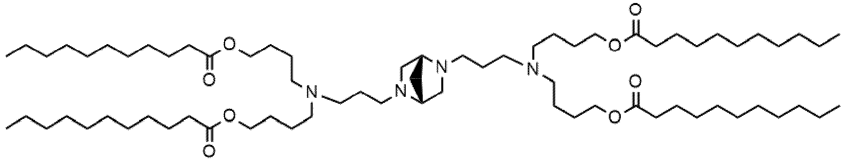 |
| AL-71 | 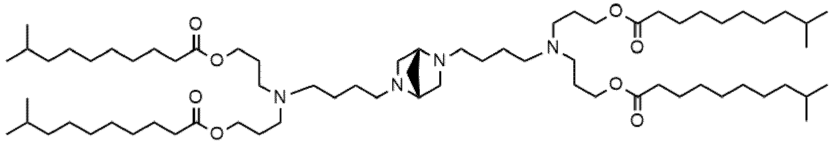 |
| AL-72 | 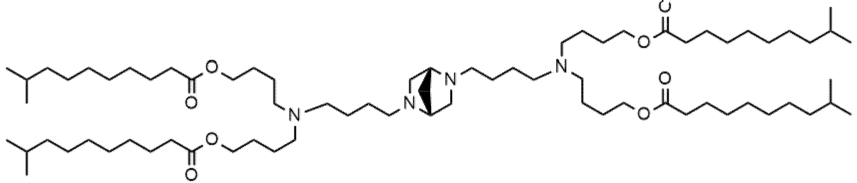 |
| AL-73 | 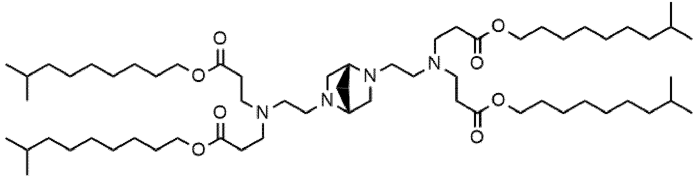 |
| AL-74 | 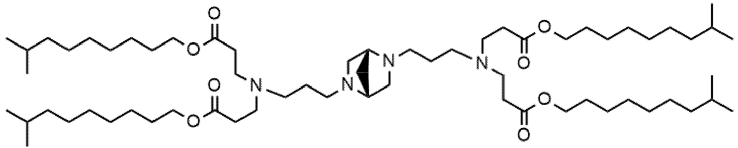 |
| AL-75 | 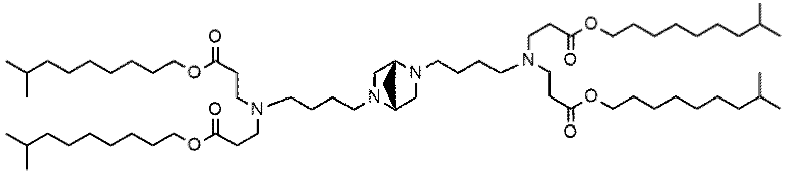 |
| AL-76 | 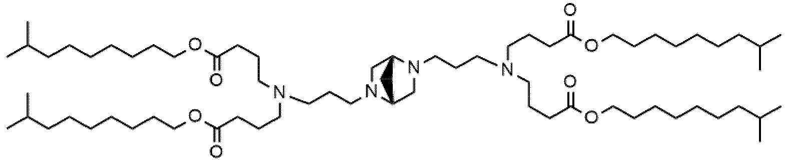 |
| AL-77 | 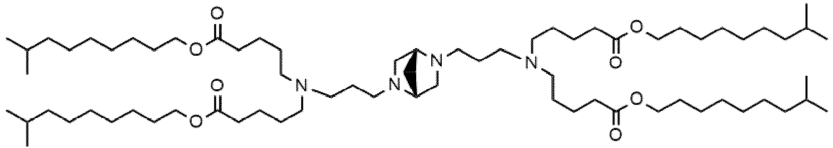 |
| AL-78 | 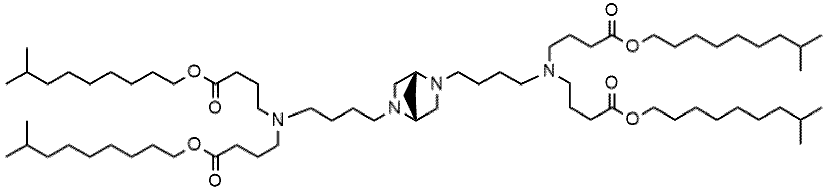 |
| AL-79 | 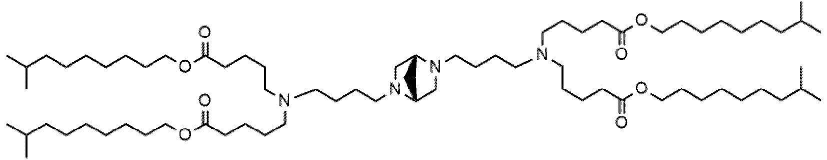 |
| AL-80 | 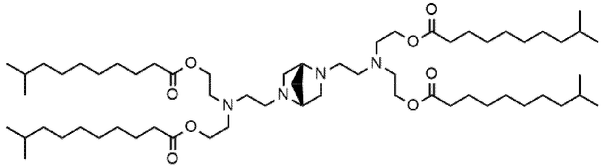 |
| AL-81 | 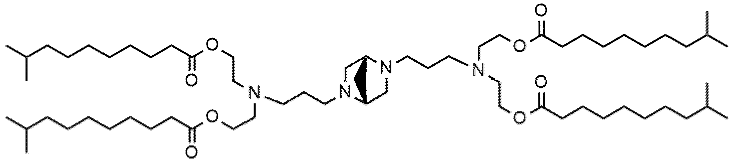 |
| AL-82 | 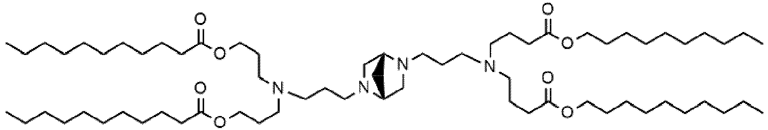 |
| AL-83 | 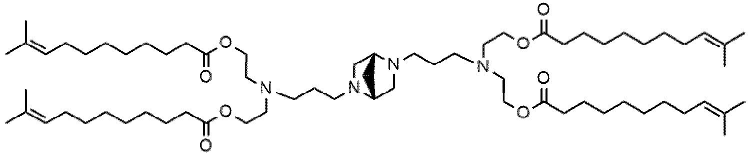 |
| AL-84 | 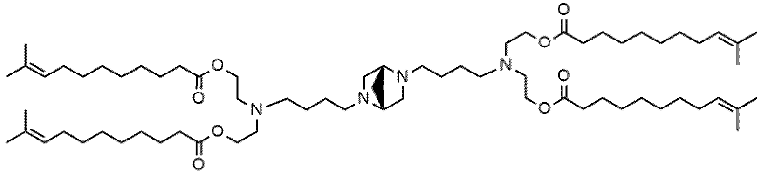 |
| AL-85 | 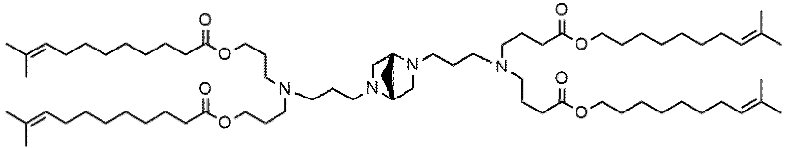 |
| AL-86 | 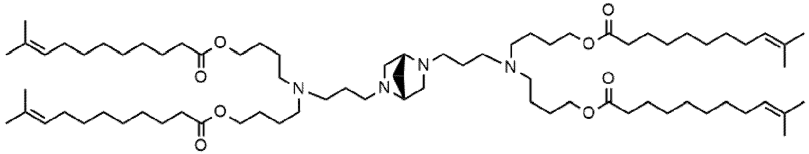 |
| AL-87 | 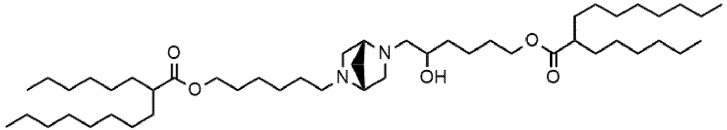 |
| AL-88 | 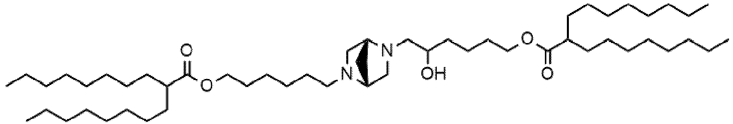 |
| AL-89 | 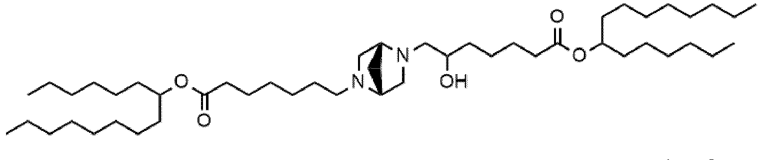 |
| AL-90 | 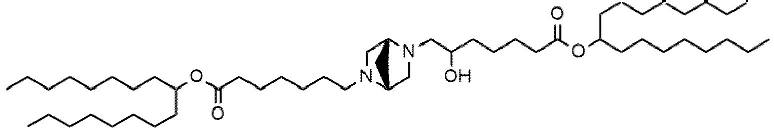 |
| AL-91 | 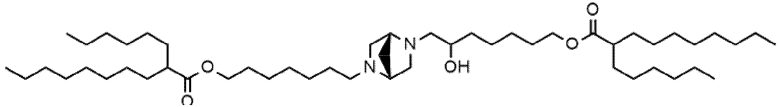 |
| AL-92 | 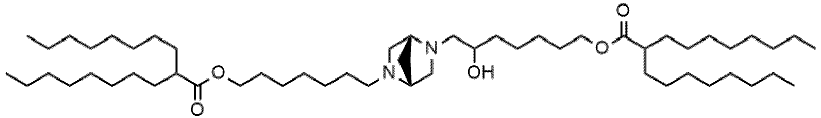 |
| AL-93 | 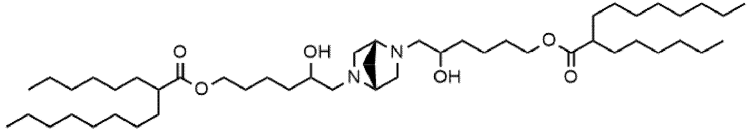 |
| AL-94 | 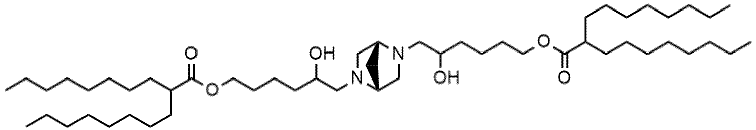 |
| AL-95 | 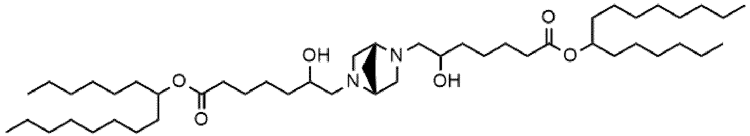 |
| AL-96 | 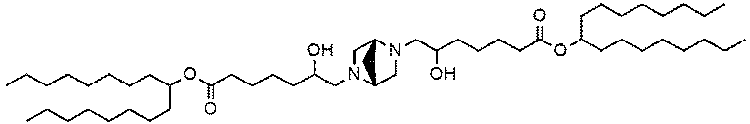 |
| AL-97 | 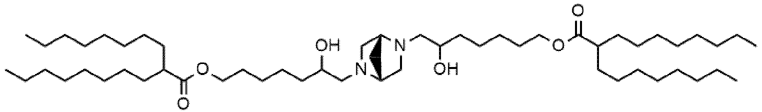 |
| AL-98 | 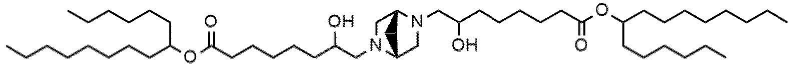 |
| AL-99 | 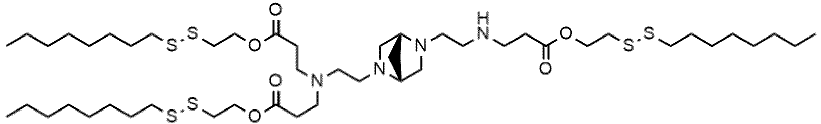 |
| AL-100 | 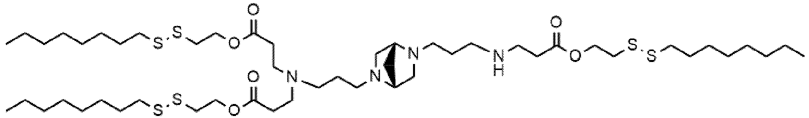 |
| AL-101 | 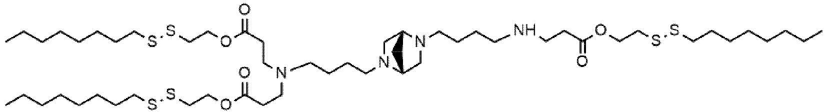 |
| AL-102 | 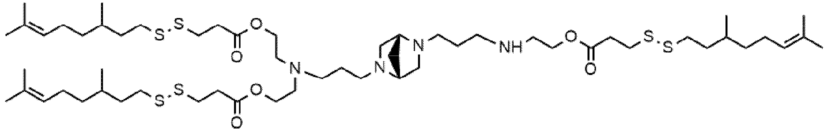 |
| AL-103 | 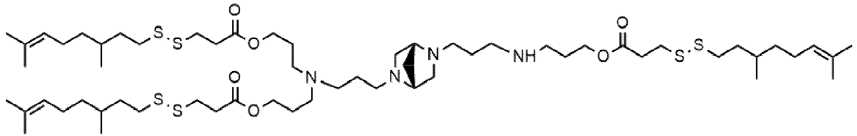 |
| AL-104 | 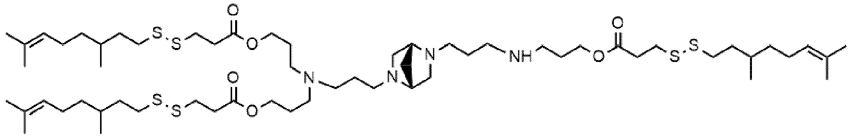 |
| AL-105 | 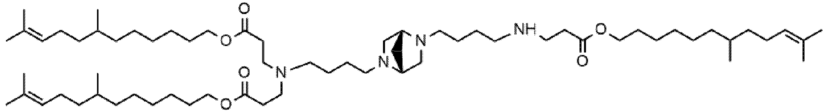 |
| AL-106 | 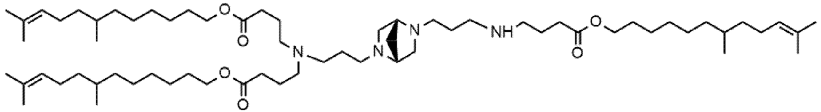 |
| AL-107 | 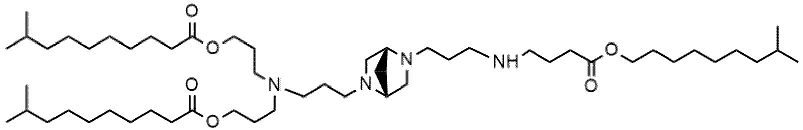 |
| AL-108 | 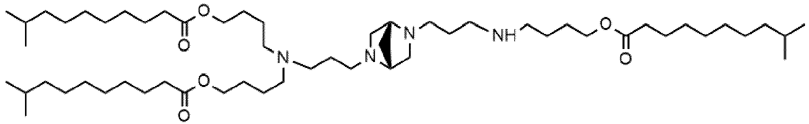 |
| AL-109 | 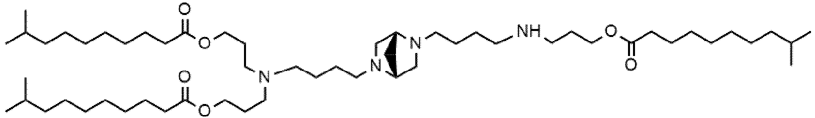 |
| AL-110 | 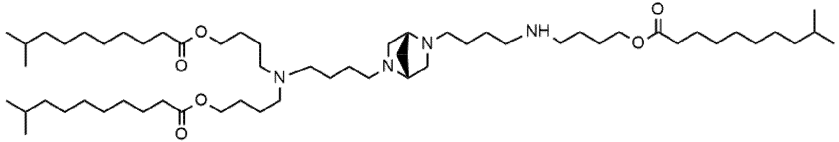 |
| AL-111 | 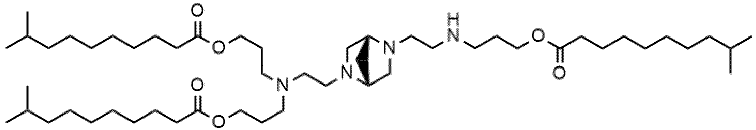 |
| AL-112 | 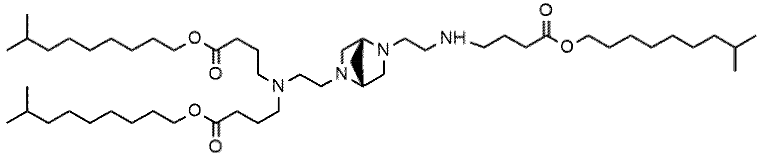 |
| AL-113 | 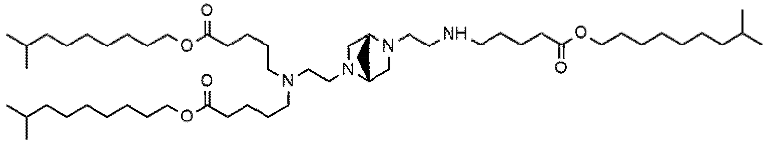 |
| AL-114 | 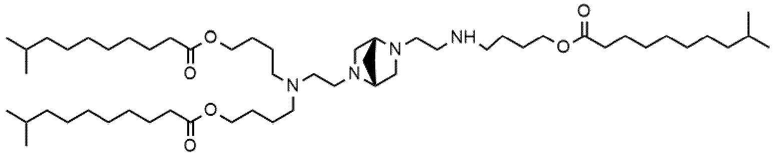 |
| AL-115 | 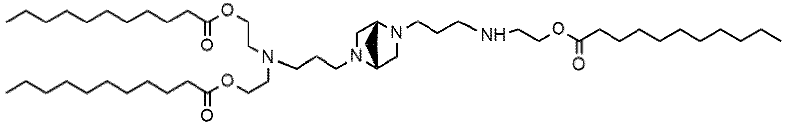 |
| AL-116 | 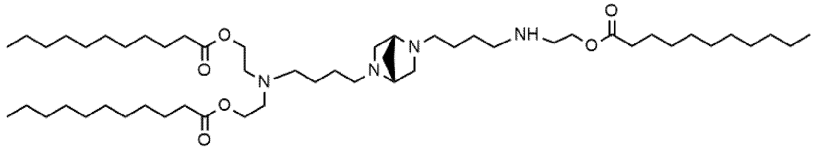 |
| AL-117 | 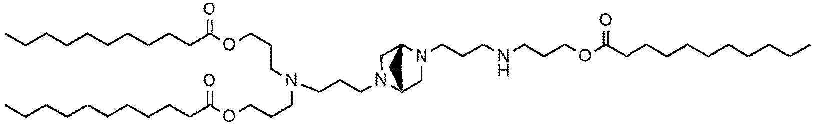 |
| AL-118 | 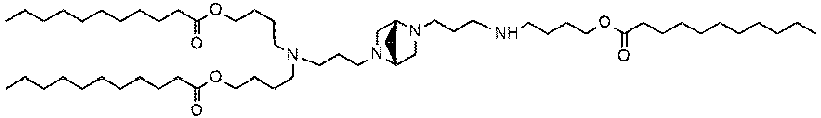 |
| AL-119 | 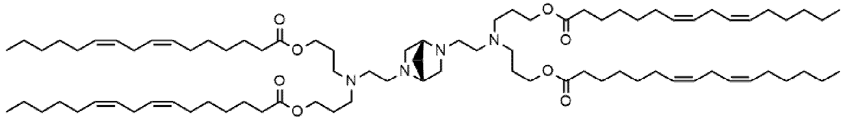 |
| AL-120 | 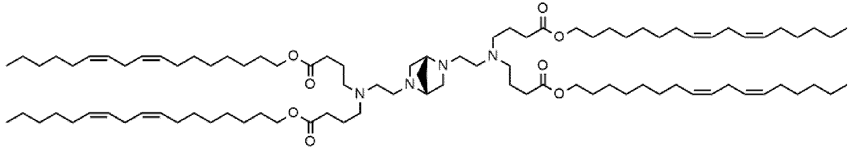 |
| AL-121 | 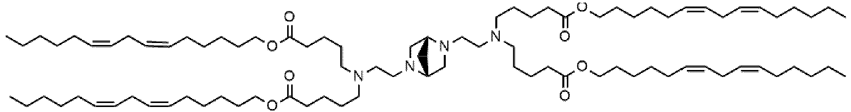 |
| AL-122 | 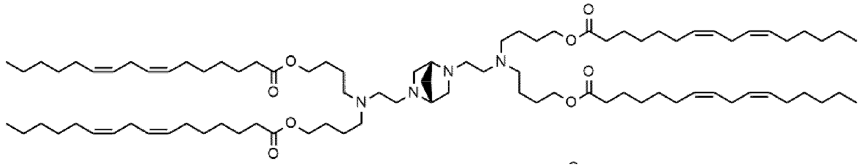 |
| AL-123 | 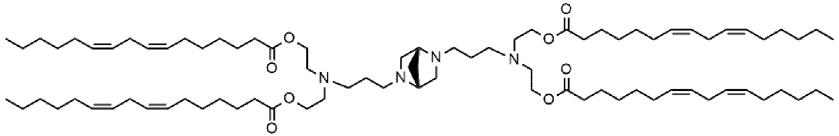 |
| AL-124 | 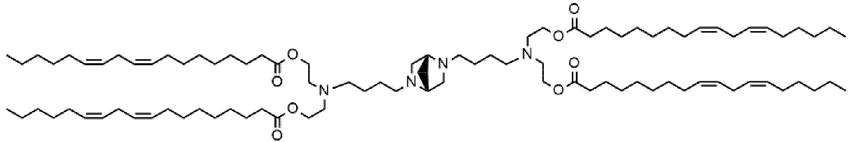 |
| AL-125 | 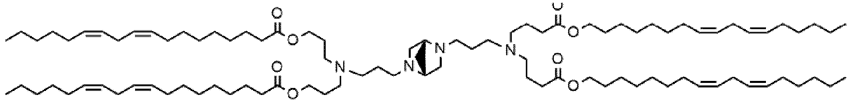 |
| AL-126 | 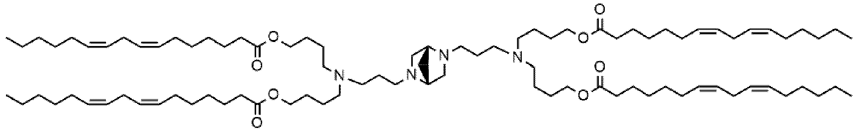 |
| AL-127 | 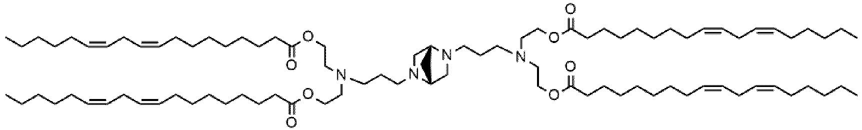 |
| AL-128 | 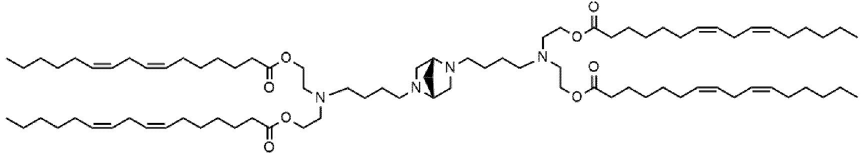 |
| AL-129 | 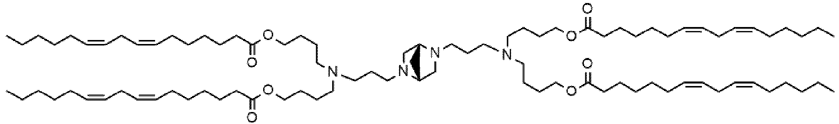 |
| AL-130 | 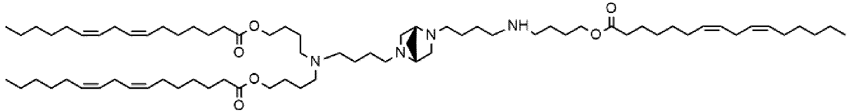 |
| AL-131 | 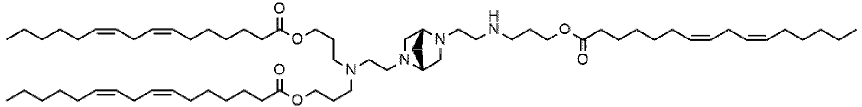 |
| AL-132 | 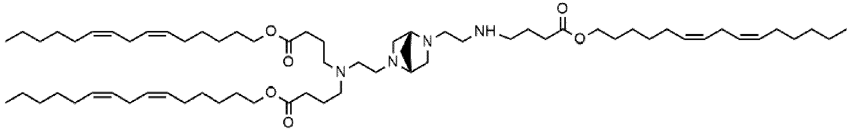 |
| AL-133 | 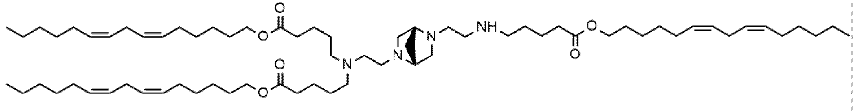 |
| AL-134 | 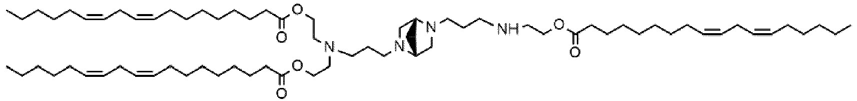 |
| AL-135 | 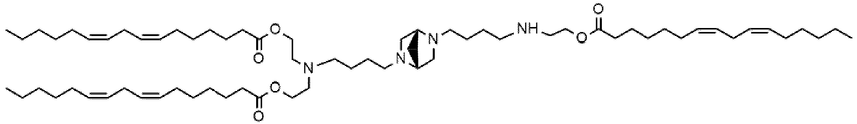 |
| AL-136 | 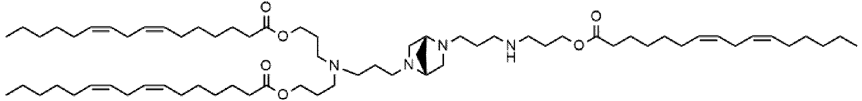 |
| AL-137 | 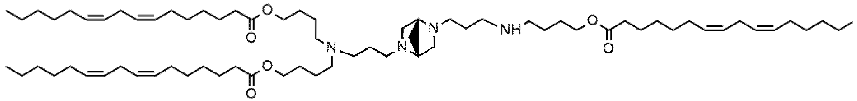 |
| AL-138 | 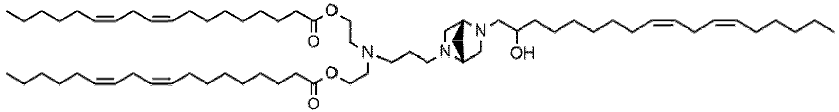 |
| AL-139 | 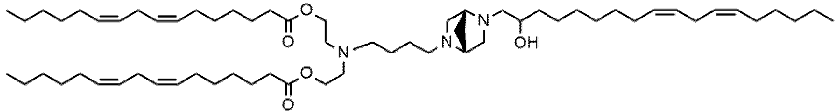 |
| AL-140 | 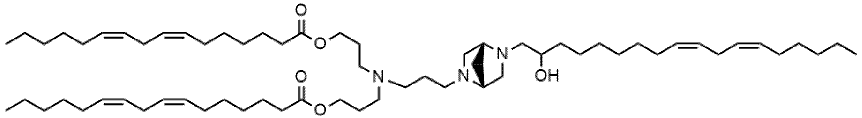 |
| AL-141 | 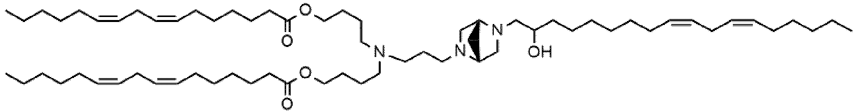 |
| AL-142 | 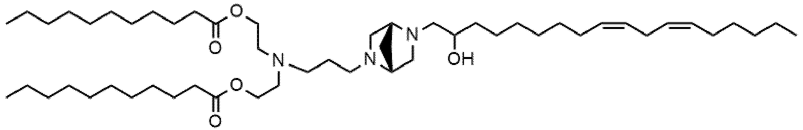 |
| AL-143 | 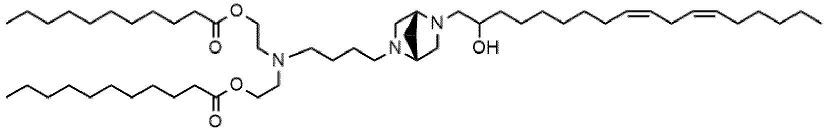 |
| AL-144 | 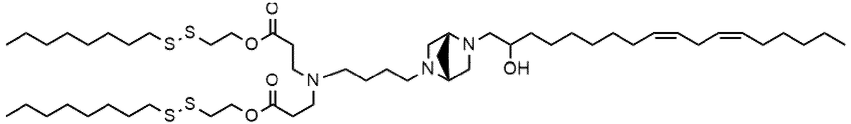 |
| AL-145 | 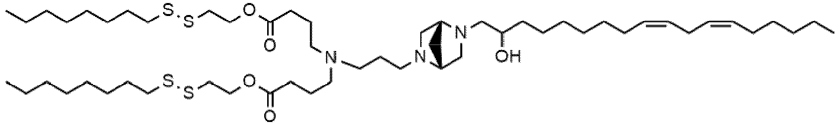 |
| AL-146 | 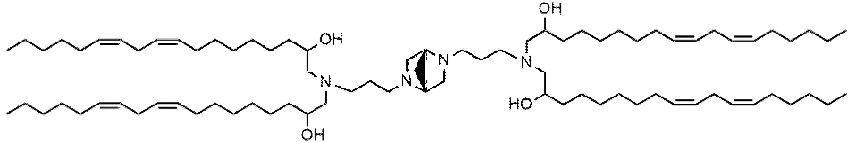 |
| AL-147 | 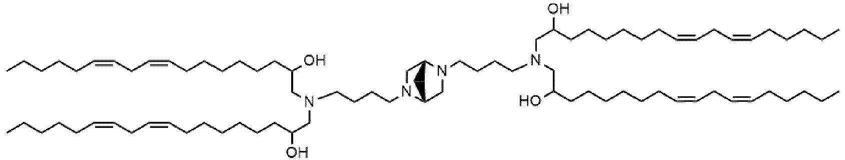 |
| AL-148 | 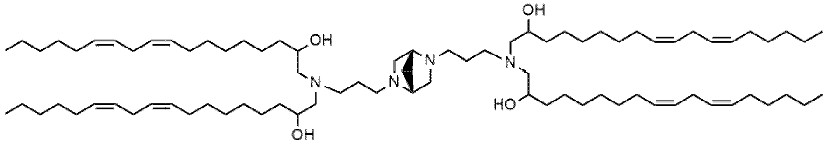 |
| AL-149 | 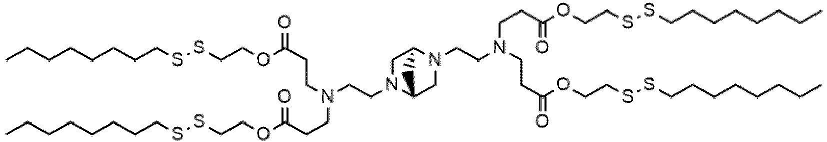 |
| AL-150 | 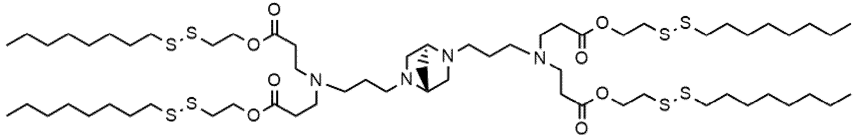 |
| AL-151 | 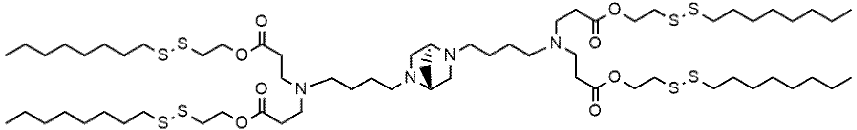 |
| AL-152 | 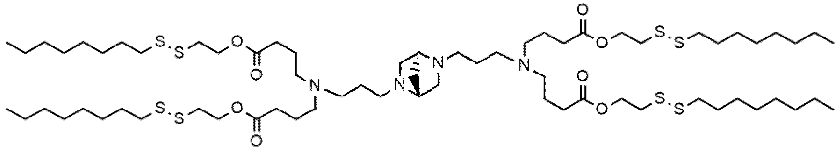 |
| AL-153 | 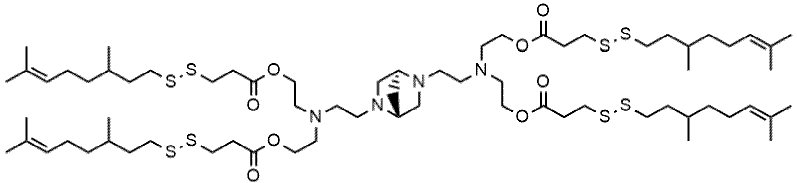 |
| AL-154 | 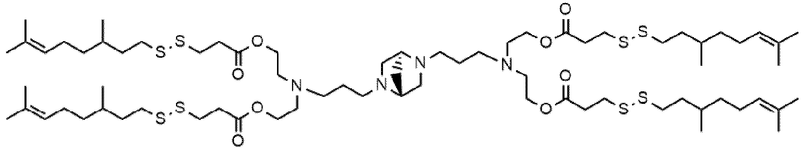 |
| AL-155 | 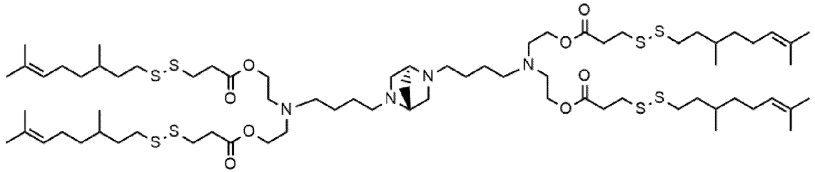 |
| AL-156 | 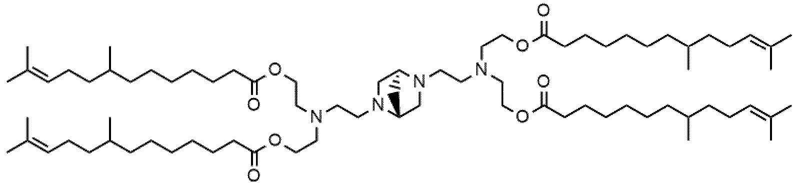 |
| AL-157 | 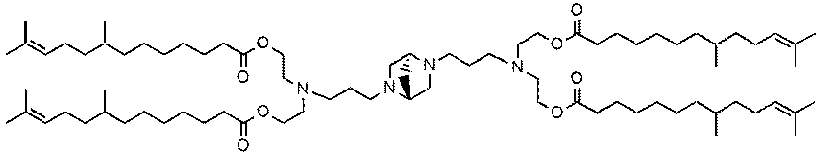 |
| AL-158 | 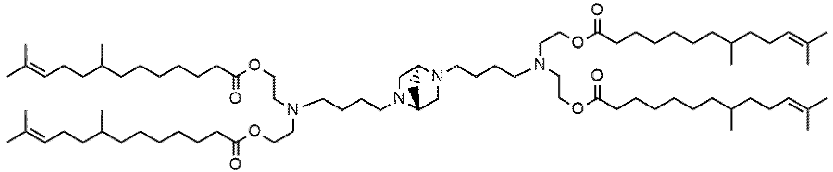 |
| AL-159 | 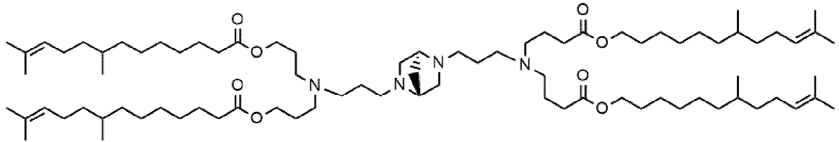 |
| AL-160 | 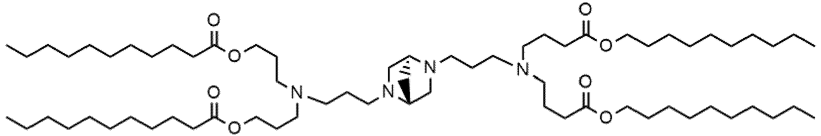 |
| AL-161 | 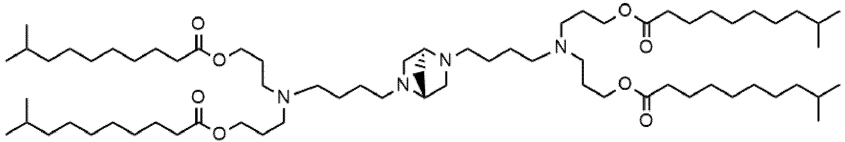 |
| AL-162 | 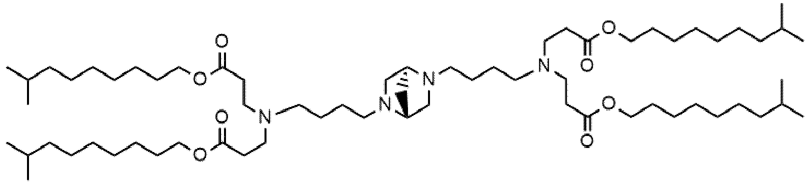 |
| AL-163 | 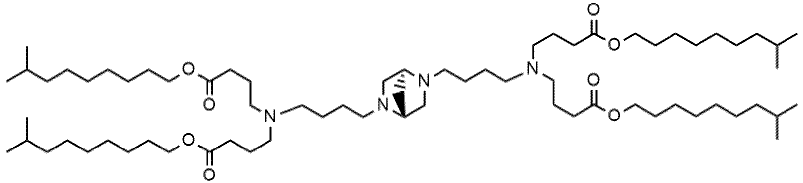 |
| AL-164 | 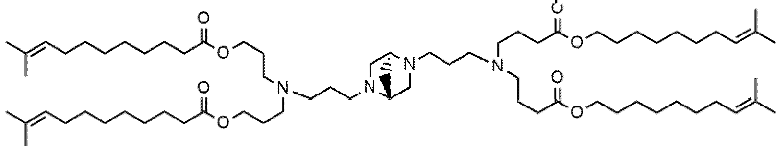 |
| AL-165 | 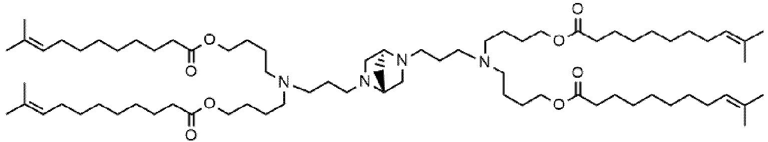 |
| AL-166 | 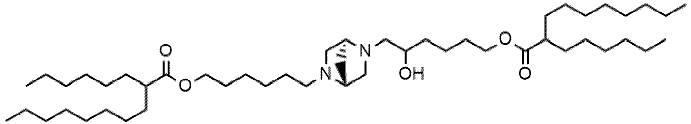 |
| AL-167 | 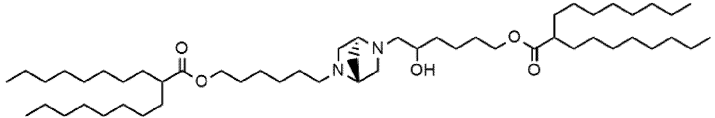 |
| AL-168 | 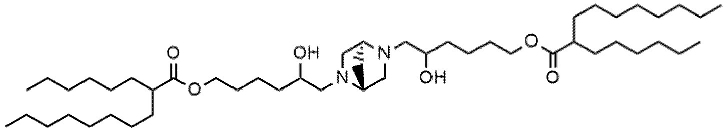 |
| AL-169 | 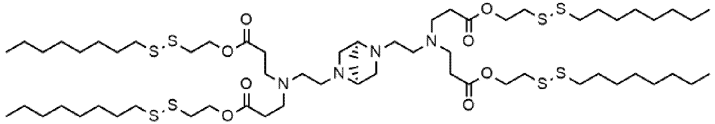 |
| AL-170 | 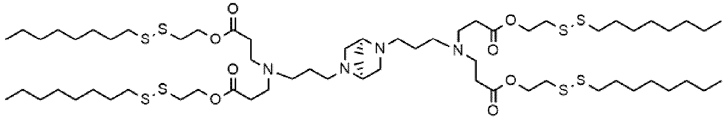 |
| AL-171 | 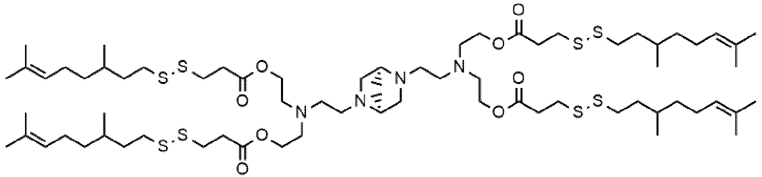 |
| AL-172 | 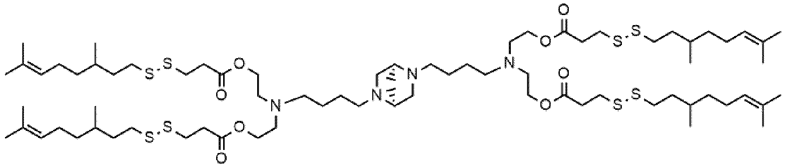 |
| AL-173 | 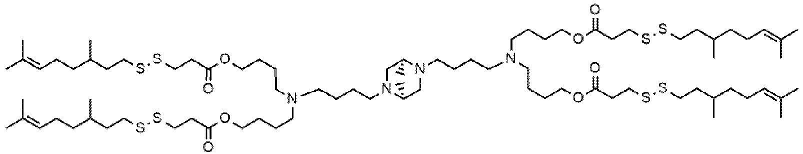 |
| AL-174 | 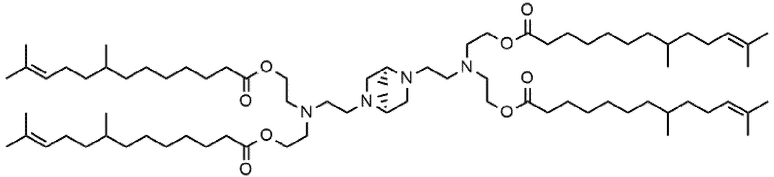 |
| AL-175 | 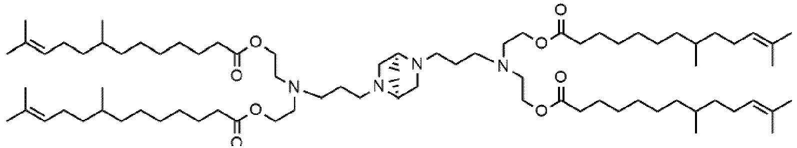 |
| AL-176 | 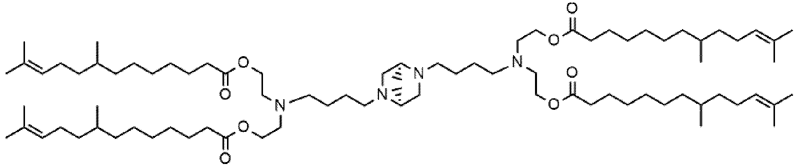 |
| AL-177 | 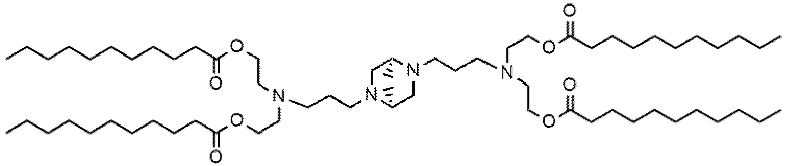 |
| AL-178 | 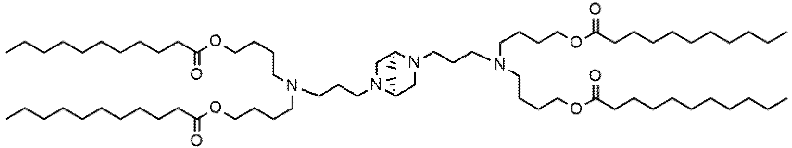 |
| AL-179 | 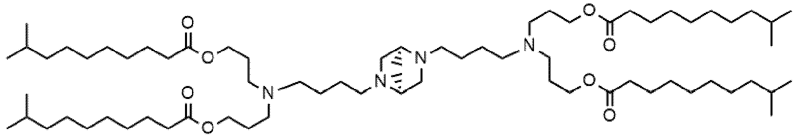 |
| AL-180 | 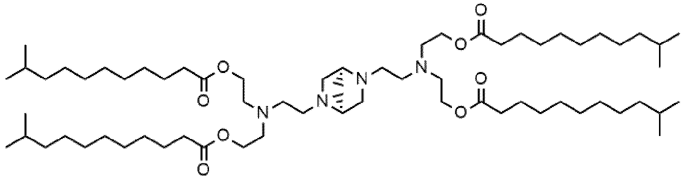 |
| AL-181 | 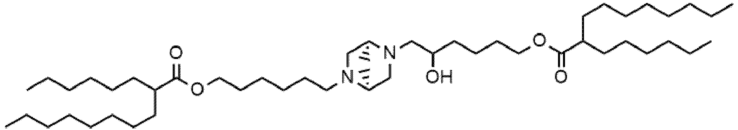 |
| AL-182 | 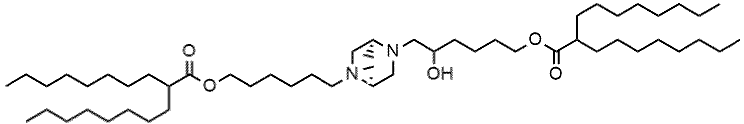 |
| AL-183 | 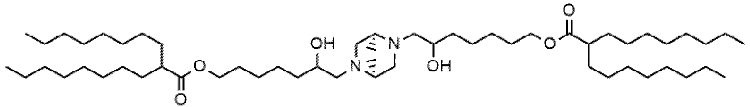 |
| AL-184 | 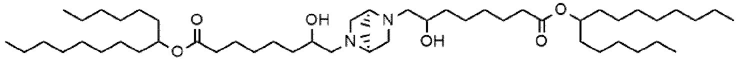 |
| AL-185 | 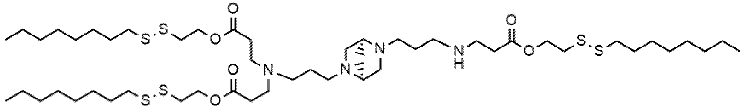 |
| AL-186 | 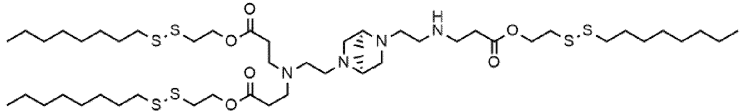 |
| AL-187 | 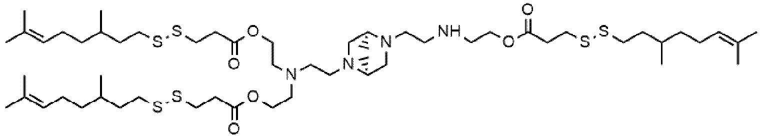 |
| AL-188 | 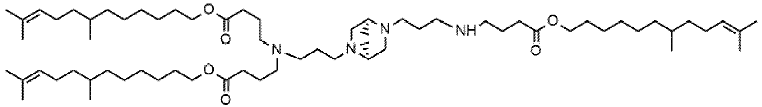 |
| AL-189 | 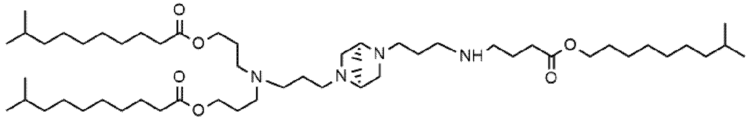 |
| AL-190 | 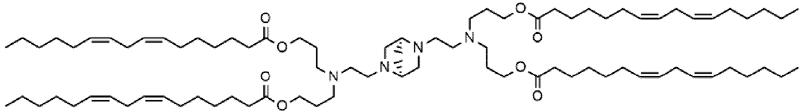 |
| AL-191 | 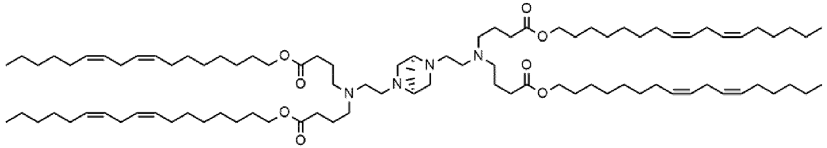 |
| AL-192 | 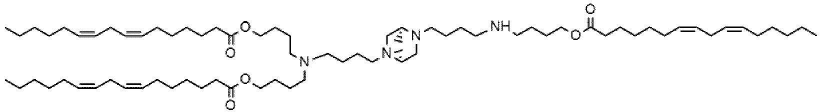 |
| AL-193 | 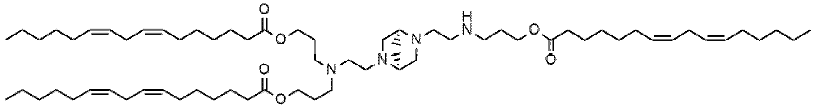 |
| AL-194 | 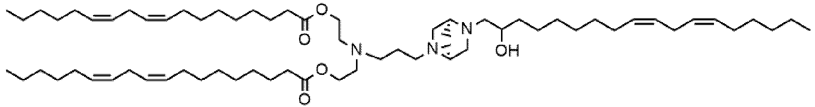 |
| AL-195 | 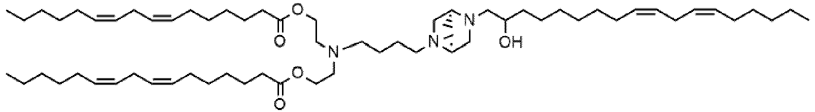 |
| AL-196 | 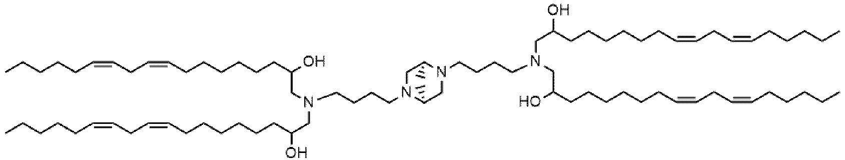 |
| AL-197 | 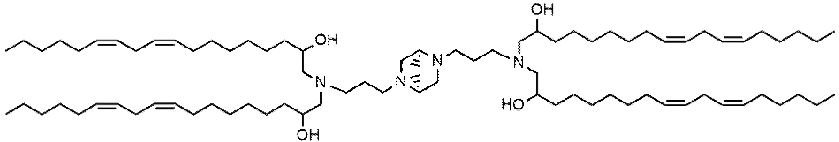 |
| AL-198 | 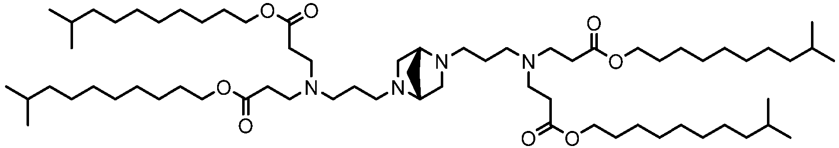 |
| AL-199 | 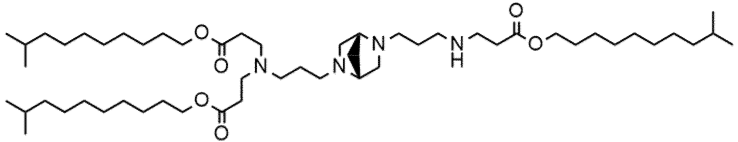 |

In another preferred embodiment, the ionizable lipid preferably has the following structure:

| Serial number | Structural formula |
|---|---|
| AL-6 | 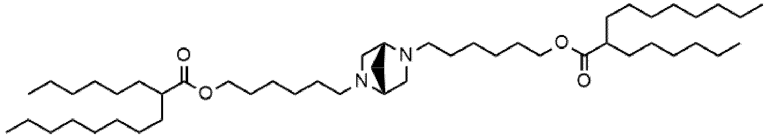 |
| AL-17 | 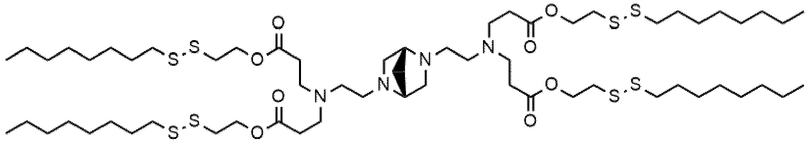 |
| AL-18 | 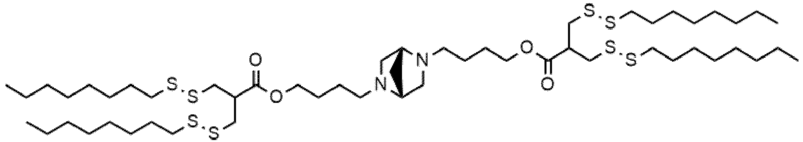 |
| AL-19 | 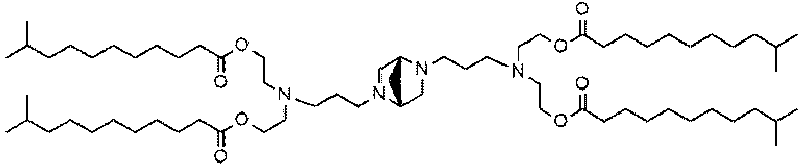 |
| AL-20 | 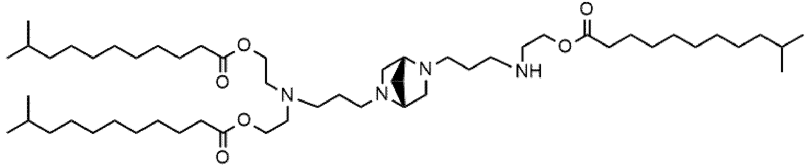 |

In another preferred embodiment, the ionizable lipid can be used for preparing a drug delivery system, which includes lipid nanoparticles (LNPs), liposomes, polymer nanoparticles, etc., preferably for preparing lipid nanoparticles.

In the second aspect of the present invention, is provided a method for preparing the ionizable lipid, or a pharmaceutically acceptable salt, tautomer, or stereoisomer thereof according to the first aspect of the present invention, which comprises method I, method II, and method III;
wherein the method I comprises the following steps:
   (A1) Compounds K2 and K3 are subjected to react with compound K1 in an inert solvent to obtain compound K4;
   (A2) Compounds K5 and K6 are subjected to react with compound K4 in an inert solvent to obtain the compound shown in Formula (I-1); 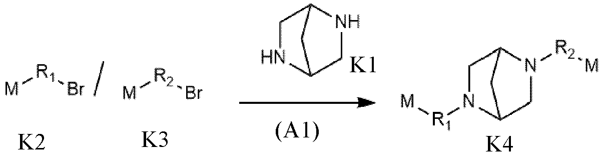 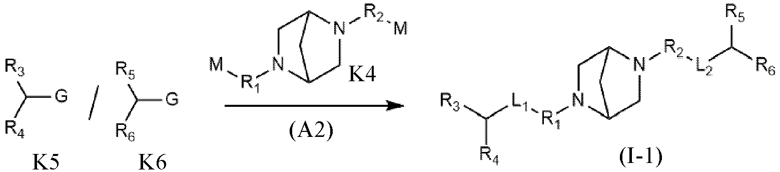
wherein R₁ and R₂ are each independently selected from -(CH₂)n-, wherein n is a positive integer from 1 to 14;
M and G are each independently and preferably selected from -OH, COOH, - SH, -NH₂, ethylene oxide;
L₁ has a structure of -(L₁ₐ-L_{1b}-L_{1c})-, L₂ has a structure of -(L₂ₐ-L_{2b}-L_{2c})-;
wherein L_{1b} and L_{2b} are each independently -(CH₂)n-, wherein n is a positive integer selected from 1 to 14;
L₁ₐ, L_{1c}, L₂ₐ and L_{2c} are each independently selected from the following functional groups: -CH₂-, -(C=O)O-, -O(C=O)-, -(S-S)-, -O(S=O)-, -(C=O)S-, - S(C=O)-, -(C=S)O-, -NH(C=O)-, -(C=S)NH-, -NH(C=S)-, -(C=O)NH- or -CH(OH)-, preferably -CH₂-, -(C=O)O-, -O(C=O)-, -(S-S)- or -CH(OH)-;
R₃, R₄, R₅ and R₆ are each independently a C2-C20 hydrocarbyl;

The method II comprises the following steps:
(B1) Compound K7 is subjected to react with compound K8 in an inert solvent to obtain compound K9;
(B2) Compound K9 is subjected to deprotection, thereby obtaining compond K10;
(B3) Compounds K11, K12 and K13 are subjected to react with compound K10 in an inert solvent to obtain the compound shown in Formula (1-2); 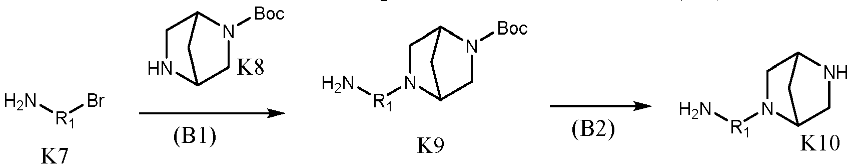 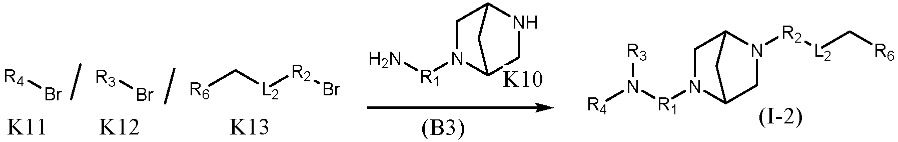
wherein R₁ and R₂ are each independently selected from -(CH₂)n-, wherein n is a positive integer from 1 to 14;
L₂ has a strusture of -(L₂ₐ-L_{2b}-L_{2c})-;
wherein L_{2b} is -(CH₂)n-, wherein n is a positive integer selected from 1-14; L₂ₐ and L_{2c} are each independently selected from the following functional groups: -CH₂-, -NH-, -(C=O)O-, -O(C=O)-, -(S-S)-, -O(S=O)-, -(C=O)S-, -S(C=O)-, -(C=S)O-, - NH(C=O)-, -(C=S)NH-, -NH(C=S)-, -(C=O)NH-, -CH(OH)-, -(C=C)-(CH₂)-(C=C)-, -(C=C)- or -(C≡C)-, preferably -CH₂-, -NH-, -(C=O)O-, -O(C=O)-, -(S-S)-, -(C=C)-(CH₂)-(C=C)- or -CH(OH)-;
R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ;
wherein R₃ₐ, R_{3c}, R₄ₐ and R_{4c} are each independently -(CH₂)n-, wherein n is a positive integer selected from 1-14;
R_{3b}, R_{3d}, R_{4b} and R_{4d} are each independently selected from the following functional groups: -CH₂-, -(C=O)O-, -O(C=O)-, -(S-S)-, -O(S=O)-, -(C=O)S-, - S(C=O)-, -(C=S)O-, -NH(C=O)-, -(C=S)NH-, -NH(C=S)-, -(C=O)NH-, -CH(OH)-, - (C=C)-(CH₂)-(C=C)-, -(C=C)- or -(C≡C)-, preferably -CH₂-, -(C=O)O-, -O(C=O)-, - (S-S)-, -(C=C)-(CH₂)-(C=C)- or -CH(OH)-;
R₃ₑ and R₄ₑ are each independently a C2-C20 hydrocarbyl;
R₆ is a C2-C20 hydrocarbyl;

The method III comprises the following steps:
(C1) Compound K7 is subjected to react with compound K14 in an inert solvent to obtain compound K15;
(C2) Compounds K11, K12, K16 and K17 are subjected to react with compound K15 in an inert solvent to obtain the compound shown in Formula (I-3); 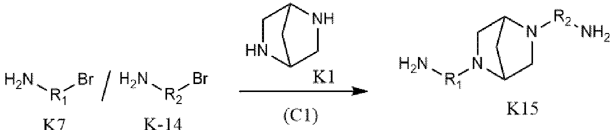 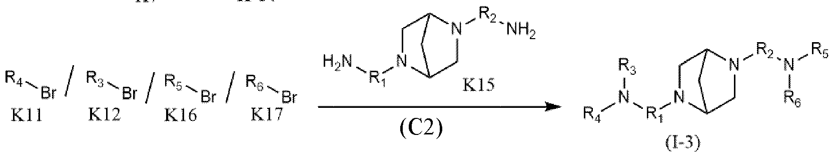
wherein
R₁ and R₂ are each independently selected from -(CH₂)n-, wherein n is a positive integer from 1 to 14;
R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ; R₅ has a structure of -R₅ₐ-R_{5b}-R_{5c}-R_{5d}-R₅ₑ, and R₆ has a structure of -R₆ₐ-R_{6b}-R_{6c}-R_{6d}-R₆ₑ;
wherein R₃ₐ, R_{3c}, R₄ₐ, R_{4c}, R₅ₐ, R_{5c}, R₆ₐ and R_{6c}are each independently -(CH₂)n-, wherein n is a positive integer selected from 1-14;
R_{3b}, R_{3d}, R_{4b}, R_{4d}, R_{5b}, R_{5d}, R_{6b} and R_{6d} are each independently selected from the following functional groups: -CH₂-, -(C=O)O-, -O(C=O)-, -(S-S)-, -O(S=O)-, - (C=O)S-, -S(C=O)-, -(C=S)O-, -NH(C=O)-, -(C=S)NH-, -NH(C=S)-, -(C=O)NH-, - CH(OH)-, -(C=C)-(CH₂)-(C=C)-, -(C=C)-, -(C≡C)-, preferably -CH₂-, -(C=O)O-, - O(C=O)-, -(S-S)-, -(C=C)-(CH₂)-(C=C)-, -CH(OH)-;
R₃ₑ, R₄ₑ, R₅ₑ and R₆ₑ are each independently a C2-C20 hydrocarbyl.

In another preferred embodiment, the inert solvent is selected from the group consisting of tetrahydrofuran, acetonitrile, chloroform, and a combination thereof.

In another preferred embodiment, the reaction temperature for method I is 0-90°C; the reaction temperature for method II is 0-90°C; and the reaction temperature for method III is 25-30°C.

In another preferred embodiment, the reaction time for method I is 1-24h; the reaction time for method II is 1-24h; and the reaction time for method III is 1-17h.

In the third aspect of the present invention, is provided a lipid nanoparticle (LNP) comprising the ionizable lipid, or a pharmaceutically acceptable salt, tautomer, or stereoisomer thereof according to the first aspect of the present invention.

In another preferred embodiment, the lipid nanoparticle further comprises auxiliary lipids.

In another preferred embodiment, the content of ionizable lipids in the lipid nanoparticle is at a molar ratio of 30-65% of the total lipid content.

In another preferred embodiment, the auxiliary lipids include auxiliary phospholipids, sterols, polymer conjugated lipids, or a combination thereof.

In another preferred embodiment, the auxiliary lipids are a combination of auxiliary phospholipids, sterols, and polymer conjugated lipids.

In another preferred embodiment, the auxiliary phospholipids are preferably selected from: 1,2-distearoyl-sn-glycerin-3-phosphocholine (DSPC), 1,2-dioloyl-sn-glycerin-3-phosphoethanolamine (DOPE), dioloyl-phosphatidylcholine (DOPC), 1,2-dioloyl-sn-glycerin-3-phosphocholine, 1,2-dipalmitoyl-sn-glycerin-3-phosphocholine, 1,2-dipalmitoyl-sn-glycerin-3-phosphoethanolamine, 1,2-myristoyl-sn-glycerin-3-phosphoethanolamine, 1,2-dioloyl-sn-glycerin-3-phosphoethanolamine, sodium salt of 1,2-dioloyl-sn-glycerin-3-phosphoryl-Rac (1-glycerol), 1,2-palmitoyl-phosphatidylglycerol, 1-palmitoyl-2-ooleylphosphatidylphospholipids, 1-palmitoyl-2-ooleylphosphatidylethanolamine, distearoyl phosphatidylethanolamine, 1-stearoyl-2-ooleylphosphatidylcholine, 1-stearoyl-2-ooleoyl-phosphatidylethanolamine, or a combination thereof.

In another preferred embodiment, the sterols comprise cholesterol or cholesterol derivatives.

In another preferred embodiment, the polymer conjugated lipids are PEGylated (PEG) lipids.

In another preferred embodiment, the PEGylated (PEG) lipid is selected from the group consisting of DMG-PEG2000, DSPE-PEG2000, DSG-PEG2000, DSPE-PEG-Mannose, DMG-PEG2000-(other active substances such as polypeptide, protein, amino acid, vitamin, etc.), and a combination thereof.

In another preferred embodiment, the lipid nanoparticle comprises an ionizable lipid, DSPC, cholesterol and DMG-PEG2000, wherein the molar ratio of ionizable lipid: DSPC: cholesterol: DMG-PEG2000 is (30-65): (5-30): (30-55): (1-5), preferably (40-65): (10-15): (35-50): (1-1.5).

In another preferred embodiment, the lipid nanoparticle comprises an ionizable lipid, DSPC, cholesterol and DMG-PEG2000, and a fifth component selected from the group consisting of:
(1) DOPA (dioleoylphosphatidylic acid) or DOPS (dioleoylphosphatidylserine);
(2) DMG-PEG₂₀₀₀-mannose; and a combination thereof;
wherein the molar ratio of ionizable lipid: DSPC: cholesterol: DMG-PEG2000: the fifth component is (30-50): (7.5-15): (25-50): (1-1.5): (0.5-25).

In another preferred embodiment, the lipid nanoparticle comprises an ionizable lipid, DSPC, cholesterol, DMG-PEG2000 and DOPA, wherein the molar ratio of ionizable lipid: DSPC: cholesterol: DMG-PEG2000: DOPA is (30-50): (7.5-15): (25-50): (1-1.5): (5-25).

In another preferred embodiment, the lipid nanoparticle comprises an ionizable lipid, DSPC, cholesterol, DMG-PEG2000 and DOPS, wherein the molar ratio of ionizable lipid: DSPC: cholesterol: DMG-PEG2000: DOPS is (30-50): (7.5-15): (25-50): (1-1.5): (4.5-20).

In another preferred embodiment, the lipid nanoparticle comprises an ionizable lipid, DSPC, cholesterol, DMG-PEG2000 and DMG-PEG₂₀₀₀-mannose,
wherein the molar ratio of ionizable lipid: DSPC: cholesterol: DMG-PEG2000: DMG-PEG₂₀₀₀-mannose is (30-50): (7.5-15): (25-50): (1-1.5): (0.5-2.5).

In another preferred embodiment, the ionizable lipid is AL-6 or AL-19.

In another preferred embodiment, the lipid nanoparticle comprises the ionizable lipid AL-6, DSPC, cholesterol, DMG-PEG2000 and DOPA, wherein the molar ratio of ionizable lipid AL-6: DSPC: cholesterol: DMG-PEG2000: DOPA is (30-50): (7.5-15): (25-50): (1-1.5): (5-25);
preferably (30-45):(7.5-10):(25-35):(1-1.5):(15-25);
more preferably (35-45):(7.5-10):(25-35):(1-1.5):(20-25).

In another preferred embodiment, the lipid nanoparticle comprises the ionizable lipid AL-6, DSPC, cholesterol, DMG-PEG2000 and DOPS, wherein the molar ratio of ionizable lipid AL-6: DSPC: cholesterol: DMG-PEG2000: DOPS is (30-50): (7.5-15): (25-50): (1-1.5): (4.5-20);
preferably (40-50):(7.5-15):(30-40):(1-1.5):(4.5-20);
more preferably (40-50):(7.5-10):(30-35):(1-1.5):(8-20).

In another preferred embodiment, the lipid nanoparticle comprises the ionizable lipid AL-6, DSPC, cholesterol, DMG-PEG2000 and DMG-PEG₂₀₀₀-mannose,
wherein the molar ratio of ionizable lipid: DSPC: cholesterol: DMG-PEG2000: DMG-PEG2000-mannose is (30-50): (7.5-15): (25-50): (1-1.5): (0.5-2.5);
preferably (40-50):(7.5-15):(35-40):(1-1.5):(0.5-2.5);
more preferably (45-50):(7.5-12.5):(35-40):(1-1.5):(0.5-2.5).

In another preferred embodiment, the lipid nanoparticle comprises the ionizable lipid AL-19, DSPC, cholesterol, and DMG-PEG2000, wherein the molar ratio of ionizable lipid AL-19: DSPC: cholesterol: DMG-PEG2000 is (30-65):(5-30):(30-55):(1-5),
preferably (40-50):(10-15):(35-50):(1-1.5);
more preferably (40-50):(10-15):(40-50):(1-1.5).

In another preferred embodiment, the lipid nanoparticle comprises the ionizable lipid AL-19, DSPC, cholesterol, DMG-PEG2000 and DOPA,
wherein the molar ratio of ionizable lipid Al-19: DSPC: cholesterol: DMG-PEG2000: DOPA is (30-50): (7.5-15): (25-50): (1-1.5): (5-25);
preferably (30-40):(10-15):(35-45):(1-1.5):(5-15);
more preferably (35-40):(10-15):(35-45):(1-1.5):(5-10).

In another preferred embodiment, the lipid nanoparticle comprises the ionizable lipid AL-19, DSPC, cholesterol, DMG-PEG2000 and DOPS,
wherein the molar ratio of ionizable lipid Al-19: DSPC: cholesterol: DMG-PEG2000: DOPS is (30-50): (7.5-15): (25-50): (1-1.5): (4.5-20);
preferably (30-40):(10-15):(35-45):(1-1.5):(4.5-20);
more preferably (35-40):(10-15):(35-45):(1-1.5):(4.5-15).

In another preferred embodiment, the lipid nanoparticle further comprises bioactive substances encapsulated within the lipid nanoparticle.

In another preferred embodiment, the bioactive substance is selected from the group consisting of nucleic acids, proteins, polypeptides, small molecules, and a combination thereof.

In another preferred embodiment, the nucleic acid comprises DNA, plasmids, messenger RNA (mRNA), small interfering RNA (siRNA), antisense oligonucleotides, small RNA, ribosomal RNA, microRNA, and transfer RNA, preferably mRNA.

In the fourth aspect of the present invention, is provided a lipid nanoparticle pharmaceutical preparation, which comprises the lipid nanoparticle according to the third aspect of the present invention, a bioactive substance encapsulated in the lipid nanoparticle, and a pharmaceutically acceptable carrier.

In another preferred embodiment, the bioactive substance is selected from the group consisting of nucleic acids, proteins, polypeptides, small molecules, and a combination thereof.

In another preferred embodiment, the nucleic acid comprises DNA, plasmids, messenger RNA (mRNA), small interfering RNA (siRNA), antisense oligonucleotides, small RNA, ribosomal RNA, microRNA, and transfer RNA, preferably mRNA.

In another preferred embodiment, the bioactive substance is nucleic acid, and in the lipid nanoparticle drug, the molar ratio of ionizable N atoms in ionizable lipid molecules to phosphate groups in nucleic acid molecules is (2-10): 1, further preferred is (4-8): 1.

In another preferred embodiment, the hydrated particle size of the lipid nanoparticle drug is 50-200 nm, preferably 70-150 nm, and most preferably 75-110 nm.

In another preferred embodiment, the lipid nanoparticle pharmaceutical preparation can be used for the treatment and/or prevention of tumors, infectious diseases, and rare diseases.

In another preferred embodiment, the dosage form of the lipid nanoparticle pharmaceutical preparation is selected from the group consisting of an injection, freeze-drying agent, nebulized inhaler, and topical agent.

In another preferred embodiment, the lipid nanoparticle pharmaceutical preparation is administered via injection, that is an intravenous, intramuscular, intradermal, subcutaneous, intrathecal, duodenal or intraperitoneal injection.

In another preferred embodiment, the lipid nanoparticle pharmaceutical preparation is administered via inhalation, such as intranasal administration.

In another preferred embodiment, the lipid nanoparticle pharmaceutical preparation is administered via transdermal route, such as transdermal application or electrode delivery.

In the fifth aspect of the present invention, is provided a method for preparing the lipid nanoparticle pharmaceutical preparation according to the fourth aspect of the present invention, which comprises:
(a) Mixing the ionizable lipid or a pharmaceutically acceptable salt, tautomer or stereoisomer thereof according to the first aspect of the present invention, and optional auxiliary lipids with an organic solvent to obtain a lipid organic phase;
(b) Mixing bioactive substances with an aqueous solvent to obtain an aqueous phase containing the bioactive substances;
(c) Mixing the lipid organic phase in step (a) with the aqueous phase in step (b) to obtain the lipid nanoparticle pharmaceutical preparation.

In another preferred embodiment, the organic solvent comprises ethanol, methanol, isopropanol, acetonitrile, dimethylformamide, dimethyl sulfoxide, dioxane, tetrahydrofuran, or a combination thereof.

In another preferred embodiment, the aqueous solvent is a buffer solution.

In another preferred embodiment, the aqueous solvent is a buffer solution with a pH range of 3-7.

In another preferred embodiment, the acidic buffer is a citrate buffer with a pH of 4.0.

In another preferred embodiment, the volume ratio of the lipid organic phase to the aqueous phase containing the bioactive substances is 1: (2-5), preferably 1: (3-4).

In another preferred embodiment, in step (c), the lipid organic phase and the aqueous phase are mixed through a microfluidic chip.

In another preferred embodiment, the method further comprises step (d): purifying, concentrating, filtering, and sterilizing the lipid nanoparticle pharmaceutical preparation obtained in step (c).

In the sixth aspect of the present invention, is provided a use of the ionizable lipid, or a pharmaceutically acceptable salt, tautomer, or stereoisomer thereof according to the first aspect of the present invention for the preparation of a drug delivery system.

In another preferred embodiment, the drug delivery system includes lipid nanoparticles (LNPs), liposomes, polymer nanoparticles, etc., preferably for preparing lipid nanoparticles.

In another preferred embodiment, the drug delivery system is used for delivering drugs for the treatment and/or prevention of tumors, infectious diseases, and rare diseases.

In the seventh aspect of the present invention, is provided a use of the lipid nanoparticle according to the third aspect of the present invention for the preparation of drugs for the treatment and/or prevention of tumors, infectious diseases, and rare diseases.

It should be understood that within the scope of the present invention, each technical features of the present invention described above and in the following (such as examples) may be combined with each other to form a new or preferred technical solution, which is not listed here due to space limitations.

### DESCRIPTION OF THE DRAWINGS

Figure 1 shows the spectrum and results of molecular weight detection of AL-6 using LC-MS. The characteristic peaks and corresponding integrated areas are shown in the figure, which proves that the obtained compound is the target compound.
Figure 2 shows the spectrum and results of purity detection of AL-6 using HPLC-CAD. The peak time of the product is 17.767 mins, and the purity reaches over 98%.
Figure 3 shows the spectrum and results of molecular weight detection of AL-17 using LC-MS. The characteristic peaks and corresponding integrated areas are shown in the figure, which proves that the obtained compound is the target compound.
Figure 4 shows the spectrum and results of purity detection of AL-17 using HPLC-CAD. The peak time of the product is 11.311 mins, and the purity reaches over 95%.
Figure 5 shows the spectrum and results of molecular weight detection of AL-18 using LC-MS. The characteristic peaks and corresponding integrated areas are shown in the figure, which proves that the obtained compound is the target compound.
Figure 6 shows the spectrum and results of purity detection of AL-18 using HPLC-CAD. The peak time of the product is 17.997 mins, and the purity reaches over 93%.
Figure 7 shows the characterization results of AL-19 by hydrogen spectrum.
Figure 8 shows the molecular weight spectrum of AL-19 detected by LCMS.
Figure 9 shows the spectrum and results of purity detection of AL-19 using HPLC-CAD. The purity of the product reaches over 95.14%.
Figure 10 shows the characterization results of AL-20 by hydrogen spectrum.
Figure 11 shows the molecular weight spectrum of AL-20 detected by LCMS.
Figure 12 shows the spectrum and results of purity detection of AL-20 using HPLC-CAD. The purity of the product reaches over 93.54%.
Figure 13 shows the electrophoresis results of the transcription template: there are three electrophoresis lanes in the figure, the first is a DNA indicator band of different lengths, the lane labeled as 1 is plasmid DNA, and the lane labeled as 2 is linearized transcription template.
Figure 14 shows the integrity peak map of the transcription template: the horizontal axis represents fragment length, and the vertical axis represents relative fluorescence units. There are three peaks labeled as LM, 4146, and UM in the left figure. LM and UM are low and high molecular weight DNA indicator bands, respectively, and 4146 is the target detection band. The fitted electrophoresis image is on the right side.
Figure 15 shows the electrophoresis results of the RNA after *in vitro* transcription: there are three electrophoresis lanes in the figure, the first is a RNA indicator band of different lengths, the lane labeled as IVT is RNA transcribed *in vitro,* and the lane labeled as CAP is mRNA capped by enzymatic method.
Figure 16 shows the integrity peak map of IVT RNA: the horizontal axis represents fragment length, and the vertical axis represents relative fluorescence units. There are two peaks labeled as LM and 1795 in the left figure, wherein LM is the low molecular weight RNA indicator band, and 1795 is the target RNA detection band. The fitted electrophoresis image is on the right side.
Figure 17 shows the integrity peak map of miRNA: the horizontal axis represents fragment length, and the vertical axis represents relative fluorescence units. There are two peaks labeled as LM and 1795 in the left figure, wherein LM is the low molecular weight RNA indicator band, and 1795 is the target RNA detection band. The fitted electrophoresis image is on the right side.
Figure 18 shows the characterization results of the physicochemical properties (particle size, PDI, encapsulation efficiency) of LNP-mRNA, with a particle size range of 70-100nm, PDI<0.2, and encapsulation efficiency between 85-100%, indicating that the physicochemical properties of LNP prepared above are similar.
Figure 19 shows the physicochemical properties, including particle size, PDI, and encapsulation efficiency of LNP(AL-20)-Luc of different formulations, with particle size below 100nm, PDI less than 0.2, and encapsulation efficiency between 60-90%.
Figure 20 shows the particle size results of lipid nanoparticles AXT-LNP-01 to AXT-LNP-20 (specific formulation shown in Table 1). It can be seen from the figure that all the particle sizes are below 150nm, and most of them are around 100nm.
Figure 21 shows the particle size distribution results of lipid nanoparticles AXT-LNP-01 to AXT-LNP-20. It can be seen from the figure that the PDI is below 0.2, and most of them are around 0.10.
Figure 22 shows the efficiency of encapsulation of mRNA by lipid nanoparticles AXT-LNP-01 to AXT-LNP-20. It can be seen from the figure that the encapsulation efficiency is higher than 80%, and it is generally around 90%.
Figure 23 shows the cellular expression and toxicity detection process of LNP-mRNA.
Figure 24 shows the *in vitro* cell expression results of LNP(AL-6)-mRNA. As shown in the figure, with the increase of mRNA concentration, the expression results of multiple formulations of LNP(AL-6) are better than those of LNP(SM-102).
Figure 25 shows the cell expression results of LNP(AL-17)-mRNA. As shown in the figure, with the increase of concentration, the cell expression of LNP(AL-17) series first increases and then decreases, and at 1ug/ml, it is generally lower than lipofectamine.
Figure 26 shows the cytotoxicity results of LNP(AL-6)-mRNA. It can be seen from the figure that the cell inhibition rate of LNP(AL-6) series products is almost zero at different mRNA concentrations, which is comparable to LNP(SM-102), proving the good safety of LNP(AL-6) molecules.
Figure 27 shows the cytotoxicity results of LNP(AL-20)-mRNA. It can be seen from the figure that the cell inhibition rate of LNP(AL-20)-Luc-1 and LNP(AL-20)-Luc-2 formulations is almost 0 at different mRNA concentrations, proving the good safety of LNP(AL-20).
Figure 28 shows the *in vivo* expression detection process of LNP-mRNA.
Figure 29 shows the *in vivo* expression results of LNP(AL-6)-hEPO and LNP(SM-102)-hEPO. It can be seen from the figure that both LNP(SM-102) and LNP(AL-6) show a trend of first increasing and then decreasing in mRNA expression *in vivo,* and both reach their maximum at around 6h. However, the expression of LNP(AL-6) series products is superior to that of LNP(SM-102).
Figure 30 shows the *in vivo* cytotoxicity results of LNP(AL-6)-Luc and LNP(SM-102)-Luc; wherein a shows a line graph of the changes in mouse body weight over time; b shows a line graph of changes in ALT (alanine aminotransferase) and AST (aspartate aminotransferase) levels in mice.
Figure 31 shows the proportion of fluorescence expression values *in vivo,* organs, and various organs of the physiological saline group and AXT-LNP-01 formulation. It can be seen from the figure that the expression level of AXT-LNP-01 formulation in the liver is as high as 87.5%.
Figure 32 shows the proportion of fluorescence expression values *in vivo,* organs, and various organs of formulations AXT-LNP-02, AXT-LNP-03, and AXT-LNP-04. The above formulations are imaging results after the addition of different proportions of DOPA. It can be seen from the figure that, compared with AXT-LNP-01, the proportion of DOPA has a significant impact on organ targeting in mice, and the lymph node-targeting ratio of AXT-LNP-04 is as high as 94.47%.
Figure 33 shows the proportion of fluorescence expression values *in vivo,* organs, and various organs of formulations AXT-LNP-05, AXT-LNP-06, and AXT-LNP-07. The above formulations are imaging results after the addition of different proportions of DOPS. It can be seen from the figure that, compared with AXT-LNP-01, the proportion of DOPS has a significant impact on organ targeting in mice, and the lymph node-targeting ratio of AXT-LNP-06 is as high as 95.03%.
Figure 34 shows the proportion of fluorescence expression values *in vivo,* organs, and various organs of formulations AXT-LNP-08, AXT-LNP-09, AXT-LNP-10 and AXT-LNP-11. The above formulations are imaging results after the addition of different proportions of DMG-PEG₂₀₀₀-mannose. It can be seen from the figure that the proportion of DMG-PEG2000-mannose has a significant impact on organ targeting in mice, and the lymph node-targeting ratio of AXT-LNP-09 is as high as 55.61%.
Figure 35 shows the proportion of fluorescence expression values *in vivo,* organs, and various organs of AXT-LNP-12, AXT-LNP-13, and AXT-LNP-14 formulations. The above formulations are imaging results obtained by changing the molar ratio of the four components of LNP. As shown in the figure, ionizable cationic lipids based on AL-19 can effectively improve their lymph node-targeting ratio by changing the molar ratio of the four components, and the lymph node targeting ratio of AXT-LNP-13 is as high as 97.06%.
Figure 36 shows the proportion of fluorescence expression values *in vivo,* organs, and various organs of AXT-LNP-15, AXT-LNP-16, and AXT-LNP-17 formulations. The above formulations are imaging results obtained by changing the molar ratio of DOPA. As shown in the figure, changing the molar ratio of DOPA to the four components can effectively improve the lymph node-targeting ratio by changing the molar ratio of the four components, and the lymph node-targeting ratio of AXT-LNP-15 is as high as 91.92%.
Figure 37 shows the proportion of fluorescence expression values *in vivo,* organs, and various organs of AXT-LNP-18, AXT-LNP-19, and AXT-LNP-20 formulations. The above formulations are imaging results obtained by changing the molar ratio of DOPS to the four components. As shown in the figure, changing the molar ratio of DOPS can effectively improve the lymph node-targeting ratio, and the lymph node-targeting ratio of AXT-LNP-13 is as high as 83.42%.

### Detailed Description

After extensive and in-depth research, the inventor unexpectedly discovered for the first time an ionizable lipid, which has the advantages of stable physical and chemical properties and low toxicity. The drug delivery system obtained by encapsulating drug loads (such as mRNA) with the ionizable lipid of the present invention exhibits high delivery efficiency and low toxicity. It not only efficiently delivers drug loads and increases the expression level of drug loads, but also improves the safety of the drug delivery system, thereby making the preventive and therapeutic effects of the drug delivery system more prominent.

Furthermore, based on the discovered novel ionizable lipids, the inventors screened a series of optimized lymph node-targeting LNP formulations. Specifically, in the present invention, for ionizable cationic lipid AL-6, anionic lipids DOPA and DOPS were added separately, based on DSPC, Chol and DMG-PEG₂₀₀₀ to obtain formulations, which were subjected to formulation screening. Finally, multiple formulations with a lymph node fluorescence ratio of over 90% were screened, and the highest ones were up to 94.47% and 95.03%, respectively. Based on the above four components, the surface was modified with DMG-PEG₂₀₀₀-mannose, and the proportion of modified components was adjusted to achieve varying degrees of lymph node targeting; For the ionizable lipid AL-19, , ratios of four components based on DSPC, Chol, and DMG-PEG₂₀₀₀ were first optimized to achieve an increase in the proportion of lymph node targeting by up to 97.06%. Furthermore, anionic DOPA and DOPS were added separately, based on these four components to achieve a high organ proportion in lymph nodes.

On this basis, the present invention has been completed.

### Terms

In order to easily understand this disclosure, certain terms are first defined. As used herein, each of the following terms shall have the meanings given below unless expressly provided herein.

The term "alkyl" refers to a saturated carbon chain with 1 to 20 carbon atoms, which can be straight or branched, or a combination thereof, unless the carbon chain is otherwise defined. Examples of an alkyl include methyl, ethyl, propyl, isopropyl, butyl, sec butyl and tert butyl, pentyl, hexyl, heptyl, octyl, etc. Unless otherwise specified in the specification, an alkyl may be optionally substituted. The term "unsaturated hydrocarbon group" refers to a group containing at least one C=C double bond (alkene) or at least one C≡C triple bond (alkyne). "Alkyl", "alkene", and "alkyne" can be collectively referred to as "hydrocarbon group".

### Ionizable lipid

As used in herein, the terms "the ionizable lipid of the present invention" and "the ionizable cationic lipid of the present invention" can be used interchangeably, both referring to lipid compounds with a structure of Formula I, or pharmaceutically acceptable salts, tautomers, or stereoisomers thereof.

Ionizable lipids will undergo protonation and conversion to cationic lipids at low pH values, and then convert to auxiliary phospholipids at normal physiological pH. The interaction between auxiliary phospholipids and the anionic cell membrane of blood cells is relatively small, which improves the biocompatibility of lipid nanoparticles. When lipid nanoparticles are internalized by cells, the pH value in the endosome is low, and the lipids are protonated and positively charged. The stability of the membrane structure is decreased or even destroyed, which is conducive to the escape of lipid nanoparticles from the endosome. Overall, the pH sensitivity of a lipids is advantageous for the *in vivo* delivery of lipid nanoparticles carrying bioactive components such as mRNA molecules.

In one aspect of the present invention, is provided an ionizable lipid having a structure as shown in Formula I: 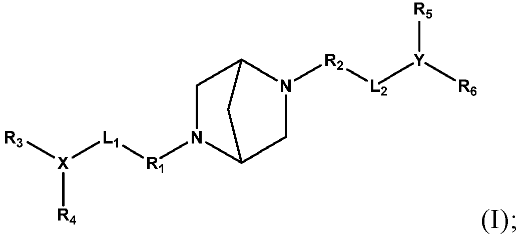 wherein
R₁ and R₂ are each independently selected from -(CH₂)n-, where n is a positive integer from 1 to 14;
X and Y are each independently -CH- or N;
L₁ and L₂ are each independently a divalent linking group or absent;
R₃, R₄, R₅, and R₆ are each independently H, CH₃, C2-C30 hydrocarbyl (such as C2-C30 alkyl, C2-C30 alkene, C2-C30 alkynyl), or -(CH₂)s-R^{a}-(CH₂)g-R^{b}-(CH₂)m-R^{c}, wherein s and g are each independently selected from a positive integer of 1-20, m is selected from an integer of 0-20, preferably s+g+m is 2-35; R^{c} is CH₃ or C2-C15 hydrocarbyl (such as C2-C15 alkyl, C2-C15 alkene, C2-C15 alkynyl);
R^{a} and R^{b} are each independently selected from -CH₂-, C2-C6 alkene or functional groups as shown below: 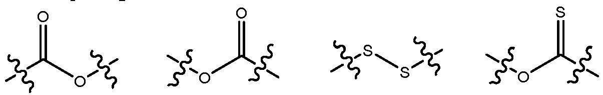 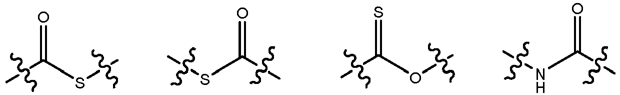 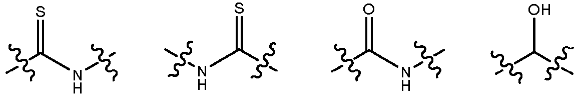
wherein 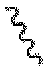 represents a linking bond;
and, R₃ and R₄ are not simultaneously H, R₅ and R₆ are not simultaneously H.

In a preferred embodiment of the present invention, in Formula I,
R₁ and R₂ are each independently selected from -(CH₂)n-, where n is a positive integer from 1 to 14;
X and Y are each independently -CH- or N;
L₁ and L₂ are each independently a divalent linking group or absent;
R₃, R₄, R₅, and R₆ are each independently H, CH₃, C2-C30 hydrocarbyl (such as C2-C30 alkyl, C2-C30 alkene, C2-C30 alkynyl), or -(CH2)s-R^{a}-(CH₂)g-R^{b}-(CH₂)m-CH₃, wherein s and g are each independently selected from a positive integer of 1-20, m is selected from an integer of 0-20, preferably s+g+m is 2-35,
R^{a} and R^{b} are each independently selected from functional groups as shown below: 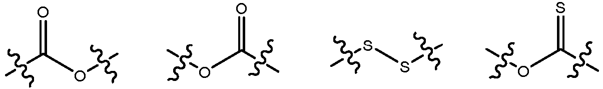 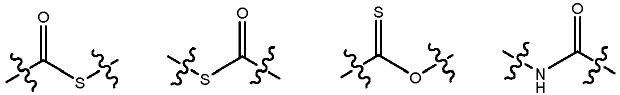 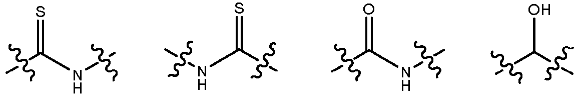
wherein 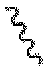 represents a linking bond;
and R₃ and R₄ are not simultaneously H, R₅ and R₆ are not simultaneously H.

In a preferred embodiment of the present invention, the ionizable lipid has a sub-structure as shown in Formula (I-1), (I-2) and (I-3), respectively: 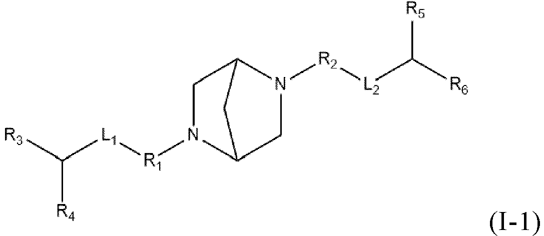 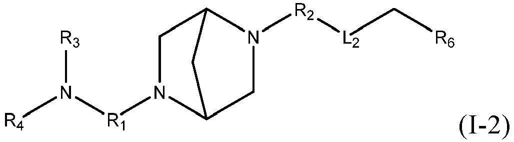 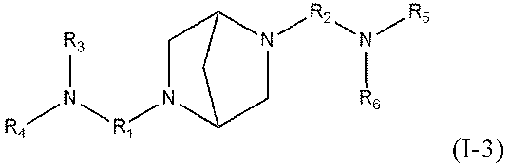
for the sub-structure shown in Formula (I-1), both X and Y in Formula (I) are - CH-; wherein R₁ and R₂ are each independently selected from -(CH₂)n-, where n is a positive integer from 1 to 14; L₁ has a structure of -(L₁ₐ-L_{1b}-L_{1c})- from right to left, wherein L_{1b} is -(CH₂)n -, and n is a positive integer selected from 1-14; L₁ₐ and L_{1c} are each independently selected from the following functional groups: -CH₂-, - (C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably -CH₂-, -(C=O)O-, - O(C=O)-, -(S-S)-, -CH(OH)-; L₂ has a structure of -(L₂ₐ-L_{2b}-L_{2c})- from left to right; wherein L_{2b} is -(CH₂)n-, and n is a positive integer selected from 1-14; L₂ₐ and L_{2c} are each independently selected from the following functional groups: -CH₂-, - (C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, -(C=O)NH-, -CH(OH)-, preferably -CH₂-, -(C=O)O-, - O(C=O)-, -(S-S)-, -CH(OH)-; R₃, R₄, R₅, and R₆ are each independently a C2-C20 hydrocarbyl.
for the sub-structure shown in Formula (I-2), X is N, Y is -CH-, L₁ is absent, L₂ is a divalent linking group, and R₅ is H, in Formula (I); wherein R₁ and R₂ are each independently selected from -(CH₂)n-, and n is a positive integer from 1 to 14; L₂ has a structure of -(L₂ₐ-L_{2b}-L_{2c})- from left to right; wherein L_{2b} is -(CH₂)n-, and n is a positive integer selected from 1-14; L₂ₐ and L_{2c} are each independently selected from the following functional groups: -CH₂-, -NH-, -(C=O)O-, -O(C=O)-, -(S-S)-, - O(S=O)-, -(C=O)S-, -S(C=O)-, -(C=S)O-, -NH(C=O)-, -(C=S)NH-, -NH(C=S)-, - (C=O)NH-, -CH(OH)-, -(C=C)-(CH₂)-(C=C)-, -(C=C)-, -(C=C)-, preferably -CH₂-, - NH-, -(C=O)O-, -O(C=O)-, -(S-S)-, -(C=C)-(CH₂)-(C=C)-, -CH(OH)-; R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ; wherein R₃ₐ, R_{3c}, R₄ₐ 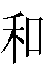 R_{4c} are each independently -(CH₂)n-, and n is a positive integer from 1 to 14; R_{3b}, R_{3d}, R_{4b} 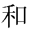 R_{4d} are each independently selected from the following functional groups: -CH₂-, -(C=O)O-, -O(C=O)-, -(S-S)-, -O(S=O)-, -(C=O)S-, - S(C=O)-, -(C=S)O-, -NH(C=O)-, -(C=S)NH-, -NH(C=S)-, -(C=O)NH-, -CH(OH)-, - (C=C)-(CH₂)-(C=C)-, -(C=C)-, -(C≡C)-, preferably -CH₂-, -(C=O)O-, -O(C=O)-, - (S-S)-, -(C=C)-(CH₂)-(C=C)-, -CH(OH)-; R₃ₑ and R₄ₑ are each independently a C2-C20 hydrocarbyl; R₆ is a C2-C20 hydrocarbyl.

For the sub-structure shown in Formula (I-3), both X and Y are N, and L1 and L2 are absent in Formula (I); wherein R₁ and R₂ are each independently -(CH₂)n-, and n is a positive integer from 1 to 14; R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ; R₅ has a structure of -R₅ₐ-R_{5b}-R_{5c}-R_{5d}-R₅ₑ, and R₆ has a structure of -R₆ₐ-R_{6b}-R_{6c}-R_{6d}-R₆ₑ; wherein R₃ₐ, R_{3c}, R₄ₐ, R_{4c}, R₅ₐ, R_{5c}, R₆ₐ and R_{6c} are each independently -(CH₂)n-, and n is a positive integer from 1 to 14; R_{3b}, R_{3d}, R_{4b}, R_{4d}, R_{5b}, R_{5d}, R_{6b} and R_{6d} are each independently selected from the following functional groups: -CH₂-, -(C=O)O-, -O(C=O)-, -(S-S)-, -O(S=O)-, - (C=O)S-, -S(C=O)-, -(C=S)O-, -NH(C=O)-, -(C=S)NH-, -NH(C=S)-, -(C=O)NH-, - CH(OH)-, -(C=C)-(CH₂)-(C=C)-, -(C=C)-, -(C≡C)-, preferably -CH₂-, -(C=O)O-, - O(C=O)-, -(S-S)-, -(C=C)-(CH₂)-(C=C)-, -CH(OH)-; and R₃ₑ, R₄ₑ, R₅ₑ, R₆ₑ are each independently a C2-C20 hydrocarbyl.

In a more preferred embodiment of the present invention, the ionizable lipid has a structure selected from Table 1, wherein AL-6, AL-17, AL-18, AL-19, and AL-20 from Table 1 are preferred.

### Auxiliary lipids

As used herein, the term "auxiliary lipids" refers to other types of lipids in lipid nanoparticles besides ionizable lipids, including auxiliary phospholipids, sterols, polymer-conjugated lipids, or a combination thereof. Auxiliary lipids are mainly used to improve the performance of lipid nanoparticles, such as stability, delivery efficiency, tolerance, and biological distribution.

In some embodiments, the auxiliary phospholipids include, but are not limited to 1,2-distearoyl-sn-glycerin-3-phosphocholine (DSPC), 1,2-dioloyl-sn-glycerin-3-phosphoethanolamine (DOPE), dioloyl-phosphatidylcholine (DOPC), 1,2-dioloyl-sn-glycerin-3-phosphocholine, 1,2-dipalmitoyl-sn-glycerin-3-phosphocholine, 1,2-dipalmitoyl-sn-glycerin-3-phosphoethanolamine, 1,2-myristoyl-sn-glycerin-3-phosphoethanolamine, 1,2-dioloyl-sn-glycerin-3-phosphoethanolamine, sodium salt of 1,2-dioloyl-sn-glycerin-3-phosphoryl-Rac (1-glycerol), 1,2-palmitoyl-phosphatidylglycerol, 1-palmitoyl-2-ooleylphosphatidylphospholipids, 1-palmitoyl-2-ooleylphosphatidylethanolamine, distearoyl phosphatidylethanolamine, 1-stearoyl-2-ooleylphosphatidylcholine, 1-stearoyl-2-ooleoyl-phosphatidylethanolamine, or a combination thereof.

In a preferred embodiment of the present invention, the auxiliary phospholipid is DSPC (1,2-distearoyl-sn-glycerin-3-phosphocholine, also known as distearoyl phosphatidylcholine). DSPC is a commonly used phosphatidylcholine, and its tail group is a saturated alkane chain with a melting point of -54°C. It has a cylindrical shape and forms a layered structure in lipid nanoparticles, thereby making the structure of lipid nanoparticles more stable.

In a preferred embodiment of the present invention, the auxiliary phospholipid is DOPE (1,2-dioloyl-sn-glycerin-3-phosphoethanolamine, also known as dioloyl phosphatidylethanolamine). DOPE is a commonly used phosphatidylethanolamine. The tail group of DOPE consists of two unsaturated alkane chains, with a melting point of -30°C and a conical shape. It is prone to form an inverted hexagon in lipid nanoparticles, causing instability of the endosomal membrane and facilitating endosomal escape of lipid nanoparticles.

In another preferred embodiment, the sterols include, but are not limited to cholesterol or cholesterol derivatives. Cholesterol can adjust the integrity and hardness of lipid membranes, enhance the stability of lipid nanoparticles, and the morphology of cholesterol derivatives can affect the delivery efficiency and biological distribution of lipid nanoparticles, such as the chain length of hydrophobic tail groups in cholesterol analogs, the flexibility of steroid rings, and the polarity of hydroxyl groups. Cholesterol also affects the morphology of lipid nanoparticles, and cholesterol derivatives result in lipid nanoparticles being multi-layered polyhedral structures divided by lipids rather than spherical shapes. At the same time, cholesterol affects the selectivity of lipid nanoparticles towards targets: Lipid nanoparticles containing cholesteryl oleate have higher selectivity towards liver endothelial cells than hepatocytes; When lipid nanoparticles contain cholesterol hving oxidative modifications on the tail groups, the content of lipid nanoparticles in liver endothelial cells and Kupffer cells is higher than that in hepatocytes.

In some embodiments, the polymer conjugated lipids are polyethylene glycol (PEG) conjugated lipids, also known as polyethylene glycol lipids or PEGylated lipids. PEGylated lipids have multiple effects on the characteristics of lipid nanoparticles: The amount of PEGylated lipids will affect the particle size and electric potential of lipid nanoparticles; reduce particle aggregation and improve the stability of lipid nanoparticles; reduce the particle clearance rate mediated by the kidney and mononuclear phagocyte system (MPS), and prolong the circulation time of particles; and the functional groups on the surface can be modified with ligands to enhance targeted delivery capability. The molar mass and length of lipids affect the characteristics of PEGylated lipids. Both DMG-PEG2000 and DSG-PEG2000 are neutral phospholipids with saturated alkyl chain lengths of C14, C16, or C18, respectively. However, DMG-PEG2000 can separate from lipid nanoparticles faster, which is beneficial for cell uptake of nanoparticles and escape from endosomes. Therefore, the delivery efficiency of DMG-PEG2000 is better than that of DSG-PEG2000.

In a preferred embodiment of the present invention, the auxiliary phospholipid is the combination of DSPC, cholesterol and DMG-PEG2000.

### Lipid nanoparticle (LNP)

As used herein, the terms "lipid nanoparticle(s)", "lipid nanosphere(s)", or "LNP(s)" refer to particles with a diameter of approximately 5 to 500 nm. In some embodiments, lipid nanoparticles comprise one or more active agents (bioactive substances). In some embodiments, lipid nanoparticles comprise nucleic acids. In some embodiments, nucleic acids condense with cationic lipids, polymers, or multivalent small molecules inside the nanoparticles, as well as external lipid coatings that interact with the biological environment. Due to the repulsive force between phosphate groups, nucleic acids are natural rigid polymers that tend to have elongated configurations. In cells, to cope with volume limitations, DNA can package itself under appropriate solution conditions with the help of ions and other molecules. Usually, DNA condensation is defined as the collapse of an extended DNA strand into compact, ordered particles containing only one or a few molecules. By binding with phosphate groups, cationic lipids can concentrate DNA and tightly stack it by neutralizing phosphate charges.

In some embodiments, bioactive substances are encapsulated into LNP. In some embodiments, bioactive substances may be anionic compounds, including but not limited to DNA, RNA (messenger RNA, transfer RNA, ribosomal RNA, microRNA, etc.), natural and synthetic oligonucleotides (including antisense oligonucleotides, interfering RNA, and small interfering RNA), nucleoproteins, peptides, nucleic acids, nucleases, DNA containing nucleoproteins, such as intact or partially deproteinized viral particles (virions), oligomeric and polymeric anionic compounds other than DNA (such as acidic polysaccharides and glycoproteins). In some embodiments, bioactive substances can be mixed with adjuvants.

In LNP vaccine products, bioactive substances are typically contained within the interior of LNP. In some embodiments, bioactive substances comprise nucleic acids. Usually, water-soluble nucleic acids condense with cationic lipids or poly cationic polymers inside the particles, and the surface of the particles is rich in auxiliary phospholipids or PEG lipid derivatives. Additional ionizable cationic lipids may also be located on the surface. When entering the lysosome, ionizable cationic lipids are ionized through the acidic environment of the lysosome and positively charged, thereby interacting with the lysosome membrane to promote endosome escape.

Regarding LNP, ionizable lipids may have different properties or functions. Due to the pKa of amino groups, when the external pH value is lower than the pKa of lipid molecules, they can undergo protonation and become positively charged. Under these conditions, lipid molecules can electrostatically bind to the phosphate groups of nucleic acids, leading to the formation of LNP and encapsulation of nucleic acids. Additionally, the surface charge of LNP in biological fluids (such as blood) at physiological pH values is essentially neutral. High LNP surface charge is associated with toxicity, fixed and free state, rapid clearance of circulation by macrophages, hemolytic toxicity, including immune activation (Filion et al. Biochim Biophys Acta., October 23, 1997; 1329 (2): 345-56).

In some embodiments, pKa can be high enough to allow ionizable cationic lipids to take on a positively charged form at acidic endosomal pH. In this way, cationic lipids can bind with endogenous intracellular anionic lipids to promote membrane lysis of non-bilayer structures, such as hexagonal HII phase, resulting in more efficient intracellular delivery. In some embodiments, the range of pKa is between 6.2-6.5. For example, pKa can be about 6.2, about 6.3, about 6.4, or about 6.5. Unsaturated tails also contribute to the ability of lipids to adopt non-bilayer structures. (Jayaraman et al., Angew Chem Int Ed Engl, August 20, 2012; 51 (34): 8529-33).

The release of nucleic acids in LNP preparations, as well as other characteristics such as liposome clearance rate and circulating half-life, can be altered by the presence of polyethylene glycol and/or sterols (such as cholesterol) or other potential additives in LNP, as well as the overall chemical structure (including the pKa of any ionizable cationic lipids that are part of the formulation).

In one aspect of the present invention, is provided a lipid nanoparticle (LNP) comprising the ionizable lipid, or a pharmaceutically acceptable salt, tautomer, or stereoisomer thereof according to the first aspect of the present invention. Furthermore, the lipid nanoparticle further comprises one or more auxiliary lipids; and the auxiliary lipids include auxiliary phospholipids, steroids, polymer conjugated lipids.

In some specific embodiments of the present invention, LNP comprising the following components is provided:
(1) LNP comprising ionizable lipid AL-6, with molar ratios of each component: AL-6:DSPC:Cholesterol:DMG-PEG₂₀₀₀:DOPA(37.5~47.5:7.5~9.5:28.9~36.6: 1.1~1.4:5~25);
(2) LNP comprising ionizable lipid AL-6, with molar ratios of each component: AL-6:DSPC:Cholesterol:DMG-PEG₂₀₀₀:DOPA(41.6~47.6:8.3~9.5:32.1~36.7: 1.3~1.4:4.8~16.7);
(3) LNP comprising ionizable lipid AL-6, with molar ratios of each component: AL-6:DSPC:Cholesterol:DMG-PEG₂₀₀₀:DMG-PEG₂₀₀₀-mannose (50:10:36~38:1.5:0.5~2.5);
(4) LNP comprising ionizable lipid AL-19, with molar ratios of each component: AL-19:DSPC:Cholesterol:DMG-PEG₂₀₀₀ (40~50:10~15:38.5~48.5:1.5);
(5) LNP comprising ionizable lipid AL-6, with molar ratios of each component: AL-19:DSPC:Cholesterol:DMG-PEG₂₀₀₀:DOPA (34.0~38.0:12.7~14.2:37.0~41.3:1.3-1.4:5.0~15.0);
(6) LNP comprising ionizable lipid AL-6, with molar ratios of each component: AL-19:DSPC:Cholesterol:DMG-PEG₂₀₀₀:DOPS (33.2~38.1:12.5~14.3:36.3~41.4:1.3-1.4:4.8~16.7).

### Lipid nanoparticle pharmaceutical preparations

In another aspect of the present invention, is provided a lipid nanoparticle pharmaceutical preparation (or lipid nanoparticle pharmaceutical composition or LNP composition), which comprises the lipid nanoparticle according to the third aspect of the present invention, a bioactive substance encapsulated in the lipid nanoparticle, and a pharmaceutically acceptable carrier. The lipid nanoparticle pharmaceutical preparation is used to deliver bioactive substances to cells in subjects in need thereof.

In some embodiments, bioactive substances are encapsulated into LNP. In some embodiments, bioactive substances may be anionic compounds, including but not limited to DNA, RNA (messenger RNA, transfer RNA, ribosomal RNA, microRNA, etc.), natural and synthetic oligonucleotides (including antisense oligonucleotides, interfering RNA, and small interfering RNA), nucleoproteins, peptides, nucleic acids, nucleases, DNA containing nucleoproteins, such as intact or partially deproteinized viral particles (virions), oligomeric and polymeric anionic compounds other than DNA (such as acidic polysaccharides and glycoproteins). In some embodiments, bioactive substances can be mixed with adjuvants.

In some embodiments, the LNP composition includes nucleic acids, ionizable cationic lipids with the structure shown in Formula (I), auxiliary phospholipids (e.g., DSPC, DOPE, DOPC, or a combination thereof), sterols (e.g., cholesterol or cholesterol derivatives, or plant sterols such as β-sitosterol), and polymer conjugated lipids (e.g., DMG-PEG2000). In some embodiments, the LNP composition includes nucleic acids; ionizable cationic lipids with the structure shown in Formula (I), the content of which is 30-65% (molar ratio, the same below) of the total lipids in the composition; auxiliary phospholipids (e.g., DSPC, DOPE, DOPC, or a combination thereof), the content of which is 5-30% of the total lipids in the composition; sterols (e.g., cholesterol or cholesterol derivatives, or plant sterols such as β-sitosterol), the content of which is 30-55% of the total lipids in the composition; and polymer conjugated lipids (e.g., DMG-PEG2000), the content of which 1-5% of the total lipids in the composition. Furthermore, in the LNP composition, the molar ratio of ionizable N atoms in ionizable lipid molecules to phosphate groups in nucleic acid molecules is (2-10): 1, preferably is (4-8): 1.

In a preferred embodiment of the present invention, the LNP composition includes nucleic acids, ionizable cationic lipids with the structure shown in Formula (I), auxiliary phospholipids (e.g., DSPC, DOPE, DOPC, or a combination thereof), sterols (e.g., cholesterol or cholesterol derivatives, or plant sterols such as β-sitosterol), and polymer conjugated lipids (e.g., DMG-PEG2000 etc.). In a more preferred embodiment of the present invention, the LNP composition includes nucleic acids, ionizable cationic lipids with the structure shown in Formula (I), auxiliary phospholipids (e.g., DSPC, DOPE, DOPC, or a combination thereof), sterols (e.g., cholesterol or cholesterol derivatives, or plant sterols such as β-sitosterol), and polymer conjugated lipids (e.g., DMG-PEG2000).

As used herein, the terms "encapsulation" and "encapsulated" refer to mRNA, DNA, siRNA, or other nucleic acid drugs being inside or binding to lipid nanoparticles. As used herein, the term "encapsulation" refers to a complete encapsulation or partial encapsulation. For example, mRNA can be selected to treat and/or prevent related diseases when administering lipid nanoparticle compositions containing mRNA to subjects in need thereof.

As used herein, the term "pharmaceutically acceptable carrier" includes but is not limited to any adjuvant, carrier, excipient, scintillation agent, sweetener, diluent, preservative, dye/coloring agent, flavor enhancer, surfactant, wetting agent, dispersant, suspension agent, stabilizer, isotonic agent, solvent, or emulsifier approved by the Food and Drug Administration for use in humans or livestock.

### The preparation method of Lipid nanoparticle pharmaceutical preparations

In another aspect of the present invention, is provided a method for preparing a lipid nanoparticle drug preparation, comprising: (a) Mixing the ionizable lipid according to the first aspect of the present invention and optional auxiliary lipid with an organic solvent to obtain a lipid organic phase; (b) Mixing bioactive substances with aqueous solvents to obtain an aqueous phase containing bioactive substances; (c) Mixing the lipid organic phase in step (a) with the aqueous phase in step (b) to obtain the lipid nanoparticle drug. Furthermore, the method further comprises step (d): purifying, concentrating, filtering, and sterilizing the lipid nanoparticle drug obtained in step (c).

In some embodiments, the organic solvent includes but is not limited to ethanol, methanol, isopropanol, acetonitrile, dimethylformamide, dimethyl sulfoxide, dioxane, tetrahydrofuran, or a combination thereof. In some embodiments, the lipid organic phase comprises a small percentage of water or pH buffer. The lipid organic phase may contain up to 60% by volume of water, such as up to about 0.05%, 0.1%, 0.5%, 1%, 2%, 3%, 4%, 5%, 10%, 15%, 20%, 25%, 30%, 35%, 40%, 45%, 50%, 55%, or 60% by volume of water. In an embodiment, the lipid organic phase contains between approximately 0.05% and 60% by volume of water, such as between approximately 0.05% and 50%, between approximately 0.05% and 40%, or between approximately 5% and 20% by volume of water.

In some embodiments, the lipid organic phase comprises a single type of lipid, such as ionizable cationic lipids, auxiliary phospholipids, sterols, or polymer conjugated lipids. In some embodiments, the lipid organic phase comprises various lipids. In a preferred embodiment of the present invention, the lipid organic phase includes ionizable cationic lipids with the structure shown in Formula (I), auxiliary phospholipids (e.g., DSPC, DOPE, DOPC, or a combination thereof), sterols (e.g., cholesterol or cholesterol derivatives, or plant sterols such as β-sitosterol), and polymer conjugated lipids (e.g., DMG-PEG2000). In a preferred embodiment of the present invention, the ionizable cationic lipid phase includes nucleic acids, ionizable cationic lipids with the structure shown in Formula (I), auxiliary phospholipids (e.g., DSPC, DOPE, DOPC, or a combination thereof), sterols (e.g., cholesterol or cholesterol derivatives, or plant sterols such as β-sitosterol), and polymer conjugated lipids (e.g., DMG-PEG2000). In a more preferred embodiment of the present invention, the ionizable cationic lipid phase includes ionizable cationic lipids with the structure shown in Formula (I), auxiliary phospholipids (e.g., DSPC, DOPE, DOPC, or a combination thereof), sterols (e.g., cholesterol or cholesterol derivatives, or plant sterols such as β-sitosterol), and polymer conjugated lipids (e.g., DMG-PEG2000). In a preferred embodiment of the present invention, the ionizable cationic lipid phase includes ionizable cationic lipids with the structure with the structure shown in Formula (I), DSPC, cholesterol and DMG-PEG2000.

In some embodiments, the aqueous solvent is water. In some embodiments, the aqueous solvent is an aqueous buffer solution with a pH between 3-8 (e.g., a pH of about 3, about 4, about 5, or about 6, etc.). The bioactive substances such as nucleic acids (e.g., mRNA) are dissolve in the aqueous solvent to obtain an aqueous phase containing bioactive substances. The aqueous phase may contain a small percentage of water miscible organic solvents. The aqueous phase may contain up to 60 volume% of at least one organic solvent that is miscible with water, such as up to about 0.05%, 0.1%, 0.5%, 1%, 2%, 3%, 4%, 5%, 6%, 7%, 8%, 9%, 10%, 15%, 20%, 25%, 30%, 35%, 40%, 45%, 50%, 55%, 60% or any volume% between the two of one organic solvent (e.g., water miscible organic solvent). In an embodiment, the aqueous phase contains between approximately 0.05% and 60% organic solvent by volume, such as between approximately 0.05% and 50%, between approximately 0.05% and 40%, or between approximately 5% and 20% organic solvent (such as a water miscible organic solvent) by volume. The aqueous buffer can be citrate buffer, Tris-HCl buffer, sodium acetate buffer, PBS buffer, or a combination thereof. In some embodiments, the aqueous buffer is a citrate buffer with a pH between 4-6 (e.g., a pH of about 4, about 5, or about 6, etc.). In an embodiment, the aqueous buffer is a citrate buffer with a pH of about 4.

In some embodiments, a solution comprising a mixture of lipid organic phase and an aqueous phase containing bioactive substances can be diluted, and the mixture includes LNP suspension. In some embodiments, the pH of the solution comprising a mixture of a lipid organic phase containing LNP suspension and an aqueous phase containing bioactive substances can be adjusted. The pH of LNP suspension can be diluted or adjusted by adding water, acid, base, or aqueous buffering agents. In some embodiments, the pH of LNP suspension is not diluted or adjusted. In some embodiments, the pH of LNP suspension is diluted or adjusted.

In some embodiments, excess reagents, solvents, and unencapsulated nucleic acids can be removed from LNP suspension by tangential flow filtration (TFF) (such as percolation filtration). Organic solvents (such as ethanol) and buffering agents can also be removed from the LNP suspension using TFF. In some embodiments, the LNP suspension is dialyzed. In some embodiments, the LNP suspension is subjected to TFF. In some embodiments, the LNP suspension is subjected to dialysis and TFF.

The main advantages of the present invention include:
(1) In the present invention, the ionizable lipids can be used in combination with other components, such as auxiliary phospholipids (DSPC, DOPE, DOPC, etc.), sterols (such as cholesterol or cholesterol derivatives), PEG derivatives (such as DMG-PEG lipids, or DMG-PEG substances modified by other groups/or PEG derivatives), to form stable nanoparticles. After encapsulating mRNA, the nanoparticles have uniform particle size, high encapsulation efficiency, and good stability, which can improve the transfection efficiency of mRNA in targeted tissues or cells with low toxicity, thereby making the preventive and therapeutic effects of mRNA vaccines/drugs more prominent.
(2) In the present invention, the targeting of LNP in mouse lymph nodes is achieved through strategies, such as designing ionizable cation structures, adding modifiers, fifth component, and optimizing the proportion of each component, thereby effectively avoiding its expression in organs such as liver, spleen, kidney, and heart, and achieving an up to 97.06% of fluorescence distribution in lymph nodes. The realization of high lymphatic targeting effect provides a technical prerequisite for targeted therapy of diseases, especially for the development of tumor vaccine drugs. This result has important significance for promoting the field of drug targeted delivery.

The present invention is further explained below in conjunction with specific example. It should be understood that these examples are only for illustrating the present invention and not intend to limit the scope of the present invention. The experimental method without specific conditions in the present embodiment is generally in accordance with conventional conditions, or in accordance with the conditions recommended by the commodity manufacturer. Unless otherwise stated, percentages and parts are calculated by weight.

### Example 1 Preparation of ionizable lipid

### 1.1 Synthesis steps and characterization of Compound AL-6:

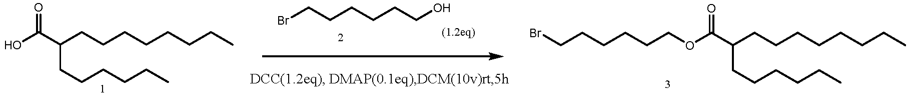

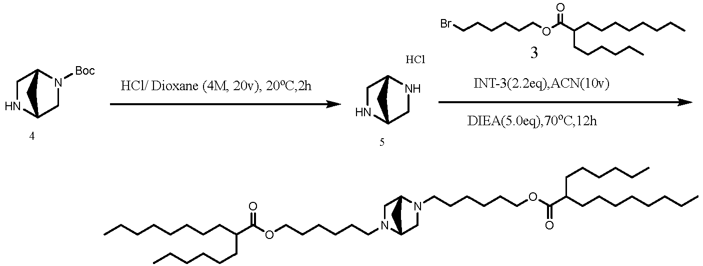

### 1.1.1 Synthesis of 6-Bromohexyl-2-Hexyldecanoate

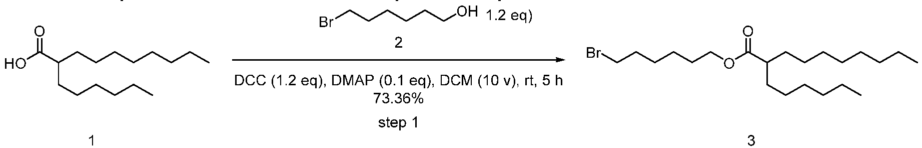

2-hexyl-undecanoic acid (55 g, 214.483 mmol, 1 equiv), dichloromethane (550 mL), 6-bromo-1-hexanol (46.60 g, 257.380 mmol, 1.2 equiv), N,N-dimethylaminopyridine (2.62 g, 21.448 mmol, 0.1 equiv) were added to a 1.0 L three necked bottle, and dicyclohexylcarbodiimide (53.11 g, 257.380 mmol, 1.2 equiv) was added at 0°C. The reaction solution was stirred at 20°C for five hours, and subjected to filtration, and the filter cake was washed with dichloromethane (100mL x 3). The organic phase was concentrated under reduced pressure, And purified through column chromatography, which was washed with n-heptane: ethyl acetate (10:1) and concentrated to obtain 6-bromohexyl-2-hexyldecanoate (66 g, 73.36%) as a pale yellow oil.

LCMS-PH-AXTER-SDPC-2022-08B-6-3: 460 [M+CH₃CN +1] ⁺¹H NMR (400 MHz, Chloroform-d) δ 4.096 (t, *J*=6.4 Hz, 2H), 3.437 (t, *J*=6.8 Hz, 2H), 2.398-2.277 (m, 1H), 1.949-1.837 (m, 2H), 1.713-1.545 (m, 4H), 1.528-1.369 (m, 6H), 1.340-1.222 (m, 20H), 0.900 (t, *J*=6.4 Hz, 6H).

### 1.1.2 Synthesis of (1R,4R)-2,5-diaza-bicyclo[2.2.1]heptane hydrochloride

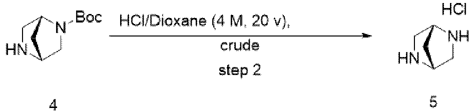

Tert butyl (1R,4R)-2,5-diaza-bicyclo[2.2.1]heptane (1.0 g, 5.044 mmol, 1 equiv) and dioxane hydrochloride gas (10 mL, 4 M) were added to a 50 mL single necked bottle, and the reaction was stirred at room temperature for 2-4 hours, concentrated and dried to give 1.1 g (1R,4R)-2,5-diaza-bicyclo[2.2.1]heptane hydrochloride as a white solid crude product.

LCMS-PH-AXTER-SDPC-2022-08B-6-5: 99 [M+1] ⁺¹H NMR (400 MHz, DMSO-*d₆*) δ 4.422 (s, 2H), 3.550 (d, *J*=13.6 Hz, 2H), 3.329-3.318 (m, 2H), 2.064 (s, 2H).

### 1.1.3 Synthesis of final product

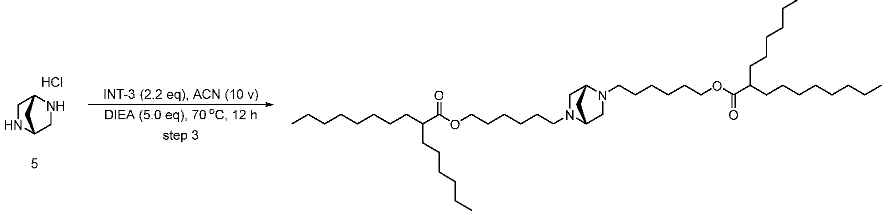

(1R,4R)-2,5-diaza-bicyclo[2.2.1]heptane hydrochloride (0.6 g, 4.457 mmol, 1 equiv), acetonitrile (6 mL), N,N-dimethylethylamine (3.46 g, 26.742 mmol, 6.0 equiv), and 6-bromohexyl-2-hexyldecanoate (4.11 g, 9.805 mmol, 2.2 equiv) were added to a 20 mL single necked bottle. The reaction solution was stirred at 70°C for 12 hours. The reaction solution was cooled to room temperature, diluted with ethyl acetate (120 mL), washed with 30 mL water for three times, dried over anhydrous sodium sulfate, filtered, and concentrated under a reduced pressure to dryness. Purification was carried out using a silica gel column, and the product was obtained at a methanol/ethyl acetate ratio of (5/95). After concentrated and dried, 1.2 g (HPLC: 94.7%) of the product was obtained. 1.2 g (HPLC: 94.7%) of the product was diluted with n-hexane (120 mL), then washed once with methanol/water (3/1, 36 mL), acetonitrile/water (3/1, 36 mL), and water (36 mL), respectively, then dried over anhydrous sodium sulfate, filtered, and concentrated under a reduced pressure to obtain 980 mg (HPLC: 95.4%) of the product. 980 mg (HPLC: 95.4%) product was purified by reverse phase preparation: Prep-HPLC (IntelFlash-1, Column: C18 silica gel; mobile phase A: water (0.5% TFA), phase B: Acetonitrile; 40% to 95% gradient in 25 min, 95% in 5 min; Detector, ELSD). The collected solution was adjusted to pH 8 with a saturated sodium bicarbonate aqueous solution and extracted twice with 60 mL of n-hexane. The merged organic phase was washed once with 30 mL of water, dried over anhydrous sodium sulfate, filtered, concentrated *in vacuo* and dried to give the final product (HPLC: 99.3%) 6-[(1R,4R)-5-{6-[(2-hexyldecyl)oxy] hexyl}-2,5-diazabicyclo[2.2.1]heptan-2-yl]hexyl 2-hexyl decanoate, and 0.5344 g of the product was delivered.

LCMS-PH-AXTER-SDPC-2022-08B-6-0: 775.7 [M +1] ⁺¹H NMR (300 MHz, Chloroform-d) δ 4.059 (t, *J* = 6.6 Hz, 4H), 3.282 (s, 2H) 2.682 (s, 4H), 2.632-2.513 (m, 2H), 2.501-2.392 (m, 2H), 2.380-2.261 (m, 2H), 1.720 (s, 2H), 1.714-1.541 (m, 8H), 1.513-1.196 (m, 56H), 0.876 (t, *J =* 7.2 Hz, 12H).

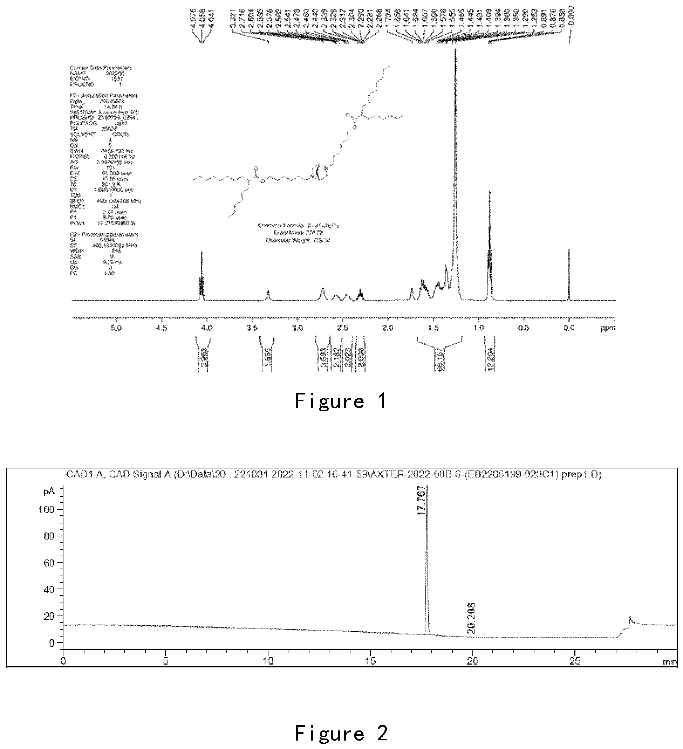

The hydrogen spectrum of AL-6 is shown in Figure 1, and the purity characterization diagram is shown in Figure 2.

### 1.2 Synthesis steps and characterization of Compound AL-17:

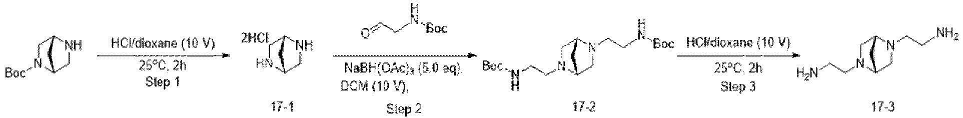

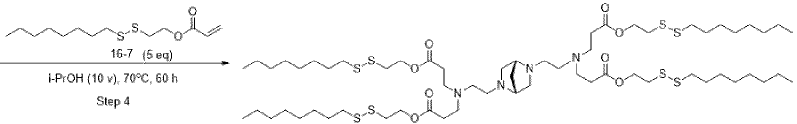

### 1.2.1 Synthesis of (1R,4R)-2,5-diazabicyclo[2.2.1]heptane dihydrochloride

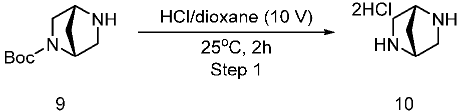

At room temperature, in a 250mL three necked bottle, tert butyl (1R,4R)-2,5-diazabicyclo [2.2.1]heptan-2-carboxylic acid ester (9 g, 45.394 mmol, 1 equiv) was dissolved in hydrogen chloride dioxane solution (90 mL) and stirred at room temperature for 2 hours. The final reaction solution was directly concentrated and dried *in vacuo* to obtain (1R,4R)-2,5-diazabicyclo[2.2.1]heptane dihydrochloride (7.4 g, 95.29%) as a white solid.

LCMS-PH-AXTER-SDPC-2022-07B-17-1: 99 [M+H]⁺¹H NMR (300 MHz, DMSO-*d6*, *ppm*) *δ* 10.038 (s, 4H), 4.477-4.363 (m, 2H), 3.542 (d, *J =* 12.391 Hz, 2H), 3.269 (dd, *J* = 12.394, 2.741 Hz, 2H), 2.058 (s, 2H).

### 1.2.2 Synthesis of Tert butyl N-{2-[(1R,4R)-5-{2-[(tert butylcarbonyl) aminoethyl}-2,5-diazabicyclo[2.2.1]heptan-2-yl] ethyl}carbamate

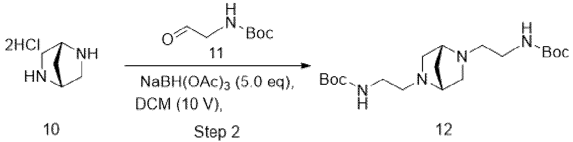

At room temperature, in a 100mL three necked bottle, (1R,4R)-2,5-diazabicyclo[2.2.1]heptane dihydrochlorid (3 g, 17.537 mmol, 1 equiv), methanol (30 mL), N-Boc-2-diaminoacetaldehyde (8.37 g, 52.611 mmol, 3 equiv), NaBH(OAc)₃ (18.58 g, 87.685 mmol, 5 equiv), and glacial acetic acid (3.16 g, 52.611 mmol, 3 equiv) were sequentially added. The reaction mixture was stirred at room temperature for another 10 hours. The reaction was detected as being completed by LCMS. The reaction system was poured into a saturated sodium carbonate solution (90 mL) for quenching, and extracted with DCM (3 x 100 mL)., The organic phases were combined, dried over MgSO₄, filtered, and dred *in vacuo.* The crude product was purified on a silica gel column (PE/EA (1:1)) to give Tert butyl N-{2-[(1R,4R)-5-{2-[(tert butylcarbonyl) aminoethyl}-2,5-diazabicyclo[2.2.1]heptan-2-yl]ethyl}carbamate (3.9 g, 52.11%).

LCMS-PH-AXTER-SDPC-2022-07B-17-2: 385 [M+H]⁺¹H NMR (300 MHz, Chloroform-*d*, *ppm)* δ 5.100 (s, 2H), 3.276 (d, *J* = 2.315 Hz, 2H), 3.161 (t, *J* = 5.385 Hz, 4H), 2.686 (qd, *J* = 9.556, 4.317 Hz, 6H), 2.553 (dt, *J* = 12.223, 6.321 Hz, 2H), 1.682 (s, 2H), 1.461 (s, 18H).

### 1.2.2 Synthesis of 2-[(1R,4R)-5-(2-aminoethyl)-2,5-diazabicyclo[2.2.1]heptan-2-yl]acetamide

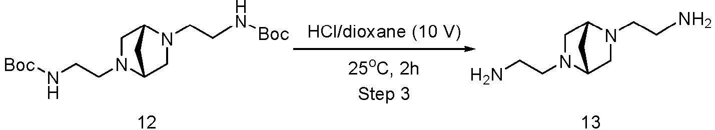

At room temperature, in a 100mL three necked bottle, tert butyl N-{2-[(1R,4R)-5-{2-[(tert butylcarbonyl) aminoethyl}-2,5-diazabicyclo[2.2.1]heptan-2-yl] ethyl} carbamate (3.5 g, 9.102 mmol, 1 equiv) and hydrogen chloride dioxane solution (35 mL) were sequentially added. The reaction was conducted at room temperature for 2 hours, and detected as being completed by LCMS. After dried *in vacuo,* the crude product was ionized using an ion exchange resin (PL-HCO3 MP SPE 500mg/6mL, 50/pk, eluted with ACN/H2O (1:4)) to obtain 2-[(1R,4R)-5-(2-aminoethyl)-2,5-diazabicyclo[2.2.1]heptan-2-yl]acetamide (2 g, crude).

LCMS-PH-AXTER-SDPC-2022-07B-17-3: 185 [M+H]⁺1H NMR (300 MHz, DMSO-*d6*, *ppm*) δ 7.607-7.097 (m, 4H), 3.272 (s, 2H), 2.728 (h, *J* = 7.149, 6.463 Hz, 6H), 2.620 (s, 6H), 1.585 (s, 2H).

### 1.2.4 Synthesis of final product

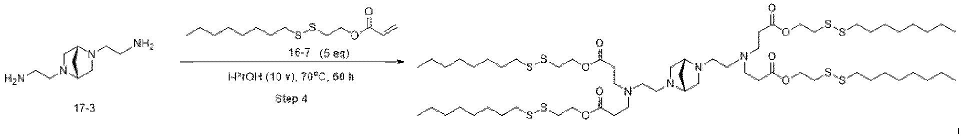

At room temperature, in a 40mL reaction flask, 2-[(1R,4R)-5-(2-aminoethyl)-2,5-diazabicyclo[2.2.1]heptan-2-yl]acetamide (800 mg, 4.341 mmol, 1 equiv), i-PrOH (8 mL), and 2-(octanedithio) ethyl acrylate (6000.42 mg, 21.705 mmol, 5 equiv) were sequentially added and reacted at 70 °C for 60 hours. The final reaction system was concentrated *in vacuo,* and the crude product was purified by Prep-HPLC (column, XB-Phenyl gel; mobile phase, i-PrOH in Water (0.1% TFA), 50% to 90% gradient in 20 min; detector, UV 200 nm; Flow: 90 mL/min.). The received product was evaporated *in vacuo* to remove the organic solution, and the residual aqueous phase was adjusted to pH 9 with sodium carbonate. The alkaline aqueous phase was extracted with EtOAc (2 x 20 mL), and the organic phases were combined, washed once with saturated saline (1 x 20 mL), dried over anhydrous sodium sulfate, fileted and dried *in vacuo.* The residue was dissolved in n-hexane (20 mL), decolorized with activated carbon (200 mg), filtered, and dried *in vacuo* To give the final product 7 (1.0039 g, 17.32%) as a yellow oil.

LCMS-PH-AXTER-SDPC-2022-07B-17-0: 1289.7 [M+H]⁺1H NMR (300 MHz, Chloroform-*d*, *ppm)* δ 4.342 (t, *J* = 6.697 Hz, 8H), 3.281 (s, 2H), 2.909 (t, *J* = 6.743 Hz, 8H), 2.825 (t, *J* = 7.184 Hz, 8H), 2.752-2.672 (m, 12H), 2.550 (p, *J =* 4.122 Hz, 6H), 2.480 (t, *J =* 7.123 Hz, 8H), 1.763-1.629 (m, 10H), 1.426-1.254 (m, 42H), 0.932-0.869 (m, 12H).

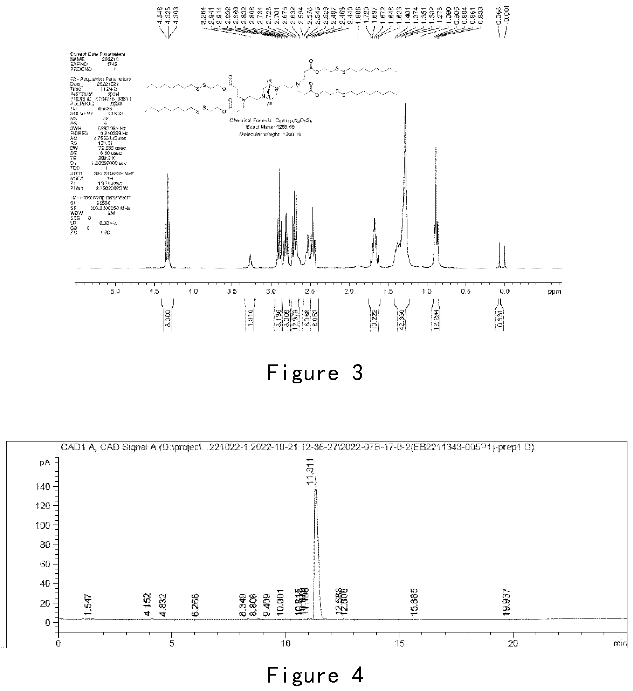

The hydrogen spectrum of AL-17 is shown in Figure 3, and the HPLC purity characterization diagram is shown in Figure 4.

### 1.3 Synthesis steps and characterization of Compound AL-18:

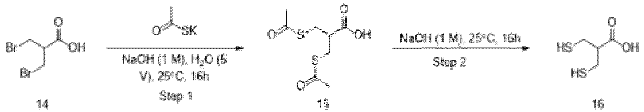

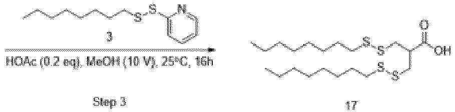

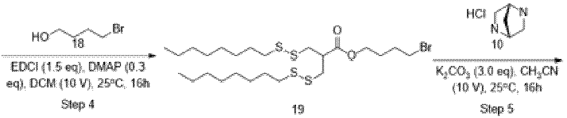

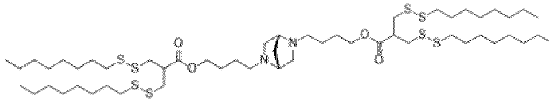

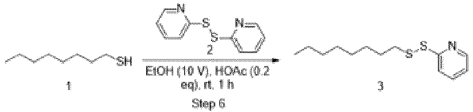

### 1.3.1 Synthesis of 3-(Acetylsulfonyl)-2-[(Acetylsulfonyl)methyl] propionic acid

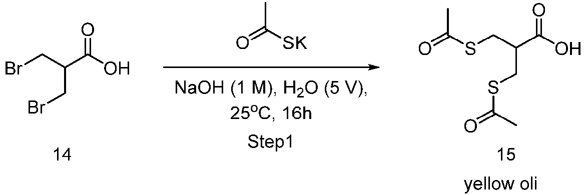

3-bromo-2-(bromomethyl) propionic acid (20 g, 81.335 mmol, 1 equiv), 1-(potassium sulfonyl) ethanone (23.22 g, 203.337 mmol, 2.5 equiv), and NaOH (1 equiv, 1 M) were added to a 250ml round bottom flask and dissolved in water (100ml) at room temperature. The obtained mixture was stirred at room temperature for 16 hours. 1 M HCl was used for acidification until a white emulsion was formed, which was extracted three times with ethyl acetate, and the combined organic layers were washed with brine (pH ≈ 1) and then dried over anhydrous Na₂SO₄. The filtrate was filtered and concentrated under a reduced pressure, thereby obtaining 3-(acetylsulfonyl)-2-[(acetylsulfonyl)methyl] propionic acid as a yellow oil, which was used directly for the next reaction without further purification.

LCMS-PH-AXTER-SDPC-2022-07B-18-3: 235 [M-1]-

### 1.3.2 Synthesis of 3-sulfonyl-2-(sulfonylmethyl) propionic acid

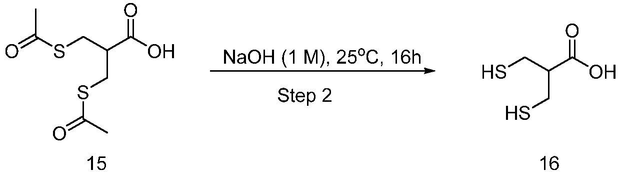

3-(acetylsulfonyl)-2-[(acetylsulfonyl)methyl] propionic acid (13 g, 55.085 mmol, 1 equiv) and NaOH (1M, 10V) were added to a three necked round bottom flask at 0 °C, and NaOH (13 g, 55.085 mmol, 1 equiv) was added in batches. The obtained mixture was stirred at room temperature for another 16 hours, then cooled to 0 °C and acidified with HCl (6M) until a white emulsion was formed, which was extracted three times with ethyl acetate, and the organic layers were combined and dried over anhydrous Na₂SO₄. The filtrate was filtered and concentrated under a reduced pressure, thereby obtaining 3-sulfonyl-2-(sulfonylmethyl) propionic acid as a yellow oil.

LCMS-PH-AXTER-SDPC-2022-07B-18-3: 151 [M-1]-

### 1.3.3 Synthesis of 3-(octyldisulfuryl)-2-[(octyldisulfuryl)methyl] propionic acid

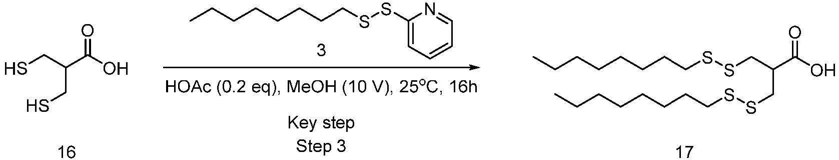

2-(octyldisulfuryl) pyridine (37.2g, 140.053 mmol, 3 equiv), AcOH (584mg, 9.737 mmol, 0.2 equiv), and MeOH (37ml, 5V) were added to a round bottom flask, and 3-sulfonyl-2-(sulfonylmethyl) propionic acid (7.4g, 48.684mmol, 1 equiv) was added dropwise at room temperature (diluted with 5V MeOH). The obtained mixture was stirred at room temperature for another 16 hours and then evaporated *in vacuo.* The residue was purified by silica gel column chromatography, and eluted with n-heptane/EA (20:1) to obtain 3-(octyldisulfuryl)-2-[(octyldisulfuryl)methyl] propionic acid as a yellow oil.
LCMS-PH-AXTER-SDPC-2022-07B-18-4: 439 [M-1]-
1H NMR (400 MHz, Chloroform-d) δ 3.354-3.287 (m, 1H), 3.125-3.074 (m, 2H), 3.034-2.984 (m, 2H), 2.746 (t, J = 7.6 Hz, 4 H), 1.735-1.680 (m, 4H), 1.561-1.270 (m, 20 H), 0.922-0.877 (m, 6H).

### 2.3.4 Synthesis of 4-bromobutyl 3-(octyldisulfuryl)-2-[(octyldisulfuryl)methyl] propionate

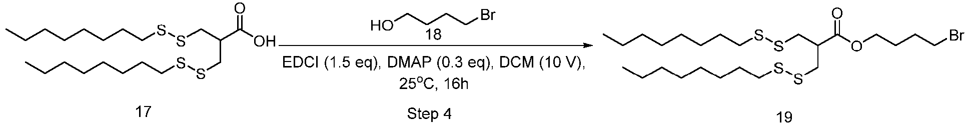

3-(octyldisulfuryl)-2-[(octyldisulfuryl)methyl] propionic acid (4.7g, 10.656 mmol, 1 equiv), 4-bromobutan-1-ol (4.9 g, 31.973 mmol, 3 equiv), DMAP (390 mg, 3.197 mmol, 0.3 equiv), and DCM (25 mL, 5 V) were added to a round bottom flask at room temperature. EDCI (3.1g, 15.986 mmol, 1.5 equiv) was added dropwise to the above mixture (dissolved in 25mL DCM). The obtained mixture was stirred at room temperature for 16 hours. The obtained mixture was filtered, the filter cake was washed with DCM, and the filtrate was concentrated at a reduced pressure. The residue was urified by silica gel column chromatography, and eluted with n-heptane to obtain 4-bromobutyl 3-(octyldisulfuryl)-2-[(octyldisulfuryl)methyl] propionate as a yellow oil.

1H NMR (400 MHz, Chloroform-d) δ 4.198 (t, J= 6.4 Hz, 2H), 3.464 (t, J=6.4 Hz, 2H), 3.278-3.243 (m, 1H), 3.067-2.792 (m, 4H), 2.730 (t, J=7.2 Hz, 4H), 2.024-1.954 (m, 2H), 1.875-1.821 (m, 2H), 1.741-1.651 (m, 4H), 1.417-1.264 (m, 20H), 0.917-0.883 (m, 6H).

### 1.3.5 Synthesis of 2-(OctylDisulfuryl) Pyridine

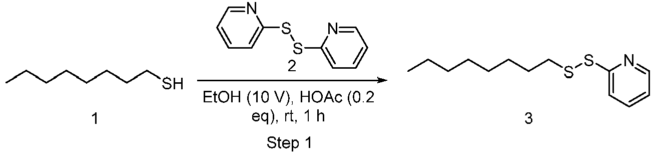

2,2'-bipyridine disulfide (60 g, 273.972 mmol, 2 equiv) was dissolved in ethanol (100 ml, 5V), and HOAc (1.6 g, 27.397 mmol, 0.2 equiv) was added at room temperature. Then, 1-octanethiol (20 g, 136.986 mmol, 1 equiv) (diluted with 100 mL EtOH) was added dropwise in an atmosphere of nitrogen and stirred at room temperature for another hour. The reaction solution was concentrated under a reduce pressure. The residue was purified by column chromatography, and eluted with PE/EA (10:1) mobile phase to obtain 2-(octyldisulfuryl) pyridine as a yellow oil.
LCMS: 256 [M+1]+
1H NMR (400 MHz, Chloroform-d) δ 1H NMR (400 MHz, DMSO-d6) δ 8.560-8.371 (m, 1H), 7.832 (td, J = 7.7, 1.9 Hz, 1H), 7.764 (d, J = 8.0 Hz, 1H), 7.277-7.191 (m, 1H), 2.835 (t, J = 7.2 Hz, 2H), 1.616 (p, J = 7.3 Hz, 2H), 1.345 (dq, J = 12.9, 6.7 Hz, 2H), 1.221 (s, 8H), 0.846 (t, J = 6.7 Hz, 3H).

### 1.3.6 Synthesis of final product

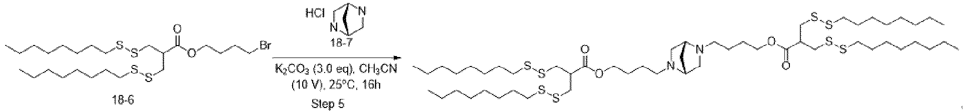

4-bromobutyl 3-(octyldisulfuryl)-2-[(octyldisulfuryl)methyl] propionic acid (3.5 g, 6.076 mmol, 3 equiv), (1R,4R)-2,5-diazabicyclo[2.2.1]heptane (269 mg, 2.025 mmol, 1 equiv), and K2CO3 (838 mg, 6.076 mmol, 3 equiv) were added to a 50 ml round bottom flask, MeCN (30 mL, 10V) was added, and the obatined mixture was stirred at room temperature for 16 hours. Then it was extracted three times with ethyl acetate, and the organic layers were combined and dried over anhydrous Na₂SO₄. After filtration, the filtrate was concentrated under a reduced pressure. The crude product was purified by preparative high performance liquid chromatography under the following conditions: chromatographic column, xb-phenyl gel; Mobile phase, i-PrOH/MeCN (1/1) and water (with 0.1% TFA added), gradient of 50% to 90% for 20 minutes; Detector, UV 200 nm; flow rate: 90mL/min). The obtained fraction was concentrated under vacuum to remove organic solvents and extracted with n-hexane (2 × 40 mL). The combined organic layers were washed with brine (1 × 40 mL) and dried over anhydrous Na₂SO₄. The filtrate was concentrated under a reduced pressure to obtain the crude product (600 mg, purity 82.8%), and then prepared again with high pressure preparative chromatography under preparation conditions (chromatographic column, xb-phenyl gel; Mobile phase, i-PrOH/MeCN (1/1) and water (without additives), gradient of 50% to 90% for 25 minutes; Detector, UV 200 nm; flow rate: 90mL/min). The obtained solution was concentrated under vacuum to remove organic solvents. The residue was extracted twice with n-hexane (2 × 40 mL). The combined organic layers were washed with brine (1 × 40 mL) and dried over anhydrous Na₂SO₄. After filtration, the filtrate was concentrated under a reduced pressure, then the final product 4-[(1R,4R)-5-(4-{[3-(octyldisulfuryl)-2-[(octyldisulfuryl)methyl]propyl] oxy}butyl)-2,5-diazabicyclo[2.2.1]heptan-2-yl]butyl 3-(octyldisulfuryl)-2-[(octyldisulfuryl)methyl] propionate (130 mg, purity 63.4%) was obtained as a yellow oil.
LCMS- PH-AXTER-SDPC-2022-07B-18-3: 1087 [M+1]⁺
¹H NMR (400 MHz, Chloroform-d) *δ* 4.197-4.121 (m, 4H), 3.454-3.339 (m, 2H), 3.245 (p, *J* = 6.8 Hz, 2H), 3.304-2.944 (m, 8H), 2.796-2.481 (m, 14H), 2.592-2.494 (m, 2H), 1.760-1.652 (m, 16H), 1.445-1.202 (m, 42H), 0.90 (d, *J =* 6.9 Hz, 12H).

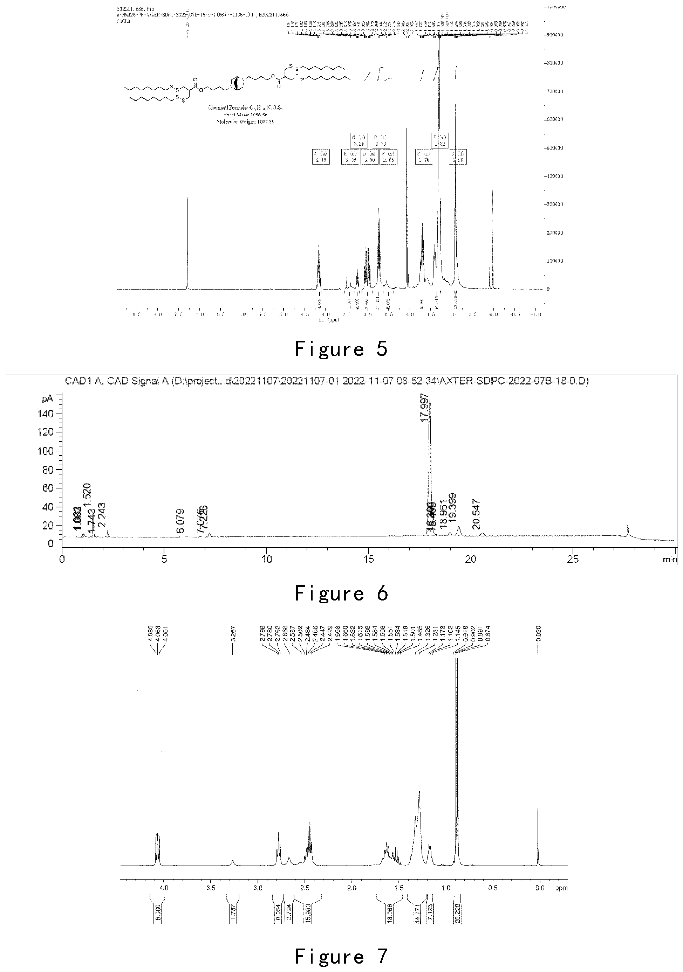

The hydrogen spectrum of AL-18 is shown in Figure 5, and the HPLC purity characterization diagram is shown in Figure 6.

### 1.4 Synthesis steps and characterization of Compound AL-19:

The synthesis process of AL-19 is as follows:

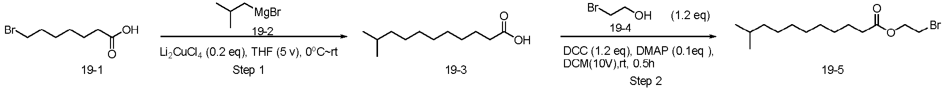

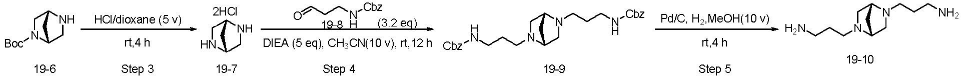

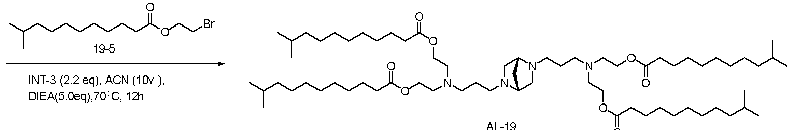

### 1.4.1 Synthesis of 19-3

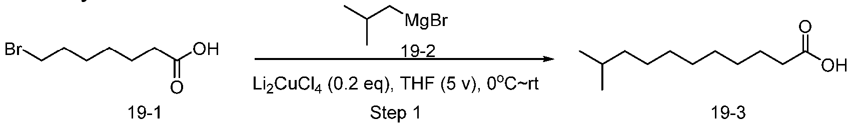

7-Bromoheptac acid (21 g, 100.47 mmol, 1 equiv) was dissolved in THF (125 mL), and Li₂CuCl₄ (128 mL, 0.2 M, 0.2 equiv) was added under a nitrogen atmosphere at 0 °C for 10 minutes. Then, isobutyl magnesium bromide (193 mL, 1 M, 1.5 equiv) was added dropwise at 0 °C. The reaction solution was stirred at room temperature for another 2 hours. The reaction was monitored using thin-layer chromatography. The reaction solution was quenched with hydrochloric acid (1 M) and extracted with ethyl acetate (3 x 100 mL). The combined organic layers were washed with water (2×100 mL) and dried over anhydrous Na₂SO₄. After filtration, the filtrate was concentrated at a reduced pressure. The residue was purified by silica gel column chromatography and eluted with PE/EA (10:1) to obtain the product 19-3 (18 g, 81.2%) as a yellow oil.

The hydrogen spectrum characterization result of 19-3 is as follows: ¹H NMR (300 MHz, Chloroform-d)δ3.639 (t, J = 6.6 Hz, 2H), 1.603- 1.459 (m, 3H), 1.367-1.238 (m, 12H), 0.862 (d, J = 6.6 Hz, 6H).

### 1.4.2 Synthesis of 19-5

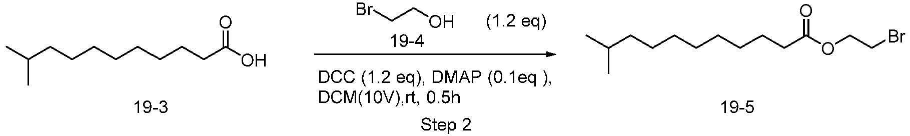

19-3 (10.656 mmol, 1 equiv), 4-bromobutan-1-ol (4.9 g, 31.973 mmol, 3 equiv), DMAP (390 mg, 3.197 mmol, 0.3 equiv), and DCM (25 mL, 5 V) were added to a round bottom flask at room temperature. EDCI (3.1g, 15.986 mmol, 1.5 equiv) was added dropwise to the above mixture (dissolved in 25 mL DCM). The obtained mixture was stirred at room temperature for 16 hours. The obtained mixture was filtered, the filter cake was washed with DCM, and the filtrate was concentrated at a reduced pressure. The residuce was purified by silica gel column chromatography, and eluted with n-heptane to obtain the product 19-5.

The hydrogen spectrum characterization result of 19-5 is as follows: ¹H NMR (300 MHz, Chloroform-d) δ 6.396 (dd, J = 17.3, 1.6 Hz, 1H), 6.119 (dd, J = 17.3, 10.4 Hz, 1H), 5.808 (dd, J = 10.4, 1.6 Hz, 1H), 4.150 (t, J = 6.8 Hz, 2H), 1.754 - 1.605 (m, 2H), 1.568 - 1.474 (m, 1H), 1.403 - 1.134 (m, 13H), 0.862 (d, J = 6.6 Hz, 6H).

### 1.4.3 Synthesis of 19-7

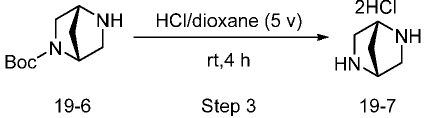

At room temperature, tert butyl (1R, 4R) -2,5-diazaheterocyclic[2.2.1] n-heptane-2-carboxylate (5 g, 25.219 mmol, 1 equiv) and dioxane solution (50 mL) in hydrochloric acid were added to a 250 mL three necked round bottom flask, and stirred at room temperature for 3 hours. The mixture was concentrated at a reduced pressure to obtain (1R,4R)-2,5-diazaheterocyclic[2.2.1]heptane as a white solid. It is not necessary to purify the crude product which could be directly used in the next step.

The hydrogen spectrum characterization result of 19-7 is as follows: ¹H NMR (300 MHz, DMSO-d6) δ 9.835 (s, 3H), 4.423 (d, *J =* 2.6 Hz, 2H), 3.588-3.477 (m, 2H), 3.299 (s, 2H).

### 1.4.4 Synthesis of 19-9

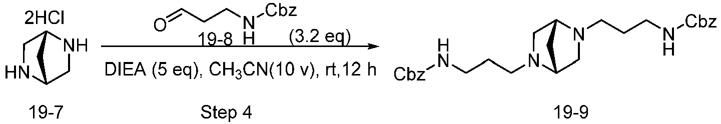

At room temperature, (1R,4R)-2,5-diazabicyclo[2.2.1]heptane (2.1 g, 12.35 mmol, 1 equiv) and MeOH (21.00 mL), benzyl (3-oxopropyl) carbamate (8.2 g, 39.53 mmol, 3.2 equiv), and STAB (13.0 g, 61.76 mmol, 5 equiv) were added to a 100 mL round bottom flask. The reaction solution was stirred at room temperature for another 12 hours. The mixture was concentrated at a reduced pressure. The residue was purified by silica gel column chromatography and eluted with CH₂Cl₂/MeOH(5:1) to give the product 19-9 (3.3 g, 55.7%) as a yellow oil.

The hydrogen spectrum characterization result of 19-9 is as follows: ¹H NMR (400 MHz, Chloroform-d) δ 7.395-7.273 (m, 10H), 5.541 (s, 2H), 5.085 (s, 4H), 3.626 (s, 2H), 3.268 (s, 4H), 3.250-2.321 (m, 8H), 1.979-1.501 (m, 6H).

### 1.4.5 Synthesis of 19-10

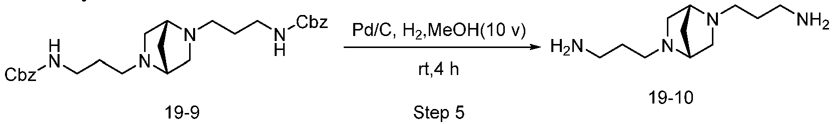

N-{3-[(1R,4R)-5-(3-{[(benzyloxy)carbonyl]amino}propyl)-2,5-diazabicyclo[2.2.1]heptan-2-yl]propyl} carbamate (3.3 g, 6.87 mmol, 1 equiv) and Pd/C (1.65 g, 50% w/w) were dissolved in MeOH (33 mL) and stirred at room temperature under a hydrogen atmosphere for 2 hours. After filtration, the filter cake was washed with MeOH (2x100 mL). The filtrate was concentrated at a reduced pressure to give 3-[(1R,4R)-5-(3-aminopropyl)-2,5-diazabicyclo[2.2.1]heptan-2-yl] propan-1-amine (1.33 g, 91.7%) as an off-white oil.

The hydrogen spectrum characterization result of 19-10 is as follows: ¹H NMR (400 MHz, Methanol-*d₄*) δ 2.874 (t, *J* = 6.9 Hz, 4H), 2.797 (d, *J* = 10.5 Hz, 3H), 2.763-2.693 (m, 3H), 2.684-2.598 (m, 4H), 1.713 (dd, *J =* 16.0, 9.0 Hz, 10H).

### 1.4.6 Synthesis of final product AL-19

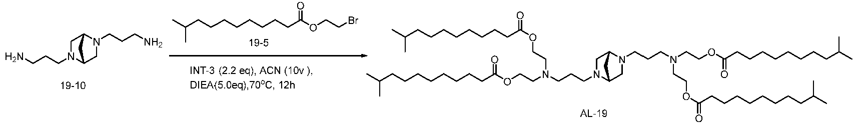

The product 19-10 (4.457 mmol, 1 equiv), acetonitrile (6 mL), N,N-dimethylethylamine (3.46 g, 26.742 mmol, 6.0 equiv), and product 19-5 (43.142 mmol, 4.4 equiv) were added to a 20 mL single necked bottle. The reaction solution was stirred at 70°C for 12 hours. The reaction solution was cooled to room temperature, diluted with ethyl acetate (120 mL), washed with 30 mL water for three times, dried over anhydrous sodium sulfate, filtered, and concentrated under a reduced pressure to dryness. The residue was purified using a silica gel column to obtain the product at a methanol/ethyl acetate ratio of (5/95), which was concentrated and dried to give 1.2 g (HPLC: 94.7%) of the product. 1.2 g (HPLC: 94.7%) of the product was diluted with n-hexane (120 mL), then washed once with methanol/water (3/1, 36 mL), acetonitrile/water (3/1, 36 mL), and water (36 mL), respectively, then dried over anhydrous sodium sulfate, filtered, and concentrated under a reduced pressure to obtain 980 mg (HPLC: 95.4%) of the product. 980 mg (HPLC: 95.4%) product was purified by reverse phase preparation: Prep-HPLC (IntelFlash-1, Column: C18 silica gel; mobile phase A: water (0.5% TFA), phase B: Acetonitrile; 40% to 95% gradient in 25 min, 95% in 5 min; Detector, ELSD). The collected solution was adjusted to pH = 8 with a saturated sodium bicarbonate aqueous solution and extracted twice with 60 mL of n-hexane. The combined organic phase was washed once with 30 mL of water and dried over anhydrous sodium sulfate. After filtration, the filtrate was concentrated at a reduced pressure to give the product AL-19.

The hydrogen spectrum characterization result of Al-19 is as follows: ¹H NMR (400 MHz, Chloroform-d) δ 4.048 (t, *J* = 6.8 Hz, 8H), 3.247 (s, 2H), 2.761 (t, *J =* 7.3 Hz, 8H), 2.649 (s, 4H), 2.446 (dt, *J* = 14.6, 7.1 Hz, 16H), 1.598-1.501 (m, 18H), 1.362-1.209 (m, 40H), 1.186-1.108 (m, 8H), 0.863 (d, *J* = 6.6 Hz, 24H).

LCMS: 1118 [M+H]⁺ is the target molecular weight; and the purity reached 95.14%.

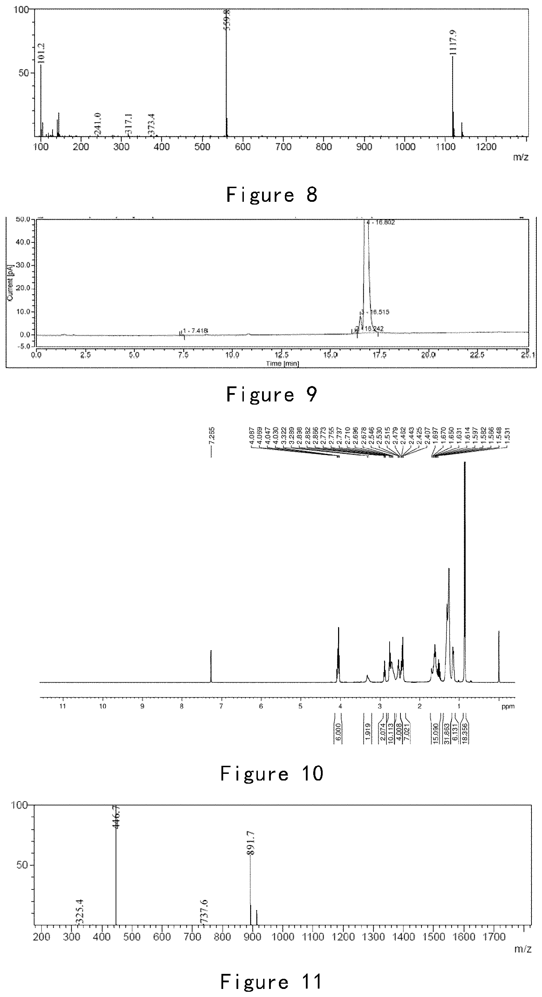

The hydrogen spectrum characterization results of AL-19 are shown in Figure 7, the LCMS results are shown in Figure 8, and the HPLC purity detection results are shown in Figure 9.

### 1.5 Synthesis steps and characterization of Compound AL-20

The synthesis process of AL-20 is as follows:

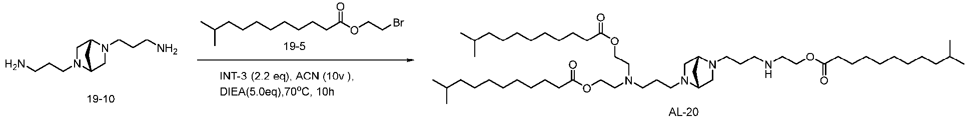

3-[(1R,4R)-5-(3-aminopropyl)-2,5-diazabicyclo[2.2.1]heptan-2-yl]propan-1-amine (19-10) (4.457 mmol, 1 equiv), acetonitrile (6 mL), N,N-dimethylethylamine (3.46 g, 26.742 mmol, 6.0 equiv), and product 19-5 (11.14 mmol, 2.5 equiv) were added to a 20 mL single necked bottle. The reaction solution was stirred at 70°C for 10 hours. The reaction solution was cooled to room temperature, diluted with ethyl acetate (120 mL), washed with 30 mL water for three times, dried over anhydrous sodium sulfate, filtered, and concentrated under a reduced pressure to dryness. The residue was purified by using a silica gel column, and methanol/ethyl acetate=(5/95) was used as a mobile phase. The elued solution was concentrated and dried to obtain 1.2 g (HPLC: 94.7%) of the product. 1.2 g (HPLC: 94.7%) of the product was diluted with n-hexane (120 mL), then washed once with methanol/water (3/1, 36 mL), acetonitrile/water (3/1, 36 mL), and water (36 mL), respectively, then dried over anhydrous sodium sulfate, filtered, and concentrated under a reduced pressure to obtain 980 mg (HPLC: 95.4%) of the product. 980 mg (HPLC: 95.4%) product was purified by reverse phase preparation: Prep-HPLC (IntelFlash-1, Column: C18 silica gel; mobile phase A: water (0.5% TFA), phase B: Acetonitrile; 40% to 95% gradient in 25 min, 95% in 5 min; Detector, ELSD). The collected solution was adjusted to pH = 8 with a saturated sodium bicarbonate aqueous solution and extracted twice with 60 mL of n-hexane. The combined organic phase was washed once with 30 mL of water, dried over anhydrous sodium sulfate, filtered and concentrated at a reduced pressure to give the product AL-20.

LCMS: 892 [M+H]⁺ is the target molecular weight.

The hydrogen spectrum characterization result of AL-20 is as follows: ¹H NMR (400 MHz, Chloroform-*d*, *ppm)* δ 4.157-3.974 (m, 6H), 3.343-3.307 (m, 2H), 2.902 (t, *J =* 6.5 Hz, 2H), 2.792-2.663 (m, 10H), 2.550 (t, *J =* 6.4 Hz, 4H), 2.499-2.426 (m, 8H), 1.771-1.451 (m, 15H), 1.413-1.221 (m, 30H), 1.193-1.143 (m, 6H), 0.882 (d, *J =* 6.5 Hz, 18H).

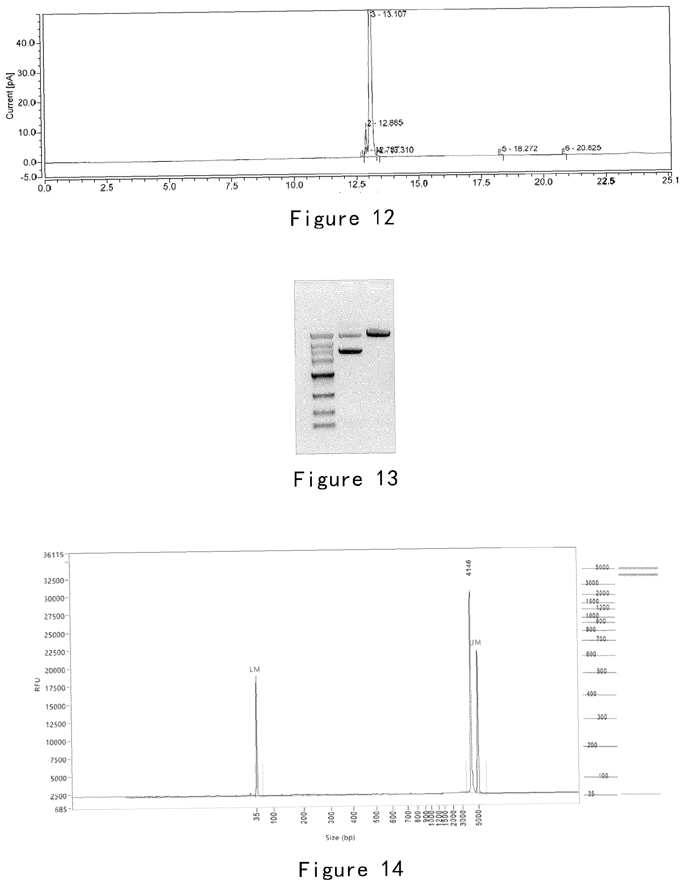

The hydrogen spectrum characterization results of AL-20 are shown in Figure 10, the LCMS results are shown in Figure 11, and the HPLC purity detection results are shown in Figure 12.

### Example 2 Synthesis Process, Quality Control Method, and Results of mRNA

### Preparation of mRNA for delivery:

The plasmid containing the transcription template was cultured on a shaker at 30°C for 24 hours. The plasmid was extracted and purified using a plasmid extraction kit, and then digested with Bsa I enzyme and purified using beads to obtain a linearized transcription template. The concentration was measured using Nanodrop One, the purity of the transcription template was analyzed using Agilent Fragment Analyzer, and the purity of the transcription template was determined to be between 98-100% using dsDNA 915 Reagent Kit.

mRNA was prepared using a two-step method. The IVT system was reacted at 37 °C for 3 hours. The DNA template was digested by DNase I and purified by RNA Clean Beads. The concentration was measured using Nanodrop One and adjusted to 1 mg/mL. The RNA used for capping was treated at 65 °C for 10 minutes, quickly placed on ice, and the secondary structure of the RNA was opened. The capping system was left to react at 37 °C for 1 hour, purified by RNA Clean Beads, and the concentration was measured using Nanodrop One. The concentration was adjusted to 1 mg/mL and stored at -20 °C after packaging.

The purity of the transcription template was analyzed using Agilent Fragment Analyzer, and the purity of the transcription template was determined to be between 95%-100% using RNA Kit (15nt). The A260/A280 ratio was determined to be around 1.9 using Nanodrop One. The residual dsRNA was detected to be 1.8-2.5 ng/µg using ELISA method. The residual DNA was detected to be 3-30 pg/µg using fluorescence quantitative PCR method.

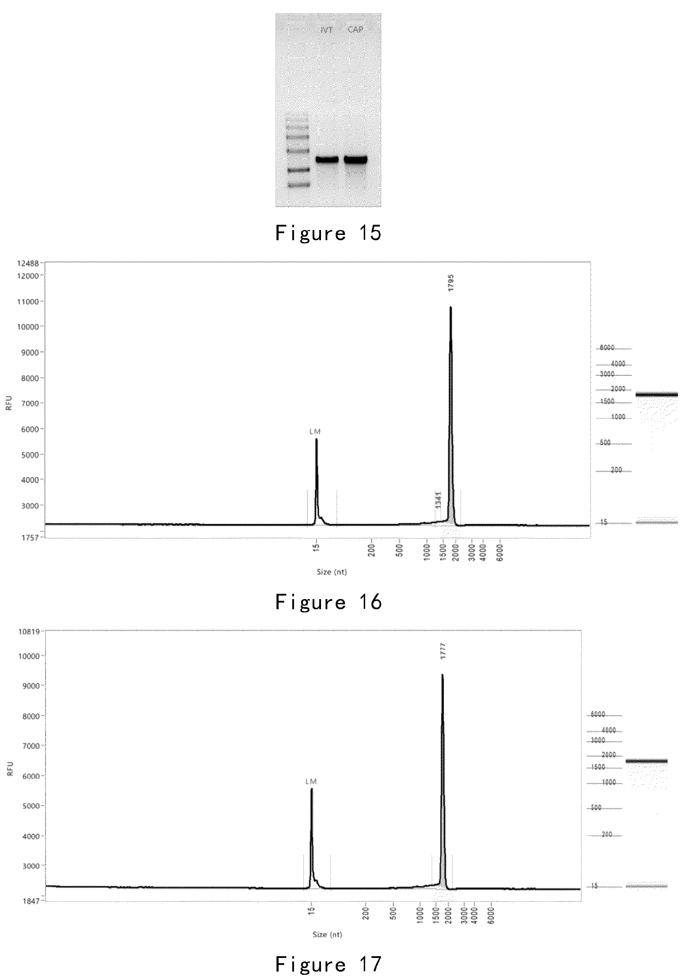

The electrophoresis map of the transcription template, the integrity peak map of the transcription template, the electrophoresis map after *in vitro* transcription reaction (IVT), the RNA integrity peak map of IVT, and the mRNA integrity peak map are shown in Figures 13, 14, 15, 16, and 17, respectively.

### Example 3 Assembly of LNP mRNA by encapsulating mRNA with ionizable lipids

### 3.1 Preparation Example 1 of Lipid Nanoparticles (LNP mRNA)

Ionizable lipids (SM-102, AL-6, AL-17, AL-19, and AL-20), DSPC (distearoylphosphatidylcholine, 1,2-distearoyl-sn-glycerin-3-phosphocholine), cholesterol, and DMG-PEG2000 were mixed in different proportions in ethanol phase and thoroughly mixed as the organic phase. mRNA was dissolved in sodium citrate (pH=4.0) solution and thoroughly mixed as the aqueous phase. The aqueous and organic phases were transferred to suitable BD syringes, respectively, and the bubbles were removed as much as possible. Using a PNI nanogenerator, the aqueous and organic phases were mixed at a volume ratio of 3:1 and a flow rate of 12 mL/min. After the sample was stabilized, purification, concentration, filtration, and sterilization were carried out. The obtained product was lipid nanoparticles with encapsulated mRNA (LNP mRNA). The final product LNP mRNA was subjected to physical and chemical quality control.

### 3.2 Preparation Example 2 of Lipid Nanoparticles (LNP mRNA)

1) Ionizable lipid AL-6 (or AL-19), DSPC, Chol, DMG-PEG₂₀₀₀, DOPA (or DOPS, DMG-PEG₂₀₀₀-mannose) were mixed at different molar ratios in ethanol phase and thoroughly mixed using a vortex analyzer;
2) mRNA was dissolved in a 50 mM sodium acetate solution (pH=4.0) and thoroughly mixed as the aqueous phase;
3) The aqueous and organic phases were transferred to suitable BD syringes, and the bubbles were removed;
4) Using a PNI nanogenerator, the aqueous and organic phases were mixed at a volume ratio of 3:1 and a flow rate of 12 mL/min. Purification, concentration, filtration, and sterilization were performed sequentially to obtain lipid nanoparticles (AXT-LNP-x, where x represents different numbers such as 01, 02, 03, etc.) with encapsulated mRNA, as shown in Table 1;
5) The final product LNP mRNA was subjected to physical and chemical quality control.

**Table 1**

| Molar% (molar ratio) | | | | | | | |
|---|---|---|---|---|---|---|---|
| Formula name | AL-6 | DSPC | Chol | DMG-PEG₂₀₀₀ | DOPA | DOPS | DMG-PEG₂₀₀₀-mannose |
| AXT-LNP-01 | 50(50) | 10(10) | 38.5(38.5) | 1.5(1.5) | / | / | / |
| AXT-LNP-02 | 47.5(50) | 9.5(10.0) | 36.6(38.5) | 1.4(1.5) | 5.0(5.3) | / | / |
| AXT-LNP-03 | 42.5(50) | 8.5(10.0) | 32.7(38.5) | 1.3(1.5) | 15.0(17.7) | / | / |
| AXT-LNP-04 | 37.5(50) | 7.5(10) | 28.9(38.5) | 1.1(1.5) | 25.0(33.4) | / | / |
| AXT-LNP-05 | 47.6(50) | 9.5(10) | 36.7(38.5) | 1.4(1.5) | / | 4.8(5) | / |
| AXT-LNP-06 | 45.4(50) | 9.1(10) | 35.0(38.5) | 1.4(1.5) | / | 9.1(10) | / |
| AXT-LNP-07 | 41.6(50) | 8.3(10) | 32.1(38.5) | 1.3(1.5) | / | 16.7(20 ) | / |
| AXT-LNP-08 | 50(50) | 10(10) | 38(38) | 1.5(1.5) | / | / | 0.5(0.5) |
| AXT-LNP-09 | 50(50) | 10(10) | 37.5(37.5) | 1.5(1.5) | / | / | 1(1) |
| AXT-LNP-10 | 50(50) | 10(10) | 37(37) | 1.5(1.5) | / | / | 1.5(1.5) |
| AXT-LNP-11 | 50(50) | 10(10) | 36(36) | 1.5(1.5) | / | / | 2.5(2.5) |

| Molar% (molar ratio) | | | | | | | |
|---|---|---|---|---|---|---|---|
| Formula name | AL-19 | DSPC | Chol | DMG-PEG₂₀₀₀ | DOPA | DOPS | |
| AXT-LNP-12 | 50(50) | 10(10) | 38.5(38.5) | 1.5(1.5) | / | / | |
| AXT-LNP-13 | 40(40) | 10(10) | 48.5(48.5) | 1.5(1.5) | / | / | |
| AXT-LNP-14 | 40(40) | 15(15) | 43.5(43.5) | 1.5(1.5) | / | / | |
| AXT-LNP-15 | 38.0(40) | 14.2(15) | 41.3(43.5) | 1.4(1.5) | 5.0(5.3) | | |
| AXT-LNP-16 | 36.0(40) | 13.5(15) | 39.2(43.5) | 1.4(1.5) | 10.0(11.1) | | |
| AXT-LNP-17 | 34.0(40) | 12.7(15) | 37.0(43.5) | 1.3(1.5) | 15.0(17.7) | | |
| AXT-LNP-18 | 38.1(40) | 14.3(15) | 41.4(43.5) | 1.4(1.5) | / | 4.8(5) | |
| AXT-LNP-19 | 36.4(40) | 13.6(15) | 39.5(43.5) | 1.4(1.5) | / | 9.1(10) | |
| AXT-LNP-20 | 33.2(40) | 12.5(15) | 36.3(43.5) | 1.3(1.5) | / | 16.7(20) | |

### 3.3 Quality Control Method, and Results of LNP-mRNA:

1) Particle size and PDI (distribution): A nanoparticle analyzer was used to detect the particle size and distribution of LNP.
2) Encapsulation rate (EE%): Total mRNA and free mRNA were stained using RiboGreen, and then detected using an enzyme-linked immunosorbent assay (ELISA) to calculate the encapsulation rate.
3) pH: the pH was determined to be 7.2-7.4 using a pH meter.
4) Osmotic pressure: A freezing point osmometer was used to detect the osmotic pressure of LNP mRNA. The osmotic pressure at a concentration of 100 µg/mL was 280-310 mOsmol/kg.
5) mRNA integrity: The integrity of encapsulated mRNA (e.i., the purity of mRNA) was analyzed using Agilent Fragment Analyzer, and the purity was determined to be over 90% using RNA Kit (15nt).

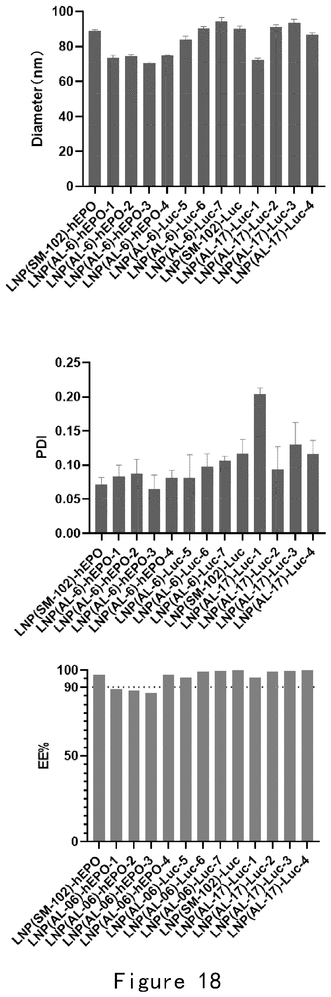

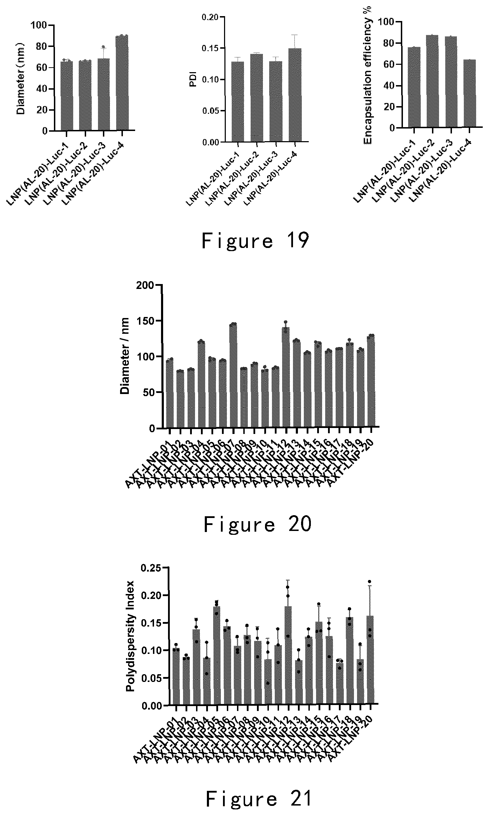

Some detection results are shown in Figures 18-22.

### Example 4 Detection of In vivo expression of LNP-mRNA

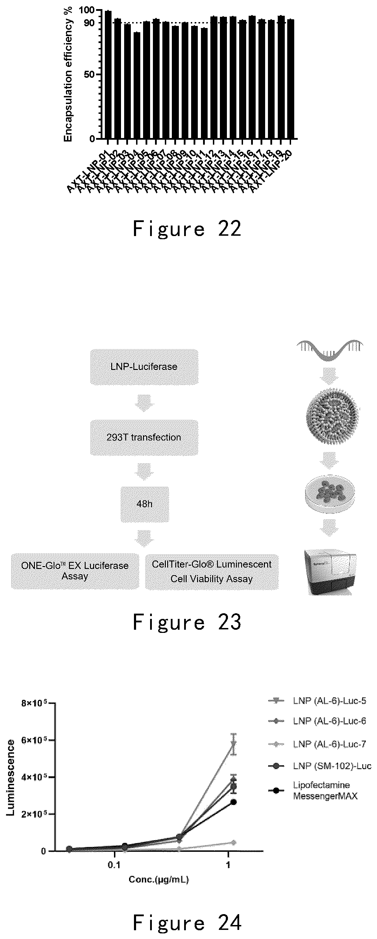

Cell expression screening experiments on LNP-mRNA prepared in Example 3.1 were performed according to the process shown in Figure 23. The specific steps are as follows:
1) Cell plating: After digestion of 293T cells, the cell density was adjusted to 2 × 10⁴/well and the cells were inoculated in a 96 well plate at 100 µL/well, then the plate was placed in a cell culture incubator overnight.
2) Experimental grouping: LNP (AL-6)-Luc and LNP (AL-17)-Luc series products were used as sample groups, LNP (SM-102)-Luciferase was used as the positive control group, and the mixture only having 100 µL of culture medium and cells was used as the negative control group.
3) Cell transfection: Transfection is not necessary for LNP-Luc. The highest concentration of mRNA was 100 ng per well, and three-fold gradient dilution was performed for a total of six gradients. Three replicates were set for each sample. 10 µL of LNP-Luciferase with different dilution ratios were uniformly added to a 96 well cell plate, mixed well, and cultured in an incubator.
4) Detection by kit: After transfection for 48 hours, the luminescence intensity of Luciferase was detected using BioTek SYNERGY ELISA reader according to the instructions of ONE Glo^{™} EX Luciferase Assay System Kit, and the average of OD values was used for comparison.

As shown in Figure 24, LNP (AL-6)-Luc series products can be expressed normally in cells, and the expression level increased with the increase of mRNA concentration. When the mRNA concentration was 1 µg/mL, the cell expression level in the AL-6 group was significantly higher than that in the positive control group (LNP (SM-102)-Luc). It can be seen that LNP-mRNA prepared from AL-6 is highly expressed *in vitro.*

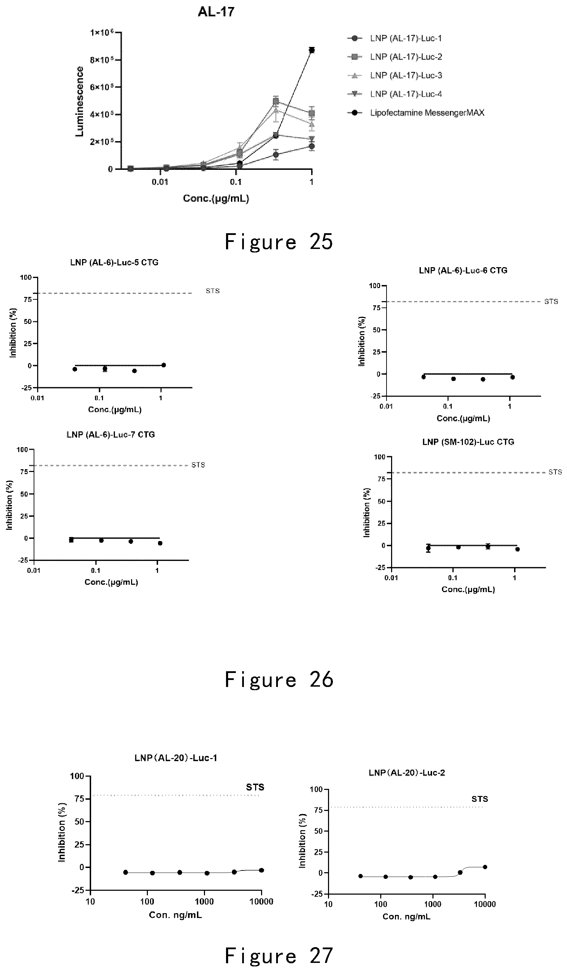

Figure 25 shows the expression of LNP (Al-17)-Luc series products in 293T cells.

### Example 5 Detection of In vivo toxicity of LNP-mRNA

Cytotoxicity experiments were performed according to the process shown in Figure 23. The specific steps are as follows:
1) Cell plating: After digestion of 293T cells, the cell density was adjusted to 2 × 10⁴/well and the cells were inoculated in a 96 well plate at 100 µL/well, then the plate was placed in a cell culture incubator overnight.
2) Experimental grouping: LNP (AL-6)-Luc and LNP (SM-102)-Luc were used as sample groups, 100 µL of culture medium was used as blank control group, a mixture only having 100 µL of culture medium and cells was used as the negative control group, a mixture of 100 µL of culture medium and cells with added Staurosporine (STS) apoptosis inducer was used as positive control group.
3) Cell transfection: Transfection is not necessary for LNP-Luc. The highest concentration of mRNA was 100 ng per well, and three-fold gradient dilution (buffer dilution) was performed for a total of four gradients. 10 µL of each LNP-Luc with different dilution ratios was uniformly added to a 96 well cell plate, and three replicates were set for each sample, mixed well, and cultured in an incubator. 10 µL of 100 µM STS (diluted in PBS) was added to a 96 well cell plate, and six replicates were set, mixed well, and cultured in an incubator.
4) Detection by kit: After 48 hours culture, the luminescence intensity was detected using BioTek SYNERGY ELISA reader according to the instructions of CellTiter-Glo^{®} Luminescent Cell V (I) bility Assay Kit, and the average of OD values was used for comparison. The cell inhibition rate was calculated by the formula "(negative control group - sample group/positive control group)/(negative control group - blank control group) × 100%" and the dose response inhibition curve was plotted.

As shown in Figures 26 and 27, the series of LNP-mRNA products formed by AL-6 in Example 3.1 showed no significant effect on cell proliferation at a concentration less than 1 µg/mL (Figure 26), indicating that there was no significant toxicity within the detected concentration range; The series of LNP-mRNA products formed by AL-20 showed no significant toxicity within the detected concentration range (Figure 27).

### Example 6 Detection of In vivo expression of LNP-mRNA

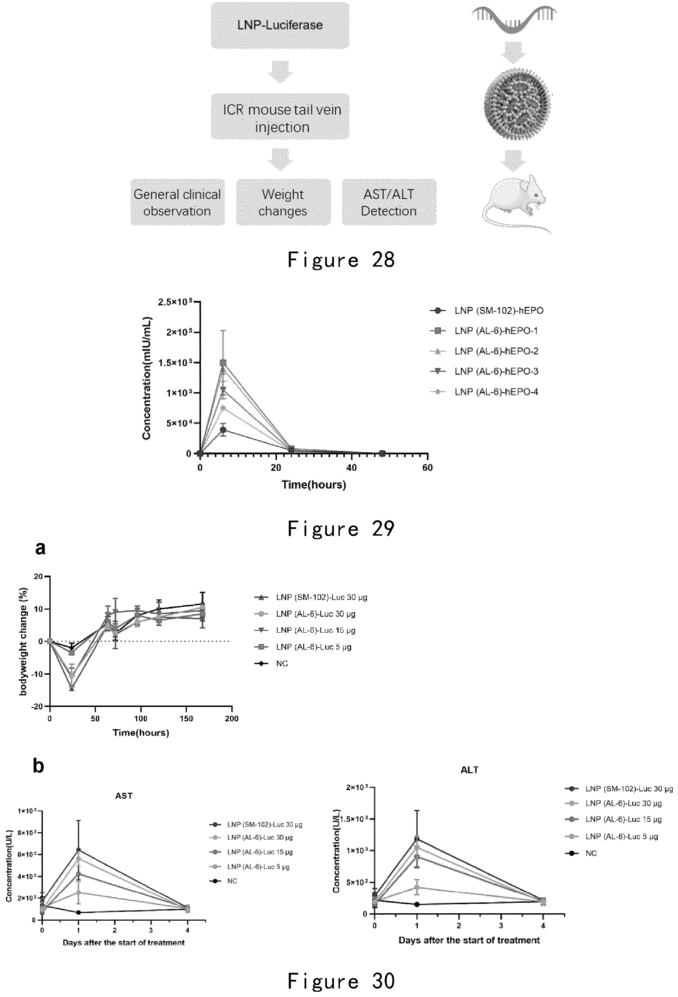

In order to elucidate the *in vivo* expression ability and toxicity of LNP-mRNA assembled using AL-6, hEPO ELISA expression detection experiments and toxicity experiments were further designed to measure its expression level and toxicity in mice with innate immune system. The steps are as follows:
The experimental procedure refers to Figure 28:
1) Encapsulation of LNP-mRNA: hEPO mRNA was dissolved in an aqueous buffer and mixed well to form the aqueous phase; Ionizable lipids (SM-102 or AL-6), DSPC, cholesterol, and DMG-PEG2000 were dissolved in anhydrous ethanol and thoroughly mixed in a certain proportion to form the organic phase. The aqueous and organic phases were transferred separately into syringes, and the PNI microfluidic nanogenerator was used to to prepare hEPO-LNP with parameters being set as volume ratio of aqueous phase to organic phase of 3:1, flow rate of 12mL/min). LNP-hEPO was concentrated, purified, sterilized and filtered, and then injected into mice after passing quality control.
2) Tail vein injection in mice: The mice were fixed and the tail vein was selected for injection. The injection dose of LNP-hEPO was 5 µg. LNP (AL-6)-hEPO was set as the sample group, LNP (SM-102)-hEPO was set as the positive control group, and the mice only injected with the solvent were the negative control group. Each LNP-hEPO sample was used to inject two mice.
3) Submaxillary blood collection in mice: Submaxillary blood collection was performed at 6, 24, and 48 hours after injection. Blood was collected in EDTA anticoagulant tubes and mixed well, numbered for subsequent use.
4) Serum extraction: The whole blood was obtained, then subjected to centrifugation at 2000 g for 10 minutes. The supernatant was plasma. After packaging, the plasm was stored at -80 °C.
5) The human Erythropoietin ELISA kit was used to detect hEPO expression levels: Performance was conducted according to instructions and two replicates were set for each serum sample. The ELISA reader was used to detect OD values and expression levels were calculated based on standard curves and dilution ratios.

As shown in Figure 29, the LNP-hEPO series products prepared from AL-6 can be successfully expressed in mice, with overall expression levels (AUC) higher than those of the positive control (SM-102). This experiment indicates that the LNP formed by AL-6 can efficiently mediate mRNA expression *in vivo.*

### Example 7 Detection of In vivo toxicity of LNP-mRNA

The process for detecting *in vivo* toxicity is shown in Figure 28, and the specific steps are as follows:
1) Encapsulation of LNP-mRNA: Luciferase mRNA was dissolved in an aqueous buffer and mixed well to form an aqueous phase; Ionizable lipids (SM-102 or AL-6), DSPC, cholesterol, and DMG-PEG2000 were dissolved in anhydrous ethanol and thoroughly mixed in a certain proportion to form an organic phase. The aqueous and organic phases were transferred separately into syringes, and the PNI microfluidic nanogenerator was used to prepare LNP-Luciferase. LNP-Luciferase was concentrated, purified, sterilized and filtered, and then injected into mice after passing quality control.
2) Tail vein injection in mice: The mice were fixed and the tail vein was selected for injection. The injection dose of LNP-Luciferase was 5, 15, 30 µg, respectively. LNP (AL-6)-Luc was set as the sample group, LNP (SM-102)-Luc was set as the positive control group and injected with 30 µg, and the mice injected only with the solvent was set as the negative control group. Each LNP-Luciferase sample was used to inject two mice.
3) Mouse weighing: Mice were weighed daily, and weight data was recorded continuously for 7 days. Weight gain or loss was calculated as a percentage.
4) Submaxillary blood collection in mice: Submaxillary blood collection was performed before injection (0 h) and at 24 h and 96 h after injection. Blood was collected in EDTA anticoagulant tubes and mixed well, numbered for subsequent use.
5) Serum extraction: The whole blood was obtained, then subjected to centrifugation at 2000 g for 10 minutes. The supernatant was plasma. After packaging, the plasm was stored at -80 °C.
6) Biochemical detection: The levels of ALT (alanine aminotransferase) and AST (aspartate aminotransferase) were detected by IUBIO iChem340 blood biochemistry analyzer. Statistical analysis was performed on concentration values, by calculating the average value, comparing the differences between groups, and drawing a curve.

As shown in Figure 30, the toxicity experiment on mice showed that after injection of different doses of LNP (AL-6)-Luc, the mice were in a relatively normal state without any deaths. After injection, the weight of mice showed a significant decrease, and the degree of decrease was significantly correlated with the injection dose. After 2 days, it gradually returned to normal, indicating that the effect of AL-6 on mouse weight is reversible.

After one day of injection, ALT and AST levels in mice increased, and the degree of increase showed a significant correlation with the injection dose; After 4 days, liver enzymes returned to pre-injection levels, indicating that the effect of AL-6 on liver function is reversible.

In this experiment, high doses of LNP (SM-102)-Luc were injected simultaneously, and its effects on mouse body weight and ALT/AST levels were comparable to those of AL-6 at the same dose. In the experimental system of the present invention, there was little difference in the effects of LNP (AL-6)-Luc high-dose group and LNP (SM-102)-Luc on mice, indirectly indicating that AL-6 is comparable to SM-102 in toxicity.

### Example 8 In vivo imaging experiment in mouse

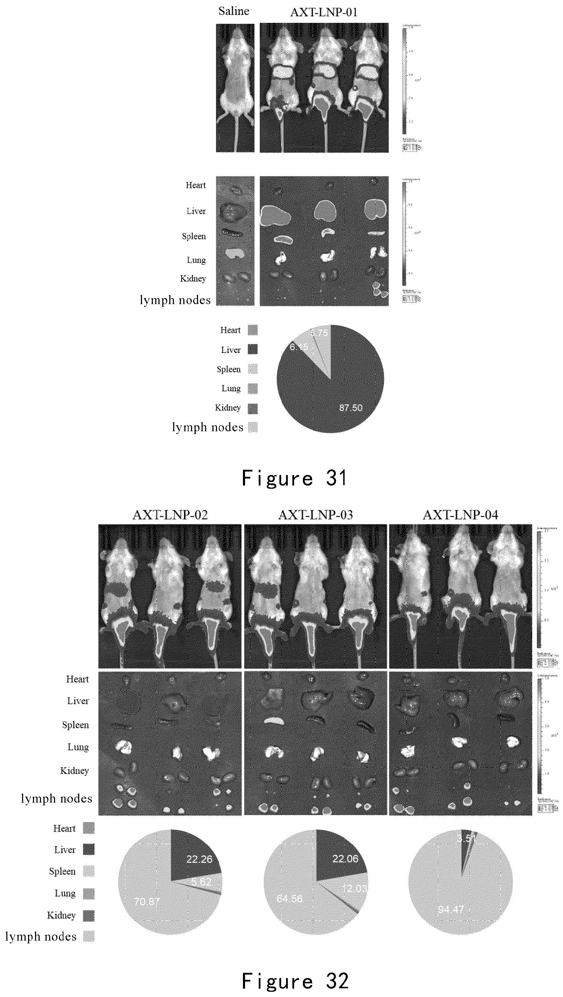

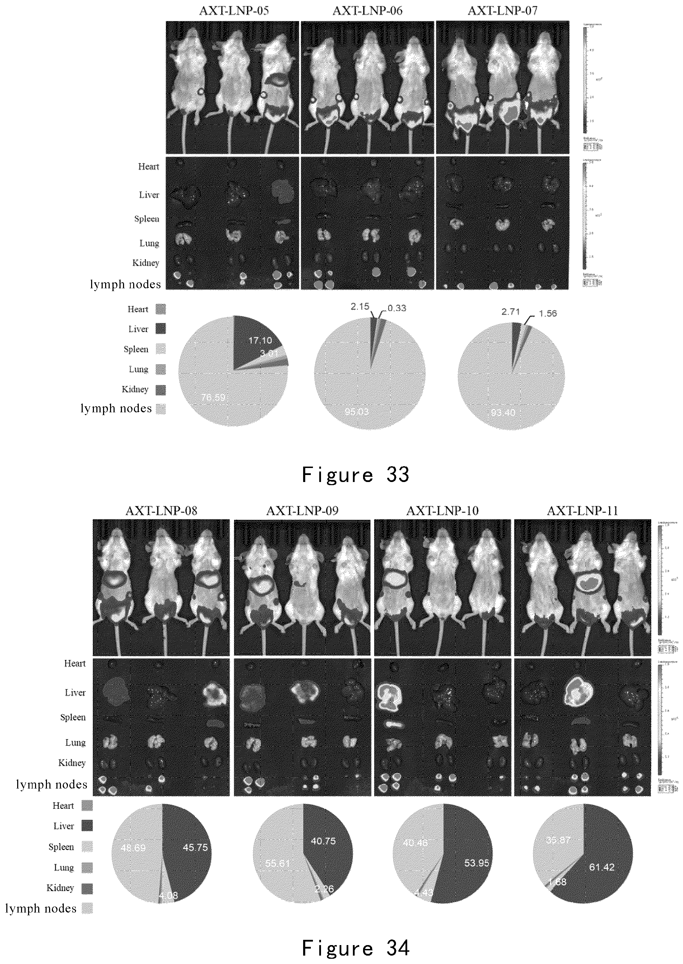

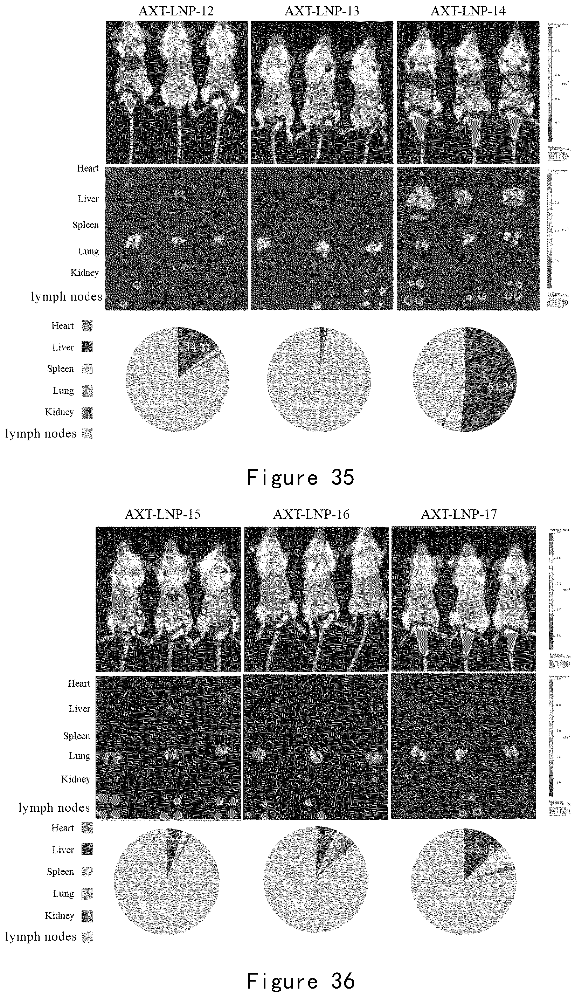

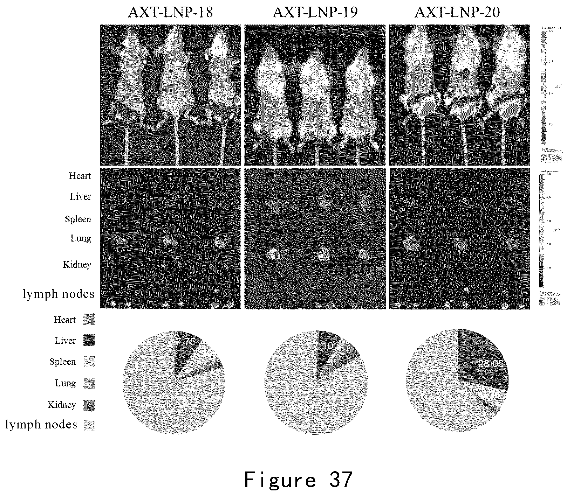

In this example, different AXT-LNP-x test substances (as shown in Table 1 above) were subcutaneously injected into BALB/c mice through dorsal injection, with a single dose of 10 µg/mouse and 3 mice for each formula. D-Luciferin substrate imaging was performed at 6 hours after the injection, and each animal, especially the head, chest, abdomen, and back, was shaved before imaging. Before imaging, 0.2 mL of D-Luciferin fluorescent substrate solution with a concentration of 15 mg/mL was injected into the abdominal cavity of each animal. Isoflurane anesthesia was used for *in vivo* imaging and immediate dissection for imaging of organs such as heart, liver, spleen, lungs, kidneys, and lymph nodes. Results of *in vivo* imaging, organ imaging, and fluorescence expression ratio of each organ for each formula are shown in Figures 31-37.

The results showed that all the test substances had strong organ targeting properties. The targeting mechanism of the above test substances was mainly tested from three aspects: the addition of the fifth component (anionic DOPA, DOPS), the component DMG-PEG2000 mannose for modification, and the targeting ionizable lipids; From the results, it can be seen that with the adjustment of anions, component DMG-PEG2000 mannose for modification, and the proportion of ionizable lipids in the formula, the proportion of targeted lymph nodes also varies to varying degrees. Moreover, the distribution of LNP in lymph nodes in most formulas can reach over 90%, and it can significantly reduce the aggregation and expression of LNP in organs such as liver, spleen, kidney, and heart.

All references mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. An ionizable lipid, or a pharmaceutically acceptable salt, tautomer, or stereoisomer thereof, wherein the ionizable lipid has a structure shown in the following Formula I: 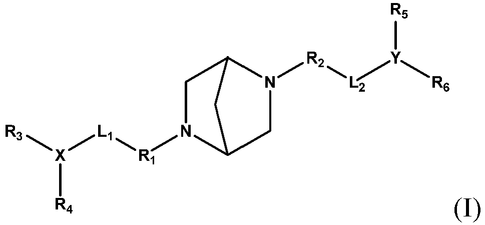 wherein
R1 and R2 are each independently selected from -(CH₂)n-, wherein n is a positive integer from 1 to 14;
X and Y are each independently -CH- or N;
L₁ and L₂ are each independently a divalent linking group or absent;
R₃, R₄, R₅, and R₆ are each independently H, CH₃, C2-C30 hydrocarbyl (such as C2-C30 alkyl, C2-C30 alkene, C2-C30 alkynyl), or -(CH₂)s-R^{a}-(CH₂)g-R^{b}-(CH₂)m-R^{c}, wherein s and g are each independently selected from a positive integer of 1-20, m is selected from an integer of 0-20, preferably s+g+m is 2-35; R^{c} is CH₃ or C2-C15 hydrocarbyl (such as C2-C15 alkyl, C2-C15 alkene, C2-C15 alkynyl);
R^{a} and R^{b} are each independently selected from -CH₂-, C2-C6 alkenyl structure or a functional group as shown below: 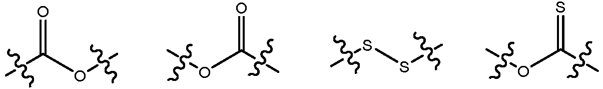 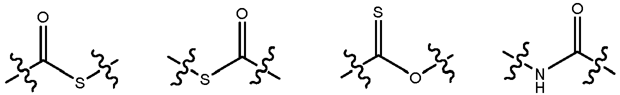 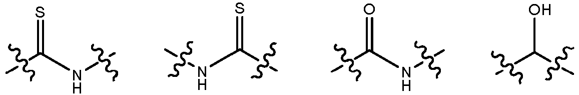
wherein 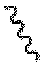 represents a linking bond;
and R₃ and R₄ are not simultaneously H, R₅ and R₆ are not simultaneously H.

2. The ionizable lipid, or a pharmaceutically acceptable salt, tautomer, or stereoisomer thereof according to claim 1, wherein the ionizable lipid has a substructure shown in the following Formula (I-1): 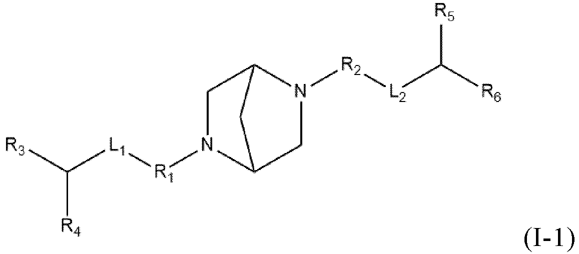 wherein
R₁ and R₂ are each independently selected from -(CH₂)n-, wherein n is a positive integer from 1 to 14;
L₁ has a structure of -(L₁ₐ-L_{1b}-L_{1c})- from right to left, wherein L_{1b} is -(CH₂)n-, wherein n is a positive integer selected from 1-14; L₁ₐ and L_{1c} are each independently selected from the following functional groups: -CH₂-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, - (C=O)NH- or -CH(OH)-, preferably -CH₂-, - (C=O)O-, -O(C=O)-, -(S-S)- or - CH(OH)-;
L₂ has a structure of -(L₂ₐ-L_{2b}-L_{2c})- from left to right, wherein L_{2b} is -(CH₂)n-, wherein n is a positive integer selected from 1-14; L₂ₐ and L_{2c} are each independently selected from the following functional groups: -CH₂-, -(C=O)O-, -O(C=O)-, -(S-S)-, -O(S=O)-, -(C=O)S-, -S(C=O)-, -(C=S)O-, -NH(C=O)-, -(C=S)NH-, -NH(C=S)-, - (C=O)NH- or -CH(OH)-, preferably -CH₂-, - (C=O)O-, -O(C=O)-, -(S-S)- or - CH(OH)-;
R₃, R₄, R₅, and R₆ are each independently a C2-C20 hydrocarbyl.

3. The ionizable lipid, or a pharmaceutically acceptable salt, tautomer, or stereoisomer thereof according to claim 1, wherein the ionizable lipid has a substructure shown in the following Formula (I-2): 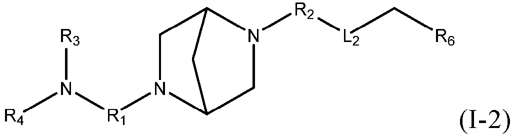 wherein
R₁ and R₂ are each independently selected from -(CH₂)n-, wherein n is a positive integer from 1 to 14;
L₂ has a structure of -(L₂ₐ-L_{2b}-L_{2c})- from left to right,
wherein L_{2b} is -(CH₂)n-, wherein n is a positive integer selected from 1-14; L₂ₐ and L_{2c} are each independently selected from the following functional groups: -CH₂-, -NH-, -(C=O)O-, -O(C=O)-, -(S-S)-, -O(S=O)-, -(C=O)S-, -S(C=O)-, -(C=S)O-, - NH(C=O)-, -(C=S)NH-, -NH(C=S)-, -(C=O)NH-, -CH(OH)-, -(C=C)-(CH₂)-(C=C)-, -(C=C)- or -(C≡C)-, preferably -CH₂-, -NH-, -(C=O)O-, -O(C=O)-, -(S-S)-, -(C=C)-(CH₂)-(C=C)- or -CH(OH)-;
R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ;
wherein R₃ₐ, R_{3c}, R₄ₐ and R_{4c} are each independently -(CH₂)n-, wherein n is a positive integer selected from 1-14;
R_{3b}, R₃ₐ, R_{4b} and R_{4d} are each independently selected from the following functional groups: -CH₂-, -(C=O)O-, -O(C=O)-, -(S-S)-, -O(S=O)-, -(C=O)S-, - S(C=O)-, -(C=S)O-, -NH(C=O)-, -(C=S)NH-, -NH(C=S)-, -(C=O)NH-, -CH(OH)-, - (C=C)-(CH₂)-(C=C)-, -(C=C)- or -(C≡C)-, preferably -CH₂-, -(C=O)O-, -O(C=O)-, - (S-S)-, -(C=C)-(CH₂)-(C=C)- or -CH(OH)-;
R₃ₑ and R₄ₑ are each independently a C2-C20 hydrocarbyl;
R₆ is a C2-C20 hydrocarbyl.

4. The ionizable lipid, or a pharmaceutically acceptable salt, tautomer, or stereoisomer thereof according to claim 1, wherein the ionizable lipid has a substructure shown in the following Formula (I-3): 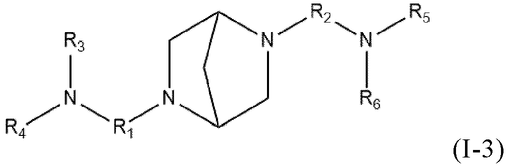 wherein
R₁ and R₂ are each independently selected from -(CH₂)n-, wherein n is a positive integer from 1 to 14;
R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ; R₅ has a structure of -R₅ₐ-R₅₆-R_{5c}-R₅ₐ-R₅ₑ, and R₆ has a structure of -R₆ₐ-R_{6b}-R_{6c}-R_{6d}-R₆ₑ;
wherein R₃ₐ, R_{3c}, R₄ₐ, R_{4c}, R₅ₐ, R_{5c}, R₆ₐ and R_{6c} are each independently -(CH₂)n-, wherein n is a positive integer selected from 1-14;
R_{3b}, R_{3d}, R_{4b}, R_{4d}, R_{5b}, R_{5d}, R_{6b} and R_{6d} are each independently selected from the following functional groups: -CH₂-, -(C=O)O-, -O(C=O)-, -(S-S)-, -O(S=O)-, - (C=O)S-, -S(C=O)-, -(C=S)O-, -NH(C=O)-, -(C=S)NH-, -NH(C=S)-, -(C=O)NH-, - CH(OH)-, -(C=C)-(CH₂)-(C=C)-, -(C=C)- or -(C≡C)-, preferably -CH₂-, -(C=O)O-, -O(C=O)-, -(S-S)-, -(C=C)-(CH₂)-(C=C)- or -CH(OH)-;
R₃ₑ, R₄ₑ, R₅ₑ and R₆ₑ are each independently a C2-C20 hydrocarbyl.

5. A method for preparing ionizable lipids, or a pharmaceutically acceptable salt, tautomer, or stereoisomer thereof according to claim 1, which comprises method I, method II, and method III;
wherein the method I comprises the following steps:
(A1) Compounds K2 and K3 are subjected to react with compound K1 in an inert solvent to obtain compound K4;
(A2) Compounds K5 and K6 are subjected to react with compound K4 in an inert solvent to obtain the compound shown in Formula (I-1); 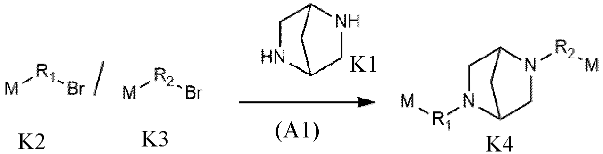 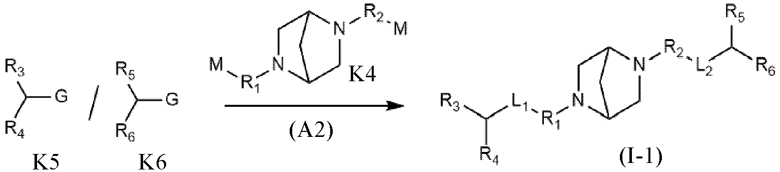
wherein R₁ and R₂ are each independently selected from -(CH₂)n-, wherein n is a positive integer from 1 to 14;
M and G are each independently and preferably selected from -OH, COOH, - SH, -NH2, ethylene oxide;
L₁ has a structure of -(L₁ₐ-L_{1b}-L_{1c})-, L₂ has a structure of -(L₂ₐ-L_{2b}-L₂ₑ)-;
wherein L_{1b} and L_{2b} are each independently -(CH₂)n-, wherein n is a positive integer selected from 1 to 14;
L₁ₐ, L_{1c}, L₂ₐ and L_{2c} are each independently selected from the following functional groups: -CH₂-, -(C=O)O-, -O(C=O)-, -(S-S)-, -O(S=O)-, -(C=O)S-, - S(C=O)-, -(C=S)O-, -NH(C=O)-, -(C=S)NH-, -NH(C=S)-, -(C=O)NH- or -CH(OH)-, preferably -CH₂-, -(C=O)O-, -O(C=O)-, -(S-S)- or -CH(OH)-;
R₃, R₄, R₅ and R₆ are each independently a C2-C20 hydrocarbyl;
The method II comprises the following steps:
(B1) Compound K7 is subjected to react with compound K8 in an inert solvent to obtain compound K9;
(B2) Compound K9 is subjected to deprotection, thereby obtaining compond K10;
(B3) Compounds K11, K12 and K13 are subjected to react with compound K10 in an inert solvent to obtain the compound shown in Formula (1-2); 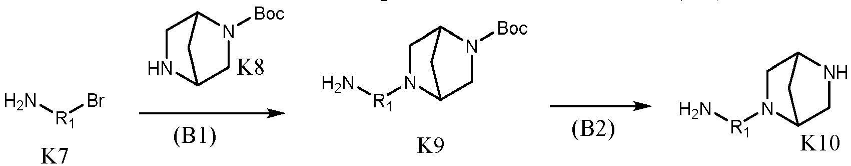 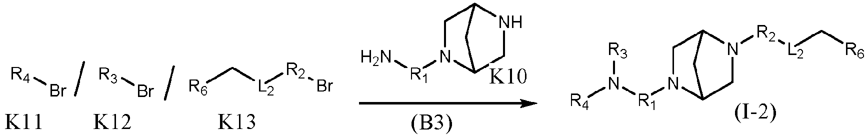
wherein R₁ and R₂ are each independently selected from -(CH₂)n-, wherein n is a positive integer from 1 to 14;
L₂ has a strusture of -(L₂ₐ-L_{2b}-L_{2c})-;
wherein L_{2b} is -(CH₂)n-, wherein n is a positive integer selected from 1-14; L₂ₐ and L_{2c} are each independently selected from the following functional groups: -CH₂-, -NH-, -(C=O)O-, -O(C=O)-, -(S-S)-, -O(S=O)-, -(C=O)S-, -S(C=O)-, -(C=S)O-, - NH(C=O)-, -(C=S)NH-, -NH(C=S)-, -(C=O)NH-, -CH(OH)-, -(C=C)-(CH₂)-(C=C)-, -(C=C)- or -(C≡C)-, preferably -CH₂-, -NH-, -(C=O)O-, -O(C=O)-, -(S-S)-, -(C=C)-(CH₂)-(C=C)- or -CH(OH)-;
R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ;
wherein R₃ₐ, R_{3c}, R₄ₐ and R_{4c} are each independently -(CH₂)n-, wherein n is a positive integer selected from 1-14;
R_{3b}, R_{3d}, R_{4b} and R_{4d} are each independently selected from the following functional groups: -CH₂-, -(C=O)O-, -O(C=O)-, -(S-S)-, -O(S=O)-, -(C=O)S-, - S(C=O)-, -(C=S)O-, -NH(C=O)-, -(C=S)NH-, -NH(C=S)-, -(C=O)NH-, -CH(OH)-, - (C=C)-(CH₂)-(C=C)-, -(C=C)- or -(C≡C)-, preferably -CH₂-, -(C=O)O-, -O(C=O)-, - (S-S)-, -(C=C)-(CH₂)-(C=C)- or -CH(OH)-;
R₃ₑ and R₄ₑ are each independently a C2-C20 hydrocarbyl;
R₆ is a C2-C20 hydrocarbyl;
The method III comprises the following steps:
(C1) Compound K7 is subjected to react with compound K14 in an inert solvent to obtain compound K15;
(C2) Compounds K11, K12, K16 and K17 are subjected to react with compound K15 in an inert solvent to obtain the compound shown in Formula (I-3); 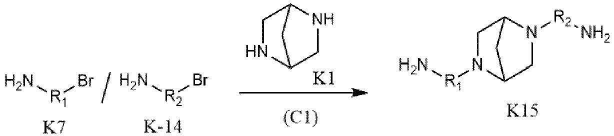 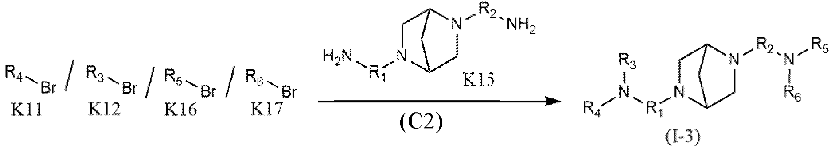
wherein
R₁ and R₂ are each independently selected from -(CH₂)n-, wherein n is a positive integer from 1 to 14;
R₃ has a structure of -R₃ₐ-R_{3b}-R_{3c}-R_{3d}-R₃ₑ, R₄ has a structure of -R₄ₐ-R_{4b}-R_{4c}-R_{4d}-R₄ₑ; R₅ has a structure of -R₅ₐ-R_{5b}-R_{5c}-R_{5d}-R₅ₑ, and R₆ has a structure of -R₆ₐ-R_{6b}-R_{6c}-R_{6d}-R₆ₑ;
wherein R₃ₐ, R₃ₑ, R₄ₐ, R_{4c}, R₅ₐ, R_{5c}, R₆ₐ and R_{6c} are each independently -(CH₂)n-, wherein n is a positive integer selected from 1-14;
R_{3b}, R_{3d}, R_{4b}, R₄ₐ, R_{5b}, R₅ₐ, R_{6b} and R_{6d} are each independently selected from the following functional groups: -CH₂-, -(C=O)O-, -O(C=O)-, -(S-S)-, -O(S=O)-, - (C=O)S-, -S(C=O)-, -(C=S)O-, -NH(C=O)-, -(C=S)NH-, -NH(C=S)-, -(C=O)NH-, - CH(OH)-, -(C=C)-(CH₂)-(C=C)-, -(C=C)-, -(C≡C)-, preferably -CH₂-, -(C=O)O-, - O(C=O)-, -(S-S)-, -(C=C)-(CH₂)-(C=C)-, -CH(OH)-;
R₃ₑ, R₄ₑ, R₅ₑ and R₆ₑ are each independently a C2-C20 hydrocarbyl.

6. A lipid nanoparticle (LNP) comprising the ionizable lipid, or a pharmaceutically acceptable salt, tautomer, or stereoisomer thereof according to any one of claims 1-4.

7. The lipid nanoparticle according to claim 6, which further comprises auxiliary lipids; and the auxiliary lipids include auxiliary phospholipids, sterols, polymer conjugated lipids, or a combination thereof;
wherein the lipid nanoparticle preferentially delivers the bioactive substances encapsulated therein to lymph nodes and reduces the delivery to organs or tissues other than lymph nodes, such as the heart, liver, spleen, lungs, kidneys, brain, etc.

8. The lipid nanoparticle according to claim 7, wherein the lipid nanoparticle comprises ionizable lipid, DSPC, cholesterol and DMG-PEG2000, wherein the molar ratio of ionizable lipid: DSPC: cholesterol: DMG-PEG2000 is (30-65): (5-30): (30-55): (1-5).

9. The lipid nanoparticle according to claim 7, wherein the lipid nanoparticle comprises an ionizable lipid, DSPC, cholesterol and DMG-PEG2000, and a fifth component selected from the group consisting of:
(1) DOPA (dioleoylphosphatidylic acid) or DOPS (dioleoylphosphatidylserine);
(2) DMG-PEG2000-mannose; and a combination thereof;
wherein the molar ratio of ionizable lipid: DSPC: cholesterol: DMG-PEG2000: the fifth component is (30-50): (7.5-15): (25-50): (1-1.5): (0.5-25).

10. The lipid nanoparticle according to claim 6, wherein the ionizable lipid has the following structure:
| Serial number | Structural formula |
|---|---|
| AL-6 | 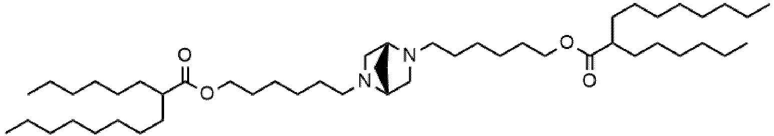 |
| AL-17 | 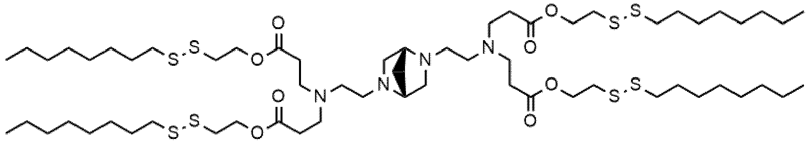 |
| AL-18 | 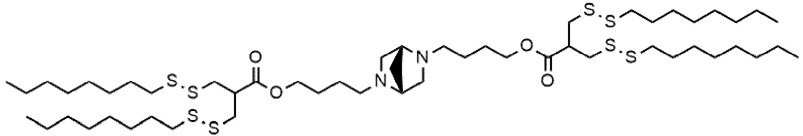 |
| AL-19 | 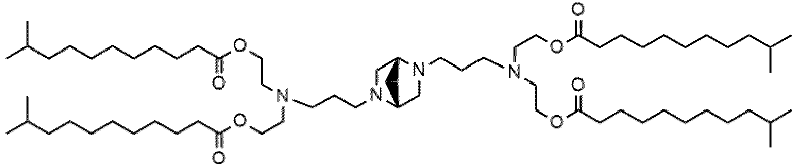 |
| AL-20 | 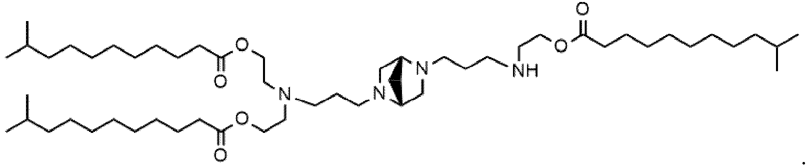 |

11. A lipid nanoparticle pharmaceutical preparation, which comprises the lipid nanoparticle according to claim 6, a bioactive substance encapsulated in the lipid nanoparticle, and a pharmaceutically acceptable carrier.

12. The lipid nanoparticle pharmaceutical preparation according to claim 11, wherein the bioactive substance is selected from the group consisting of nucleic acids, proteins, peptides, small molecules, and a combination thereof.

13. A method for preparing the pharmaceutical preparation according to claim 11, which comprises the steps:
(a) Mixing the ionizable lipid or a pharmaceutically acceptable salt, tautomer or stereoisomer thereof according to any one of claims 1-4, and optional auxiliary lipids with an organic solvent to obtain a lipid organic phase;
(b) Mixing bioactive substances with an aqueous solvent to obtain an aqueous phase containing the bioactive substances;
(c) Mixing the lipid organic phase in step (a) with the aqueous phase in step (b) to obtain the lipid nanoparticle pharmaceutical preparation.

14. Use of the ionizable lipid, or a pharmaceutically acceptable salt, tautomer, or stereoisomer thereof according to any one of claims 1-4 for the preparation of a drug delivery system.

15. Use of the lipid nanoparticle according to claim 6 for the preparation of drugs for the treatment and/or prevention of tumors, infectious diseases, and rare diseases.
